# EUROPEAN PATENT APPLICATION

(11) **EP 4 729 506 A1**
(43) Date of publication of application: **22.04.2026**
(21) Application number: 25203510.0
(22) Date of filing: 19.09.2025
(51) Int. Cl.: C07C 211/54, C07C 211/58, C07C 211/61, C07D 307/77, C07D 307/91, C07D 333/50, C07D 333/76, H10K 85/60, H10K 50/15

(54) **LIGHT EMITTING ELEMENT, MONOAMINE COMPOUND FOR THE LIGHT EMITTING ELEMENT, AND ELECTRONIC APPARATUS INCLUDING THE LIGHT EMITTING ELEMENT**

(30) Priority: 16.10.2024 JP 2024180770
(71) Applicant: Samsung Display Co., Ltd., Yongin-si, Gyeonggi-Do (KR)
(72) Inventor: SAKAMOTO, Naoya, Yokohama-shi, 220-0011 (JP)
(74) Representative: Marks & Clerk LLP

(57) **Abstract**

A light emitting element includes a first electrode, a hole transport region, an emission layer, an electron transport region, and a second electrode. The hole transport region includes a monoamine compound represented by Formula 1.

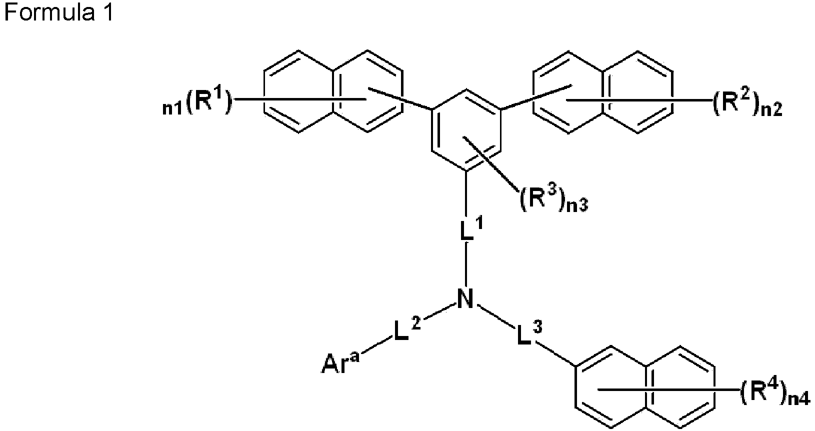

## Description

### BACKGROUND

### 1. Field

One or more embodiments of the present disclosure relate to a light emitting element, a monoamine compound used in the light emitting element, and an electronic apparatus including the light emitting element.

### 2. Description of the Related Art

Various electronic apparatuses include image display devices. Among such devices, organic electroluminescence (EL) display devices have been widely adopted and are under active development. These organic EL display devices are self-luminous and include light-emitting elements in which holes and electrons, injected separately from a first electrode and a second electrode, recombine in an emission layer to emit light (e.g., display of images).

For application of light emitting elements to display devices, there is a continuous demand or desire for higher luminous efficiency and/or longer operational lifetime. Accordingly, the development of materials for light-emitting elements that may stably achieve these desired characteristics remains an active area of research and development.

### SUMMARY

One or more aspects of embodiments of the present disclosure are directed toward a light emitting element that has increased light efficiency and lifespan, and an electronic apparatus including the same.

One or more aspects of embodiments of the present disclosure are directed toward to a monoamine compound as a material for a light emitting element, which improves light efficiency and lifespan of the light emitting element.

Additional aspects will be set forth in part in the description which follows and, in part, will be apparent from the description, or may be learned by practice of the presented embodiments.

According to one or more embodiments of the present disclosure, a light emitting element includes a first electrode, a hole transport region on (e.g., arranged on) the first electrode and including a monoamine compound represented by Formula 1, an emission layer on (e.g., arranged on) the hole transport region, an electron transport region on (e.g., arranged on) the emission layer, and a second electrode on (e.g., arranged on) the electron transport region.

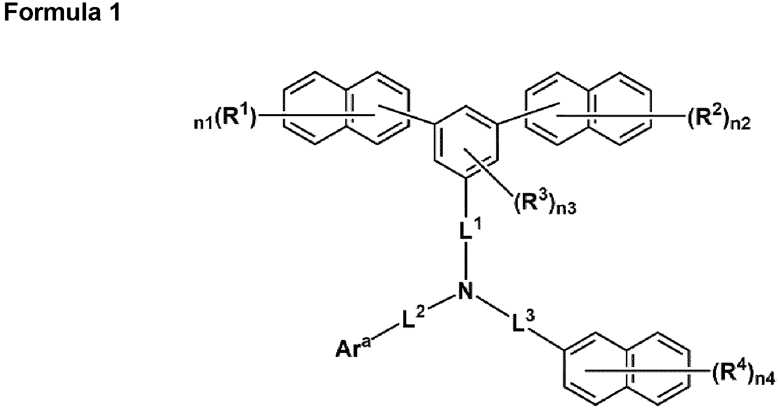

In Formula 1, L¹ is a direct linkage, a substituted or unsubstituted arylene group having 6 to 30 ring-forming carbon atoms, or a substituted or unsubstituted heteroarylene group having 5 to 30 ring-forming carbon atoms, L² and L³ are each independently a direct linkage, an unsubstituted phenylene group, a phenylene group substituted with deuterium, or a phenylene group substituted with a phenyl group, Ar^{a} is an unsubstituted phenyl group, an unsubstituted naphthalenyl group, a naphthalenyl group substituted with a phenyl group, an unsubstituted phenanthrenyl group, a phenanthrenyl group substituted with a phenyl group, an unsubstituted dibenzofuranyl group, an unsubstituted dibenzothiophenyl group, an unsubstituted carbazolyl group, a carbazolyl group substituted with a phenyl group, an unsubstituted benzonaphthofuranyl group, or an unsubstituted benzonaphthothiophenyl group, R¹ to R³ are each independently hydrogen, deuterium, a halogen, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms, R⁴ is hydrogen, deuterium, a halogen, a substituted or unsubstituted aryl group having 6 to 30 ring-forming carbon atoms, or a substituted or unsubstituted heteroaryl group having 5 to 30 ring-forming carbon atoms, n1, n2, and n4 are each independently an integer of 0 to 7, n3 is an integer of 0 to 3, and a chemical structure of Formula 1 in which any hydrogen is optionally substituted with deuterium is included.

In one or more embodiments, the monoamine compound represented by Formula 1 may be represented by any one selected from among Formulas 1-A1 to 1-A3.

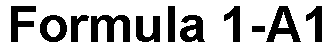

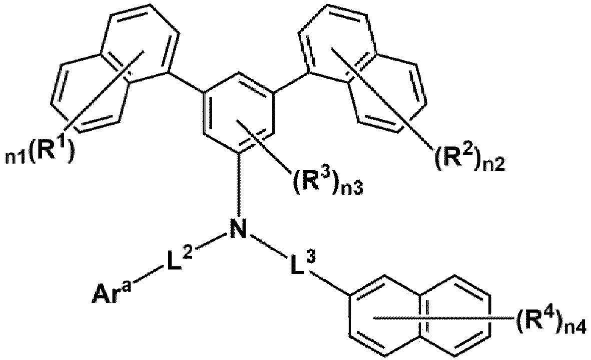

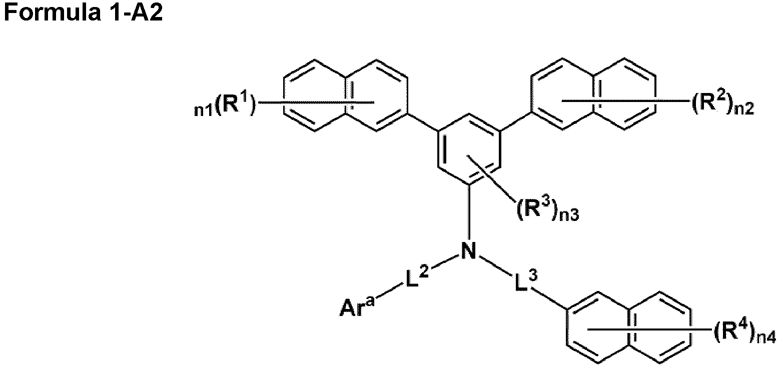

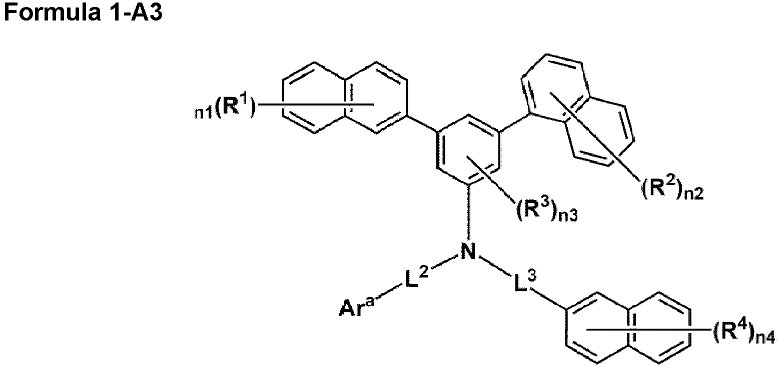

In Formulas 1-A1 and 1-A3, L², L³, Ar^{a}, R¹ to R⁴, and n1 to n4 are each independently the same as defined in Formula 1.

In one or more embodiments, the monoamine represented by Formula 1 may be represented by any one selected from among Formulas 1-B1 to 1-B3.

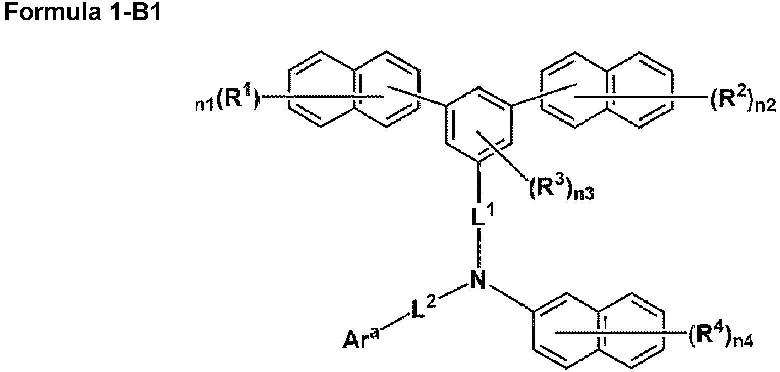

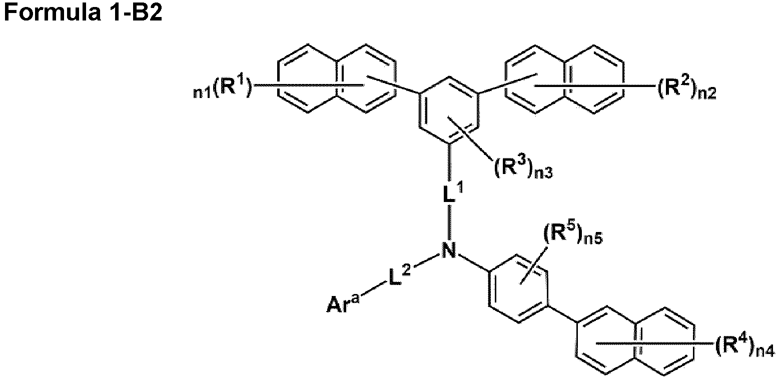

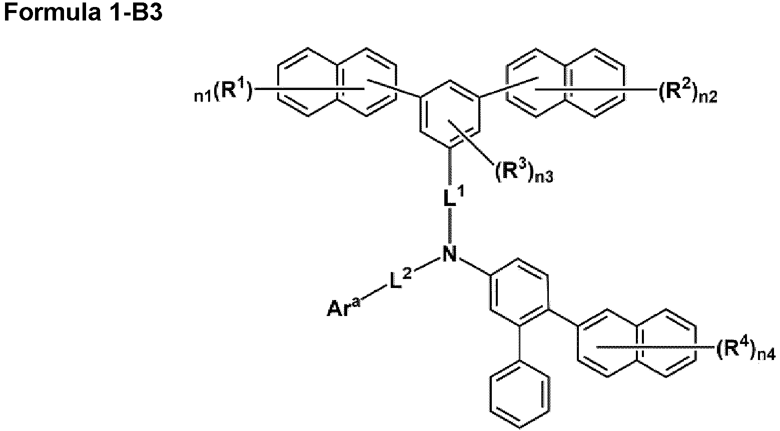

In Formula 1-B2, n5 is an integer of 0 to 4, and R⁵ is hydrogen or deuterium. In Formulas 1-B1 to 1-B3, L¹, L², Ar^{a}, R¹ to R⁴, and n1 to n4 are each independently the same as defined in Formula 1.

In one or more embodiments, in Formula 1, L² may be a direct linkage or be represented by any one selected from among L2-1 to L2-4.

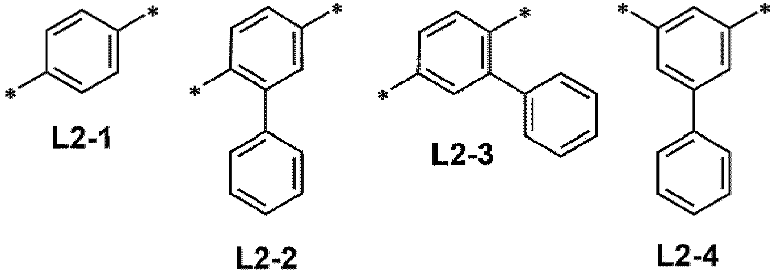

In one or more embodiments, in Formula 1, Ar^{a} may be represented by any one selected from among AR-1 to AR-38.

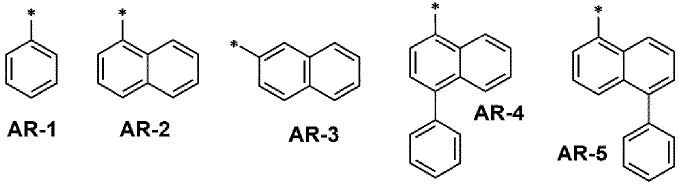

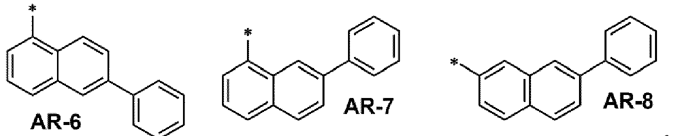

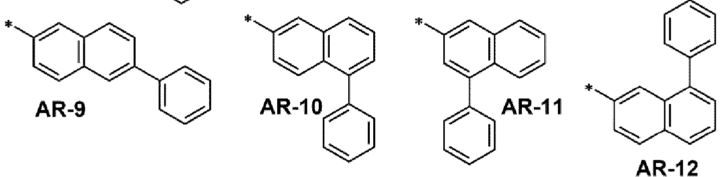

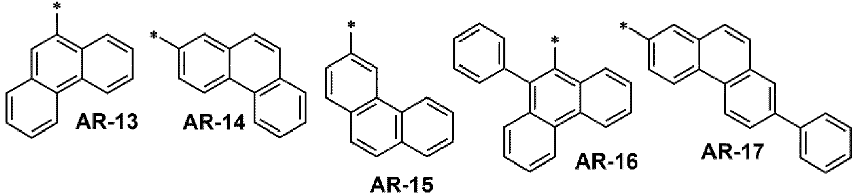

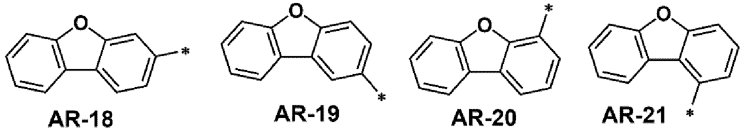

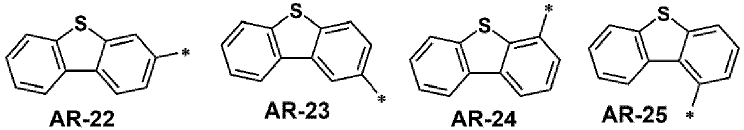

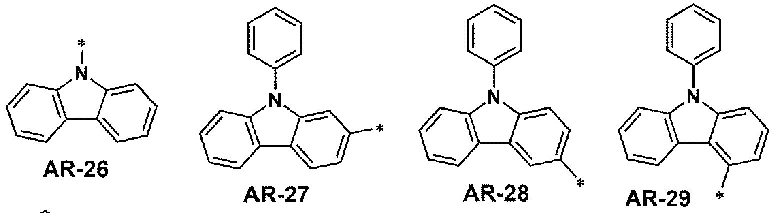

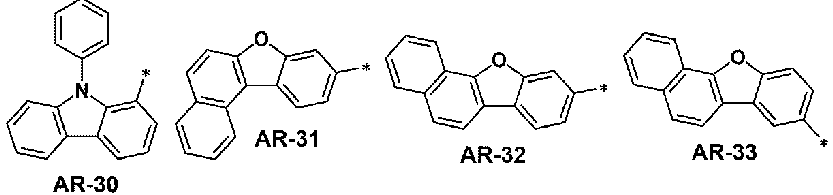

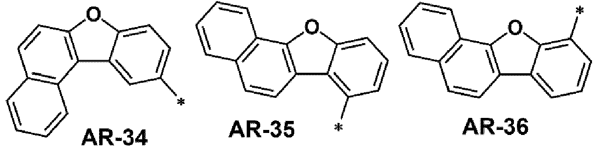

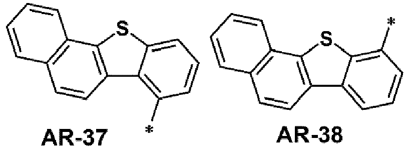

In one or more embodiments, R¹ to R³ may each independently be hydrogen, deuterium, fluorine, or an unsubstituted phenyl group.
In Formula 1, R⁴ may be hydrogen, deuterium, fluorine, an unsubstituted phenyl group, or an unsubstituted dibenzofuranyl group.
In one or more embodiments, in Formula 1, a naphthalenyl group containing R⁴ may be represented by any one selected from among R4-1 to R4-6.

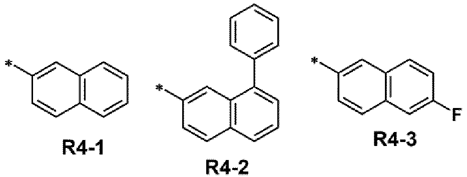

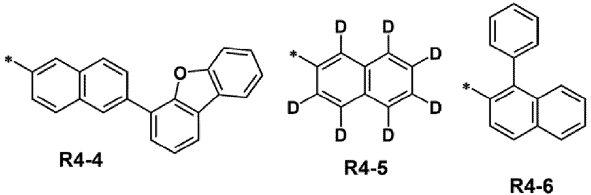

In R4-5, D is deuterium.

In one or more embodiments, the emission layer may include a compound represented by Formula E-1.

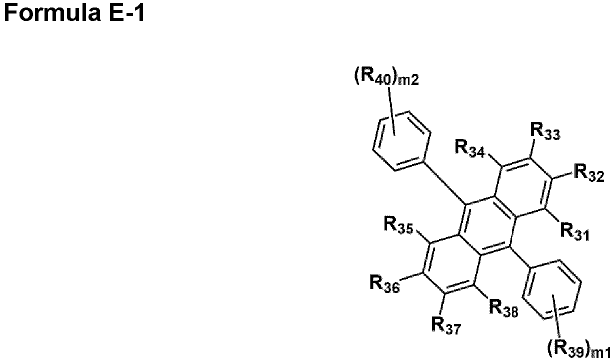

In Formula E-1, m1 and m2 are each independently an integer of 0 to 5, and R₃₁ to R₄₀ are each independently hydrogen, deuterium, a halogen, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring.

In one or more embodiments of the present disclosure, provided is a monoamine compound represented by Formula 1.

In one or more embodiments of the present disclosure, an electronic apparatus includes a base layer, a circuit layer on (e.g., arranged on) the base layer, and a display element layer on (e.g., arranged on) the circuit layer and including a light emitting element, wherein the light emitting element includes a first electrode, a hole transport region on (e.g., arranged on) the first electrode and including a monoamine compound represented by Formula 1, an emission layer on (e.g., arranged on) the hole transport region, an electron transport region on (e.g., arranged on) the emission layer, and a second electrode on (e.g., arranged on) the electron transport region.

In one or more embodiments, the electronic apparatus may further include at least one of a light control layer or a color filter layer, wherein the light control layer may include quantum dots, and the color filter layer may include a pigment and/or a dye.

In one or more embodiments, the electronic apparatus may include a display device comprising the light emitting element, wherein the display device may include any one selected from among a television, a monitor, an outdoor billboard, a personal computer, a laptop computer, a personal digital terminal, a vehicle display device, a game console, a portable electronic device, and a camera.

At least some of the above and other features of the invention are set out in the claims.

### BRIEF DESCRIPTION OF DRAWINGS

The accompanying drawings are included to provide a further understanding of the present disclosure, and are incorporated in and constitute a part of this disclosure. The drawings illustrate embodiments of the present disclosure and, together with the description, serve to explain principles of the disclosure. Above and/or other aspects of the disclosure should become apparent and appreciated from the following description of embodiments taken in conjunction with the accompanying drawings. In the drawings:
FIG. 1 is a plan view showing a display device according to one or more embodiments of the present disclosure;
FIG. 2 is a cross-sectional view showing a portion corresponding to the line I-I' of FIG. 1 according to one or more embodiments of the present disclosure;
FIG. 3 is a cross-sectional view schematically showing a light emitting element of one or more embodiments of the present disclosure;
FIG. 4 is a cross-sectional view schematically showing a light emitting element of one or more embodiments of the present disclosure;
FIG. 5 is a cross-sectional view schematically showing a light emitting element of one or more embodiments of the present disclosure;
FIG. 6 is a cross-sectional view schematically showing a light emitting element of one or more embodiments of the present disclosure;
FIG. 7 is a cross-sectional view showing a display device according to one or more embodiments of the present disclosure;
FIG. 8 is a cross-sectional view showing a display device according to one or more embodiments of the present disclosure;
FIG. 9 is a cross-sectional view showing a display device according to one or more embodiments of the present disclosure;
FIG. 10 is a cross-sectional view showing a display device according to one or more embodiments of the present disclosure;
FIG. 11 is a view showing the inside of a vehicle in which a display device of one or more embodiments is arranged;
FIG. 12 is a perspective view showing an electronic apparatus of one or more embodiments of the present disclosure;
FIG. 13 is an exploded perspective view showing an electronic apparatus of one or more embodiments of the present disclosure;
FIG. 14 is a block diagram of an electronic apparatus according to one or more embodiments of the present disclosure; and
FIG. 15 shows schematic views of electronic apparatuses according to one or more embodiments of the present disclosure.

### DETAILED DESCRIPTION

The present disclosure may be modified in many alternate forms, and thus specific/example embodiments will be exemplified in the drawings and described in more detail. It should be understood, however, that it is not intended to limit the present disclosure to the particular forms disclosed, but rather, is intended to cover all modifications, equivalents, and alternatives falling within the scope of the present disclosure.

In this disclosure, it will be understood that if (e.g., when) an element (or a region, a layer, a portion, and/or the like) is referred to as being "on", "connected to", or "coupled to" another element, it may be directly arranged on, connected to, or coupled to the other element, or other elements may be arranged therebetween. In the present disclosure, "directly on" may refer to that there are no additional layers, films, regions, plates, and/or like, between a layer, a film, a region, a plate, *etc.* and the other part. For example, "directly on" may refer to two layers or two members are arranged without utilizing an additional member such as an adhesive member therebetween.

Like reference numerals or symbols refer to like elements throughout the disclosure, and duplicative descriptions thereof may not be provided for conciseness. In the drawings, the thickness, ratio, and size of the elements may be exaggerated for effectively describing the technical contents. The terms "and/or" and/or "/" and/or "or" may include any and all combinations of one or more of the associated listed elements. Expressions such as "at least one of," "one of," and "selected from," when preceding a list of elements, modify the entire list of elements and do not modify the individual elements of the list. For example, "at least one of a, b, or c", "at least one selected from a, b, and c", "at least one selected from among a to c", etc., may indicate only a, only b, only c, both (e.g., simultaneously) a and b, both (e.g., simultaneously) a and c, both (e.g., simultaneously) b and c, all of a, b, and c, or variations thereof. The "/" utilized herein may be interpreted as "and" or as "or" depending on the situation.

It will be understood that, although the terms "first", "second", and/or the like, may be used herein to describe one or more suitable elements, the elements are not to be limited by these terms. These terms are only used to distinguish one element from another element. For instance, a first element could be termed a second element without departing from the scope of the disclosure. Similarly, a second element could be termed a first element. The singular expressions "a", "an", "one," and "the" are intended to include the plural forms as well, unless the context clearly indicates otherwise. Further, the utilization of "may" when describing embodiments of the present disclosure refers to "one or more embodiments of the present disclosure."

In addition, the terms "below", "under", "on the lower side", "above", "over", "on the upper side", and/or the like may be used to describe the relationships between the elements illustrated in the drawings. These terms are relative concepts and are described on the basis of the directions indicated in the drawings.

It will be further understood that the terms "comprise(s)/comprising," "include(s)/including," and/or "has(have)/ having", if (e.g., when) used in this disclosure, specify the presence of stated features, numbers, steps, operations, elements, components, or combinations thereof, but do not preclude the possibility of the presence or addition of one or more other features, numbers, steps, operations, elements, components, and/or combinations thereof. Additionally, the terms "comprise(s)/comprising," "include(s)/including," "has(have)/having," or other similar terms include or support the terms "consisting of" and "consisting essentially of," indicating the presence of stated features, numbers, steps, operations, elements, and/or components, without or essentially without the presence of other features, numbers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As used herein, an integer selected from 0 to 3 refers to an integer selected from 0, 1, 2, and 3; an integer selected from 0 to 4 refers to an integer selected from 0, 1, 2, 3, and 4; an integer selected from 0 to 5 refers to an integer selected from 0, 1, 2, 3, 4, and 5; an integer selected from 0 to 7 refers to an integer selected from 0, 1, 2, 3, 4, 5, 6, and 7; an integer selected from 0 to 8 refers to an integer selected from 0, 1, 2, 3, 4, 5, 6, 7, and 8; an integer selected from 0 to 9 refers to an integer selected from 0, 1, 2, 3, 4, 5, 6, 7, 8, and 9; and an integer selected from 0 to 10 refers to an integer selected from 0, 1, 2, 3, 4, 5, 6, 7, 8, 9, and 10.

As used herein, the term "substituted or unsubstituted" may indicate that one is substituted or unsubstituted with at least one substituent selected from the group consisting of deuterium, a halogen, a cyano group, a nitro group, an amine group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, a hydrocarbon ring group, an aryl group, and a heterocyclic group. In addition, each of the substituents presented as an example above may be substituted or unsubstituted. For example, a biphenyl group may be interpreted as an aryl group or as a phenyl group substituted with a phenyl group.

As used herein, the term "bonded to an adjacent group to form a ring" may indicate that one is bonded to an adjacent group to form a substituted or unsubstituted hydrocarbon ring or a substituted or unsubstituted heterocycle. The hydrocarbon ring may include an aliphatic hydrocarbon ring and/or an aromatic hydrocarbon ring. The heterocycle may include an aliphatic heterocycle and/or an aromatic heterocycle. The hydrocarbon ring and the heterocycle may each be monocyclic or polycyclic. In addition, the rings formed by adjacent groups being bonded to each other may be linked to another ring to form a spiro structure.

As used herein, the term "adjacent group" may refer to a substituent substituted for an atom which is directly linked to an atom substituted with a corresponding substituent, another substituent substituted for an atom which is substituted with a corresponding substituent, or a substituent sterically positioned at the nearest position to a corresponding substituent. For example, two methyl groups in 1,2-dimethylbenzene may be interpreted as mutually "adjacent groups", and two ethyl groups in 1,1-diethylcyclopentane may be interpreted as mutually "adjacent groups". In addition, two methyl groups in 4,5-dimethylphenanthrene may be interpreted as mutually "adjacent groups".

As used herein, examples of a halogen may include fluorine, chlorine, bromine, and iodine.

As used herein, an alkyl group may be linear or branched. The number of carbon atoms in the alkyl group may be 1 to 60, 1 to 50, 1 to 30, 1 to 20, 1 to 10, or 1 to 6. Examples of the alkyl group may include a methyl group, an ethyl group, an n-propyl group, an isopropyl group, an n-butyl group, a s-butyl group, a t-butyl group, an i-butyl group, a 2-ethylbutyl group, a 3,3-dimethylbutyl group, an n-pentyl group, an i-pentyl group, a neopentyl group, a t-pentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, an n-nonyl group, an n-decyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-icosyl group, a 2-ethylicosyl group, a 2-butylicosyl group, a 2-hexylicosyl group, a 2-octylicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, and/or the like, but embodiments of the present disclosure are not limited thereto.

As used herein, a cycloalkyl group may indicate a cyclic alkyl group. The number of carbon atoms in the cycloalkyl group may be 3 to 60, 3 to 50, 3 to 30, 3 to 20, or 3 to 10. Examples of the cycloalkyl group include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptyl group, and/or the like, but embodiments of the present disclosure are not limited thereto.

As used herein, an alkenyl group indicates a hydrocarbon group including at least one carbon-carbon double bond in the middle or end of an alkyl group having 2 or more carbon atoms. The alkenyl group may be linear or branched. The number of carbon atoms is not particularly limited, for example, may be 2 to 60, 2 to 30, 2 to 20, or 2 to 10. Examples of the alkenyl group include a vinyl group, a 1-butenyl group, a 1-pentenyl group, a 1,3-butadienyl group, a styrenyl group, a styryl vinyl group, and/or the like, but embodiments of the present disclosure are not limited thereto.

As used herein, an alkynyl group indicates a hydrocarbon group including at least one carbon-carbon triple bond in the middle or end of an alkyl group having 2 or more carbon atoms. The alkynyl group may be linear or branched. The number of carbon atoms is not particularly limited, for example, may be 2 to 30, 2 to 20, or 2 to 10. Examples of the alkynyl group may include an ethynyl group, a propynyl group, and/or the like, but embodiments of the present disclosure are not limited thereto.

As used herein, a hydrocarbon ring group indicates any functional group or substituent derived from an aliphatic hydrocarbon ring or an aromatic hydrocarbon ring. The hydrocarbon ring group may be a saturated hydrocarbon ring group having 6 to 60, 6 to 30, 5 to 30, or 5 to 20 ring-forming carbon atoms.

As used herein, an aryl group indicates any functional group or substituent derived from an aromatic hydrocarbon ring. The aryl group may be a monocyclic aryl group or a polycyclic aryl group. The number of ring-forming carbon atoms in the aryl group may be 6 to 60, 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, and/or the like, but embodiments of the present disclosure are not limited thereto.

As used herein, a fluorenyl group may be substituted, and two substituents may be bonded to each other to form a spiro structure. Examples that the fluorenyl group is substituted are as follows. However, embodiments of the present disclosure are not limited thereto.

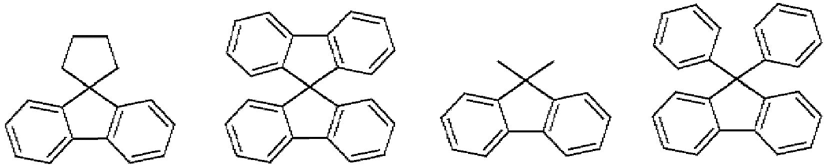

As used herein, a heterocyclic group indicates any functional group or substituent derived from a ring containing at least one of B, O, **N,** P, Si, or S as a hetero atom. The heterocyclic group includes an aliphatic heterocyclic group and/or an aromatic heterocyclic group. The aromatic heterocyclic group may be a heteroaryl group. The aliphatic heterocyclic group and the aromatic heterocyclic group may each be monocyclic or polycyclic.

As used herein, the heterocyclic group may contain at least one of B, O, **N,** P, Si, or S as a hetero atom. When the heterocyclic group contains two or more hetero atoms, the two or more hetero atoms may be the same as or different from each other. The heterocyclic group may be a monocyclic heterocyclic group or a polycyclic heterocyclic group, and may include a heteroaryl group. The number of ring-forming carbon atoms in the heterocyclic group may be 2 to 60, 2 to 30, 5 to 30, 2 to 20, or 2 to 10.

As used herein, the aliphatic heterocyclic group may contain at least one of B, O, **N,** P, Si, or S as a hetero atom. The number of ring-forming carbon atoms in the aliphatic heterocyclic group may be 2 to 60, 2 to 30, 2 to 20, or 2 to 10. Examples of the aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, and/or the like, but embodiments of the present disclosure are not limited to thereto.

As used herein, a heteroaryl group may include at least one of B, O, **N,** P, Si, or S as a hetero atom. When the heteroaryl group contains two or more hetero atoms, the two or more hetero atoms may be the same as or different from each other. The heteroaryl group may be a monocyclic heteroaryl group or a polycyclic heteroaryl group. The number of ring-forming carbon atoms in the heteroaryl group may be 2 to 60, 2 to 30, 2 to 20, or 2 to 10. Examples of the heteroaryl group may include a thiophene group, a furan group, a pyrrole group, an imidazole group, a pyridine group, a bipyridine group, a pyrimidine group, a triazine group, a triazole group, an acridyl group, a pyridazine group, a pyrazinyl group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, and/or the like, but embodiments of the present disclosure are not limited thereto.

As used herein, the above description of the aryl group may also apply to an arylene group, except that the arylene group is a divalent group. The above description of the heteroaryl group may also apply to a heteroarylene group, except that the heteroarylene group is a divalent group.

As used herein, a silyl group includes an alkyl silyl group and/or an aryl silyl group. Examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and/or the like, but embodiments of the present disclosure are not limited thereto.

As used herein, the number of carbon atoms in a carbonyl group is not particularly limited, but may be 1 to 40, 1 to 30, or 1 to 20. For example, the carbonyl group may have the following structure, but embodiments of the present disclosure are not limited thereto.

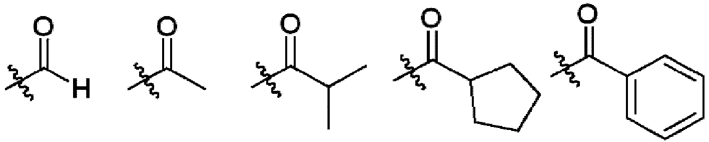

As used herein, the number of carbon atoms in a sulfinyl group or a sulfonyl group is not particularly limited, for example, may be 1 to 30. The sulfinyl group may include an alkyl sulfinyl group and/or an aryl sulfinyl group. The sulfonyl group may include an alkyl sulfonyl group and/or an aryl sulfonyl group.

As used herein, a thio group may include an alkyl thio group and/or an aryl thio group. The thio group may indicate one that a sulfur atom is bonded to the alkyl group or the aryl group defined above. Examples of the thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, and/or the like, but embodiments of the present disclosure are not limited to thereto.

As used herein, an oxy group may indicate one that an oxygen atom is bonded to the alkyl group or the aryl group defined above. The oxy group may include an alkoxy group and/or an aryl oxy group. The alkoxy group may be linear, branched, or cyclic. The number of carbon atoms in the alkoxy group is not particularly limited, but may be, for example, 1 to 20, or 1 to 10. Examples of the oxy group include methoxy, ethoxy, n-propoxy, isopropoxy, butoxy, pentyloxy, hexyloxy, octyloxy, nonyloxy, decyloxy, benzyloxy, and/or the like, but embodiments of the present disclosure are not limited thereto.

As used herein, a boron group may indicate one that a boron atom is bonded to the alkyl group or the aryl group defined above. The boron group includes an alkyl boron group and/or an aryl boron group. Examples of the boron group include a dimethylboron group, a diethylboron group, a t-butylmethylboron group, a diphenylboron group, a phenylboron group, and/or the like, but embodiments of the present disclosure are not limited thereto.

As used herein, the number of carbon atoms in an amine group is not particularly limited, but may be 1 to 50, 1 to 30, or 1 to 20. The amine group may include an alkyl amine group and/or an aryl amine group. Examples of the amine group may include a methylamine group, a dimethylamine group, a phenylamine group, a diphenylamine group, a naphthylamine group, a 9-methyl-anthracenylamine group, and/or the like, but embodiments of the present disclosure are not limited thereto.

As used herein, the above-described examples of the alkyl group also apply to an alkyl group from an alkylthio group, an alkyl sulfoxy group, an alkylaryl group, an alkylamino group, an alkyl boron group, an alkyl silyl group, and an alkyl amine group.

As used herein, the above-described examples of the aryl group also apply to an aryl group from an aryloxy group, an arylthio group, an aryl sulfoxy group, an arylamino group, an aryl boron group, an aryl silyl group, and an aryl amine group.

As used herein, the phosphine oxide group may be substituted with, for example, at least one of the alkyl groups or aryl groups described above.

As used herein, the phosphine sulfide group may be substituted with, for example, at least one of the alkyl groups or aryl groups described above.

As used herein, a direct linkage may indicate a single bond. As used herein, 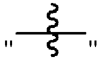 and "-*" each indicate a position to be connected.

Hereinafter, embodiments of the disclosure will be described with reference to the accompanying drawings.

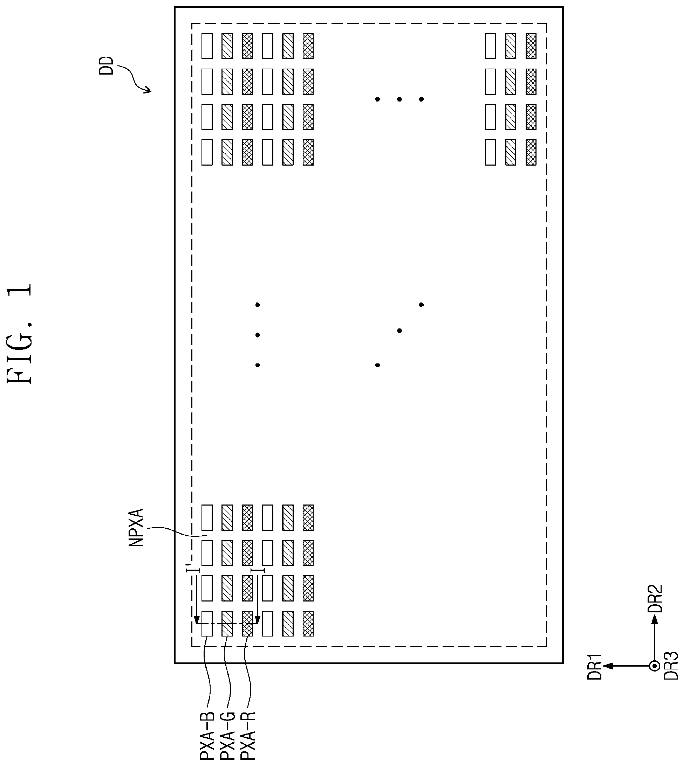

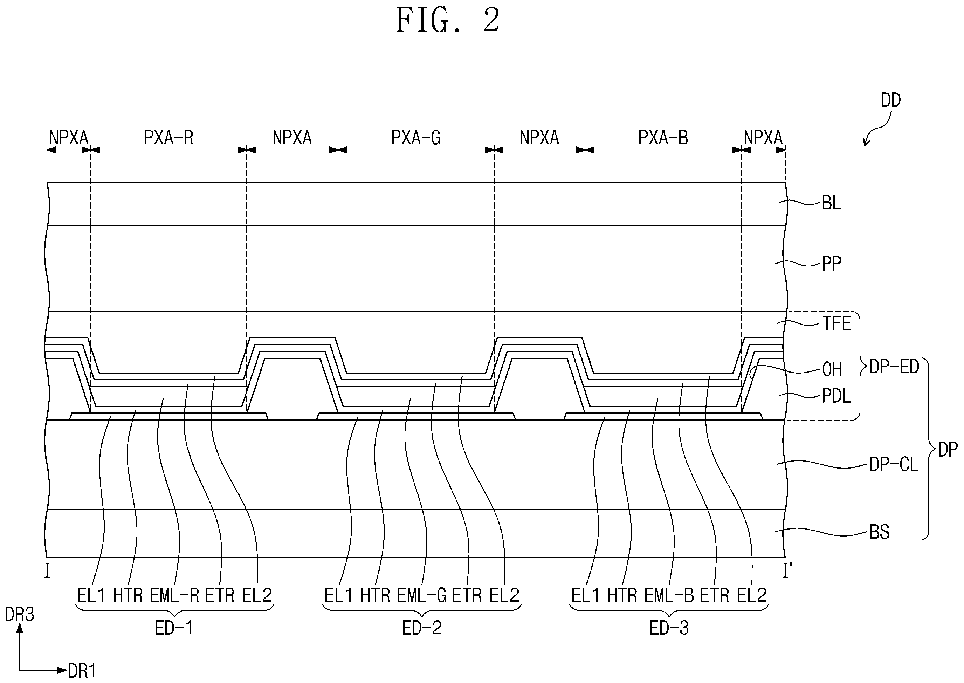

FIG. 1 is a plan view showing a display device DD according to one or more embodiments of the present disclosure. FIG. 2 is a cross-sectional view showing a display device DD according to one or more embodiments of the present disclosure. FIG. 2 is a cross-sectional view showing a portion corresponding to the line I-I' of the display device DD of FIG. 1 according to one or more embodiments.

The display device DD may include a display panel DP and an optical layer PP arranged on the display panel DP. The display panel DP may include light emitting elements ED-1, ED-2, and ED-3. The display device DD may include a plurality of light emitting elements ED-1, ED-2, and ED-3. The optical layer PP may be arranged on the display panel DP to control reflected light in the display panel DP due to external light. The optical layer PP may include, for example, a polarizing layer and/or a color filter layer. In one or more embodiments, the optical layer PP may not be provided in the display device DD.

A base substrate BL may be arranged on the optical layer PP. The base substrate BL may be a member providing a base surface on which the optical layer PP is arranged. The base substrate BL may be a glass substrate, a metal substrate, a plastic substrate, and/or the like. However, embodiments of the present disclosure are not limited thereto, and the base substrate BL may be an inorganic layer, an organic layer, or a composite material layer. In one or more embodiments, the base substrate BL may not be provided.

The display device DD according to one or more embodiments may further include a filling layer. The filling layer may be arranged between a display element layer DP-ED and the base substrate BL. The filling layer may be an organic material layer. The filling layer may include at least one of an acrylic resin, a silicone-based resin, or an epoxy-based resin.

The display panel DP may include a base layer BS, a circuit layer DP-CL provided on the base layer BS, and a display element layer DP-ED. The display element layer DP-ED may include pixel defining films PDL, a plurality of light emitting elements ED-1, ED-2, and ED-3 arranged between the pixel defining films PDL, and an encapsulation layer TFE arranged on the plurality of light emitting elements ED-1, ED-2, and ED-3.

The base layer BS may be a member providing a base surface on which the display element layer DP-ED is arranged. The base layer BS may be a glass substrate, a metal substrate, a plastic substrate, and/or the like. However, embodiments of the present disclosure are not limited thereto, and the base layer BS may be an inorganic layer, an organic layer, or a composite material layer.

In one or more embodiments, the circuit layer DP-CL may be arranged on the base layer BS, and the circuit layer DP-CL may include a plurality of transistors. The transistors may each include a control electrode, an input electrode, and an output electrode. For example, in one or more embodiments, the circuit layer DP-CL may include switching transistor(s) and driving transistor(s) for driving the light emitting elements ED-1, ED-2 and ED-3 of the display element layer DP-ED.

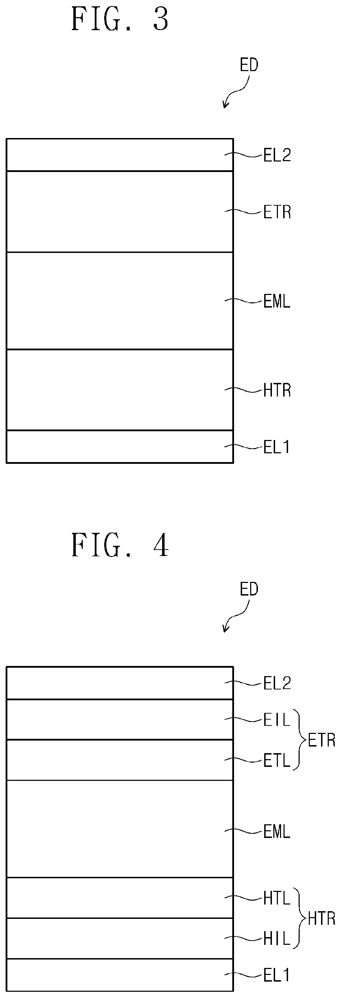

The light emitting elements ED-1, ED-2, and ED-3 may each have a structure of one of light emitting elements ED of embodiments according to FIGS. 3 to 6, which will be described later. The light emitting elements ED-1, ED-2, and ED-3 may each include a first electrode EL1, a hole transport region HTR, respective emission layers EML-R, EML-G, and EML-B, an electron transport region ETR, and a second electrode EL2.

FIG. 2 shows one or more embodiments in which the respective emission layers EML-R, EML-G, and EML-B of the light emitting elements ED-1, ED-2, and ED-3 are arranged in openings OH defined in the pixel defining films PDL, and the hole transport region HTR, the electron transport region ETR, and the second electrode EL2 are each provided as a common layer throughout the light emitting elements ED-1, ED-2, and ED-3. However, embodiments of the present disclosure are not limited thereto, for example, in one or more embodiments, the hole transport region HTR and the electron transport region ETR may each be provided to be patterned inside the openings OH defined in the pixel defining films PDL. For example, in one or more embodiments, the hole transport region HTR, the respective emission layers EML-R, EML-G, and EML-B, and the electron transport region ETR of the light emitting elements ED-1, ED-2, and ED-3 may be patterned and provided through an inkjet printing method.

The encapsulation layer TFE may cover the light emitting elements ED-1, ED-2, and ED-3. The encapsulation layer TFE may seal the light emitting elements ED-1 ED-2, and ED-3 of the display element layer DP-ED. The encapsulation layer TFE may be a thin film encapsulation layer. The encapsulation layer TFE may be a single layer or a stack layer of a plurality of layers. The encapsulation layer TFE may include at least one insulating layer. The encapsulation layer TFE according to one or more embodiments may include at least one inorganic film (hereinafter, an encapsulation inorganic film). In addition, the encapsulation layer TFE according to one or more embodiments may include at least one organic film (hereinafter, an encapsulation organic film) and at least one encapsulation inorganic film.

The encapsulation inorganic film protects the display element layer DP-ED from moisture/oxygen, and the encapsulation organic film protects the display element layer DP-ED from foreign substances such as dust particles. The encapsulation inorganic film may include silicon nitride, silicon oxy nitride, silicon oxide, titanium oxide, aluminium oxide, and/or the like, but embodiments of the present disclosure are not particularly limited thereto. The encapsulation organic film may include an acrylic compound, an epoxy-based compound, and/or the like. In one or more embodiments, the encapsulation organic film may include a photopolymerizable organic material, but embodiments of the present disclosure are not particularly limited thereto.

The encapsulation layer TFE may be arranged on the second electrode EL2, and may be arranged to fill the openings OH.

Referring to FIG. 1 and FIG. 2, the display device DD may include non-light emitting regions NPXA and light emitting regions PXA-R, PXA-G, and PXA-B. The light emitting regions PXA-R, PXA-G, and PXA-B may each be a region that emits light generated from each of the light emitting elements ED-1, ED-2, and ED-3, correspondingly. The light emitting regions PXA-R, PXA-G, and PXA-B may be spaced apart from one another if (e.g., when) viewed on a plane (e.g., in plan view).

The light emitting regions PXA-R, PXA-G, and PXA-B may each be a region separated by the pixel defining films PDL. The non-light emitting regions NPXA may be regions between neighboring light emitting regions PXA-R, PXA-G, and PXA-B, and may correspond to the pixel defining films PDL. In one or more embodiments, as used herein, the light emitting regions PXA-R, PXA-G, and PXA-B may each correspond to a pixel. The pixel defining films PDL may separate the light emitting elements ED-1, ED-2, and ED-3. The respective emission layers EML-R, EML-G, and EML-B of the light emitting elements ED-1, ED-2, and ED-3 may be arranged in the openings OH defined by the pixel defining films PDL and thus be separated.

The light emitting regions PXA-R, PXA-G, and PXA-B may be divided into a plurality of groups according to the color of light generated from the light emitting elements ED-1, ED-2, and ED-3. In the display device DD of one or more embodiments shown in FIG. 1 and FIG. 2, three light emitting regions PXA-R, PXA-G, and PXA-B which emit red light, green light, and blue light, respectively, are shown as an example. For example, in one or more embodiments, the display device DD may include a red light emitting region PXA-R, a green light emitting region PXA-G, and a blue light emitting region PXA-B, which are distinct from one another.

In the display device DD according to one or more embodiments, the plurality of light emitting elements ED-1, ED-2, and ED-3 may be to emit light having different wavelength ranges. For example, in one or more embodiments, the display device DD may include a first light emitting element ED-1 that emits red light, a second light emitting element ED-2 that emits green light, and a third light emitting element ED-3 that emits blue light. For example, the red light emitting region PXA-R, the green light emitting region PXA-G, and the blue light emitting region PXA-B of the display device DD may correspond to the first light emitting element ED-1, the second light emitting element ED-2, and the third light emitting element ED-3, respectively.

However, embodiments of the present disclosure are not limited thereto, and the first to third light emitting elements ED-1, ED-2, and ED-3 may be to emit light in substantially the same wavelength range, or at least one light emitting element emits light in a different wavelength range from the others. For example, in one or more embodiments, the first to third light emitting elements ED-1, ED-2, and ED-3 may all emit blue light.

The light emitting regions PXA-R, PXA-G, and PXA-B in the display device DD according to one or more embodiments may be arranged in the form of a stripe. Referring to FIG. 1, a plurality of red light emitting regions PXA-R may be arranged with each other along a second direction axis DR2, a plurality of green light emitting regions PXA-G may be arranged with each other along the second direction axis DR2, and a plurality of blue light emitting regions PXA-B may be arranged with each other along the second direction axis DR2. In one or more embodiments, the red light emitting region PXA-R, the green light emitting region PXA-G, and the blue light emitting region PXA-B may be alternately arranged in that order along a first direction axis DR1.

FIG. 1 and FIG. 2 show that the light emitting regions PXA-R, PXA-G, and PXA-B are all similar in size, but embodiments of the present disclosure are not limited thereto, and the light emitting regions PXA-R, PXA-G, and PXA-B may be different in size from one another according to wavelength range of emitted light. In this regard, the areas of the light emitting regions PXA-R, PXA-G, and PXA-B may indicate areas if (e.g., when) viewed on a plane defined by the first direction axis DR1 and the second direction axis DR2 (i.e., areas in plan view).

In one or more embodiments, the arrangement of the light emitting regions PXA-R, PXA-G, and PXA-B is not limited to what is shown in FIG. 1, and the order in which the red light emitting region PXA-R, the green light emitting region PXA-G, and the blue light emitting region PXA-B are arranged comes with varied combination according to display quality characteristics desired or required for the display device DD. For example, in one or more embodiments, the light emitting regions PXA-R, PXA-G, and PXA-B may be arranged in the form of a pentile (for example, an RGBG matrix, an RGBG structure, or an RGBG matrix structure) (PENTILE^{®}) or a diamond (e.g., red, green, and blue (RGB) light-emitting regions arranged in the shape of diamonds) (Diamond Pixel^{™}). PENTILE^{®} is a duly registered trademark of Samsung Display Co., Ltd. Diamond Pixel^{™} is a trademark of Samsung Display Co., Ltd.

In one or more embodiments, the area of each of the light emitting regions PXA-R, PXA-G, and PXA-B may be different in size from one another. For example, in one or more embodiments, the green light emitting region PXA-G may be smaller than the blue light emitting region PXA-B in size, but embodiments of the present disclosure are not limited thereto.

Hereinafter, FIGS. 3 to 6 are each a cross-sectional view schematically showing a light emitting element according to one or more embodiments of the present disclosure. A light emitting element ED according to one or more embodiments may include a first electrode EL1, a hole transport region HTR, an emission layer EML, an electron transport region ETR, and a second electrode EL2, which are sequentially stacked (e.g., in the stated order).

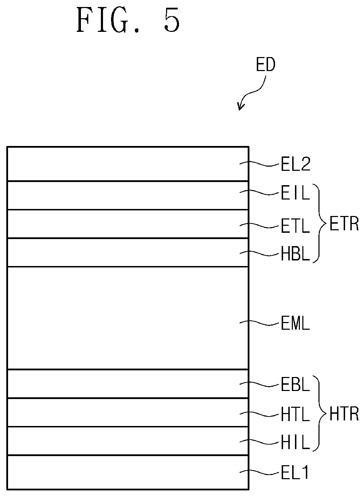

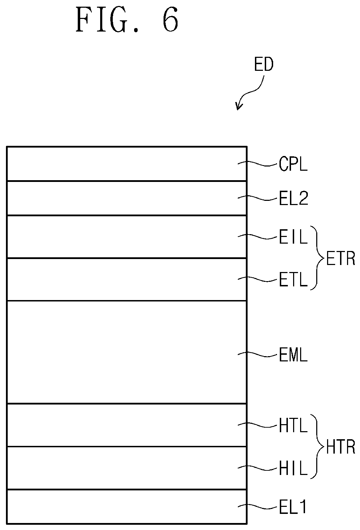

FIG. 4 shows, compared with FIG. 3, a cross-sectional view of a light emitting element ED of one or more embodiments in which the hole transport region HTR includes a hole injection layer HIL and a hole transport layer HTL, and the electron transport region ETR includes an electron injection layer EIL and an electron transport layer ETL. In addition, FIG. 5 shows, compared with FIG. 3, a cross-sectional view of a light emitting element ED of one or more embodiments, in which the hole transport region HTR includes a hole injection layer HIL, a hole transport layer HTL, and an electron blocking layer EBL, and the electron transport region ETR includes an electron injection layer EIL, an electron transport layer ETL, and a hole blocking layer HBL. FIG. 6 shows, compared with FIG. 4, a cross-sectional view of a light emitting element ED of one or more embodiments, in which a capping layer CPL arranged on the second electrode EL2 is provided.

The first electrode EL1 has conductivity (e.g., is an electron conductor). The first electrode EL1 may be formed of a metal material, a metal alloy, or a conductive compound. The first electrode EL1 may be an anode or a cathode. However, embodiments of the present disclosure are not limited thereto. In one or more embodiments, the first electrode EL1 may be a pixel electrode. The first electrode EL1 may be a transmissive electrode, a transflective electrode, or a reflective electrode. The first electrode EL1 may include at least one selected from among silver (Ag), magnesium (Mg), copper (Cu), aluminium (Al), platinum (Pt), palladium (Pd), gold (Au), nickel (Ni), neodymium (Nd), iridium (Ir), chromium (Cr), lithium (Li), calcium (Ca), lithium fluoride (LiF), molybdenum (Mo), titanium (Ti), tungsten (W), indium (In), tin (Sn), and zinc (Zn), at least two compounds selected therefrom, two or more mixtures selected therefrom, or an oxide thereof.

If (e.g., when) the first electrode EL1 is a transmissive electrode, the first electrode EL1 may include a transparent metal oxide such as indium tin oxide (ITO), indium zinc oxide (IZO), zinc oxide (ZnO), and indium tin zinc oxide (ITZO). If (e.g., when) the first electrode EL1 is a transflective electrode or a reflective electrode, the first electrode EL1 may include Ag, Mg, Cu, Al, Pt, Pd, Au, Ni, Nd, Ir, Cr, Li, Ca, LiF/Ca (a stack structure of LiF and Ca), LiF/Al (a stack structure of LiF and Al), Mo, Ti, W, or a compound thereof or a mixture thereof (e.g., a mixture of Ag and Mg). In one or more embodiments, the first electrode EL1 may have a multilayer structure including a reflective film or a transflective film formed of one or more of the above-described materials, and a transparent conductive film formed of indium tin oxide (ITO), indium zinc oxide (IZO), zinc oxide (ZnO), indium tin zinc oxide (ITZO), and/or the like. For example, in one or more embodiments, the first electrode EL1 may have a three-layer structure of ITO/Ag/ITO, but embodiments of the present disclosure are not limited thereto. In one or more embodiments, the first electrode EL1 may include one of the above-described metal materials, a combination of two or more metal materials selected from among the above-described metal materials, or an oxide of the above-described metal materials. The first electrode EL1 may have a thickness of about 700 angstroms (Å) to about 10000 Å. For example, in one or more embodiments, the first electrode EL1 may have a thickness of about 1000 Å to about 3000 Å.

The light emitting element ED of one or more embodiments includes a monoamine compound of one or more embodiments of the present disclosure. In one or more embodiments, the hole transport region HTR includes a monoamine compound of one or more embodiments. In one or more embodiments, at least one of the hole injection layer HIL, the hole transport layer HTL, or the electron blocking layer EBL may include a monoamine compound of one or more embodiments. For example, in one or more embodiments, the hole transport layer HTL may include a monoamine compound of one or more embodiments of the present disclosure.

The monoamine compound of one or more embodiments may include only one amine group. As used herein, substituents (e.g., aryl groups) bonded to an amine group may be bonded to form a ring (e.g., carbazole groups), and these are not included in the amine group used herein, but these (e.g., carbazole groups) are included in heteroaryl groups. A ring group containing a nitrogen atom as a ring-forming atom (e.g., a pyridine group, a pyrimidine group, a triazine group, a carbazole group, and/or the like) is not included in an amine group and is included in a heterocyclic group.

The monoamine compound of one or more embodiments includes first to third substituents directly or indirectly bonded to the amine group of the monoamine compound. The first substituent is a 3,5-dinaphthalenylphenyl group, and the dinaphthalenylphenyl group is a phenyl group substituted with two naphthalenyl groups. The second substituent is a 2-naphthalenyl group. The third substituent is an unsubstituted phenyl group, an unsubstituted naphthalenyl group, a naphthalenyl group substituted with a phenyl group, an unsubstituted phenanthrenyl group, a phenanthrenyl group substituted with a phenyl group, an unsubstituted dibenzofuranyl group, an unsubstituted dibenzothiophenyl group, an unsubstituted carbazolyl group, a carbazolyl group substituted with a phenyl group, an unsubstituted benzonaphthofuranyl group, or an unsubstituted benzonaphthothiophenyl group, Accordingly, the monoamine compound of one or more embodiments may exhibit excellent or suitable hole transport properties and excellent or suitable material stability, and the light emitting element ED of one or more embodiments including the monoamine compound may exhibit high luminous efficiency and long-life characteristics.

According to one or more embodiments of the present disclosure, the light emitting element ED includes a monoamine compound of one or more embodiments. The monoamine compound of one or more embodiments is represented by Formula 1.

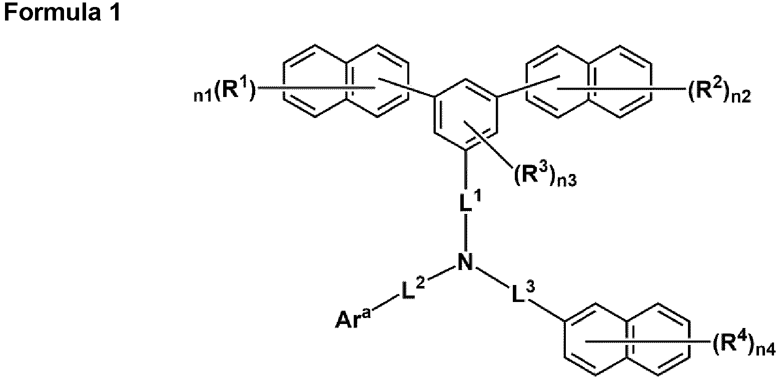

In Formula 1, a naphthalenyl group containing R¹, a naphthalenyl group containing R², and a phenyl group containing R³ may correspond to the first substituent described above (i.e., the dinaphthalenylphenyl group). A naphthalenyl group containing R⁴ may correspond to the second substituent described above. Ar^{a} may correspond to the third substituent described above.

In Formula 1, L¹ is a direct linkage, a substituted or unsubstituted arylene group having 6 to 30 ring-forming carbon atoms, or a substituted or unsubstituted heteroarylene group having 5 to 30 ring-forming carbon atoms. For example, in one or more embodiments, L¹ may be a direct linkage.

L² and L³ are each independently a direct linkage, an unsubstituted phenylene group, a phenylene group substituted with deuterium, or a phenylene group substituted with a phenyl group. For example, in one or more embodiments, L² may be a direct linkage or be represented by any one selected from among L2-1 to L2-4. L³ may be a direct linkage or be represented by any one selected from among L2-1 to L2-4. When L³ is represented as L2-1, any hydrogen in L2-1 may be optionally substituted with deuterium.

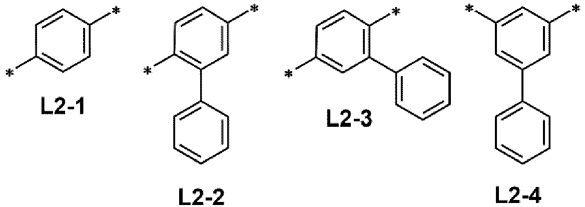

In Formula 1, Ar^{a} is an unsubstituted phenyl group, an unsubstituted naphthalenyl group, a naphthalenyl group substituted with a phenyl group, an unsubstituted phenanthrenyl group, a phenanthrenyl group substituted with a phenyl group, an unsubstituted dibenzofuranyl group, an unsubstituted dibenzothiophenyl group, an unsubstituted carbazolyl group, a carbazolyl group substituted with a phenyl group, an unsubstituted benzonaphthofuranyl group, or an unsubstituted benzonaphthothiophenyl group. For example, in one or more embodiments, Ar^{a} may be represented by any one selected from among AR-1 to AR-38.

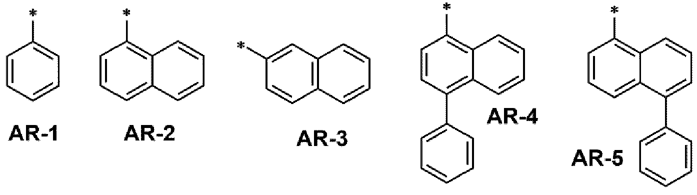

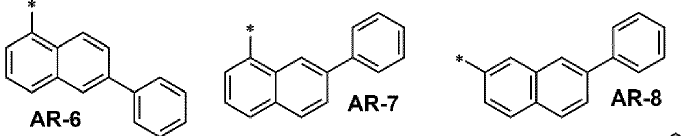

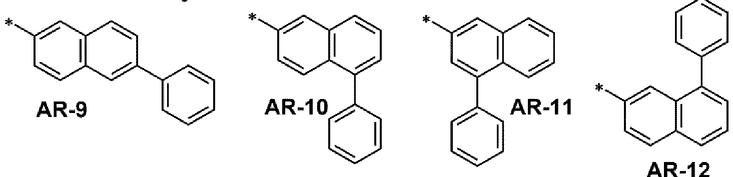

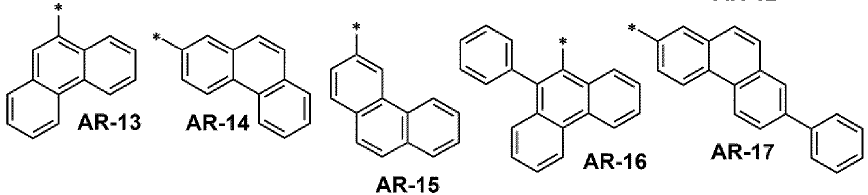

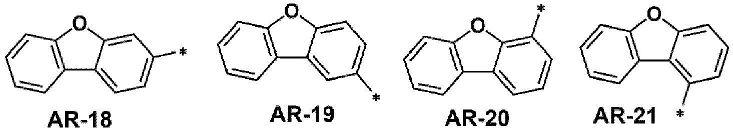

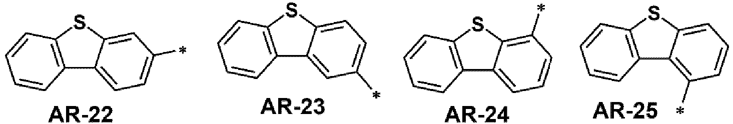

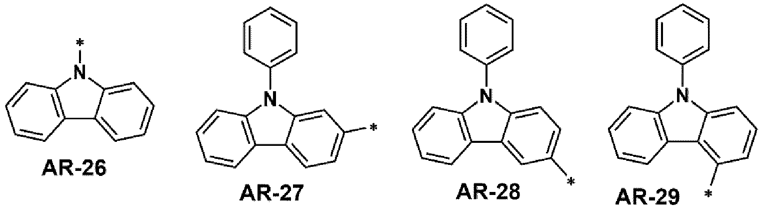

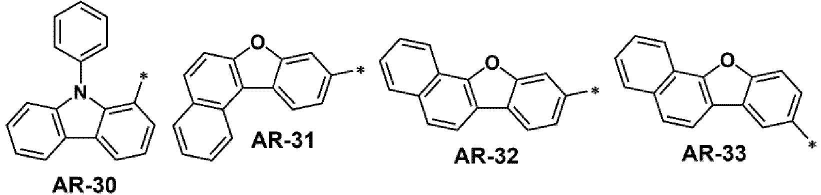

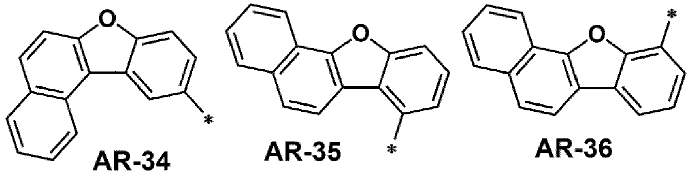

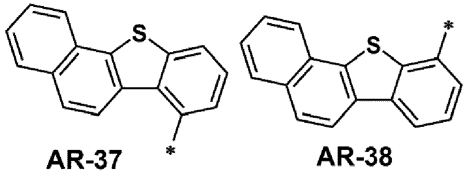

In Formula 1, R¹ to R³ are each independently hydrogen, deuterium, a halogen, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms. For example, in one or more embodiments, R¹ to R³ may each independently be hydrogen, deuterium, fluorine, or an unsubstituted phenyl group.

In Formula 1, R⁴ is hydrogen, deuterium, a halogen, a substituted or unsubstituted aryl group having 6 to 30 ring-forming carbon atoms, or a substituted or unsubstituted heteroaryl group having 5 to 30 ring-forming carbon atoms. For example, in one or more embodiments, R⁴ may be hydrogen, deuterium, fluorine, an unsubstituted phenyl group, or an unsubstituted dibenzofuranyl group.

In Formula 1, in one or more embodiments, the naphthalenyl group containing R⁴ may be represented by any one selected from among R4-1 to R4-6. In R4-5, D is deuterium.

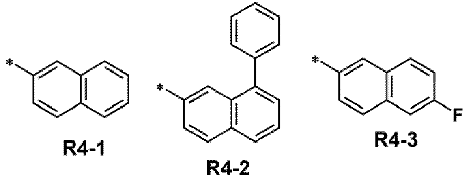

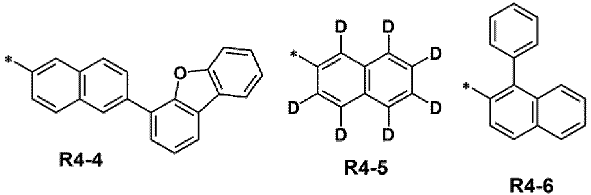

In Formula 1, n1, n2, and n4 are each independently an integer of 0 to 7. n3 is an integer of 0 to 3.

When n1 is an integer of 2 or greater, a plurality of R¹'s may all be the same or at least one thereof may be different from the others. The embodiment in which n1 is 0 may be the same as an embodiment in which n1 is 7 and seven R¹'s are all hydrogens. When n2 is an integer of 2 or greater, a plurality of R²'s may all be the same or at least one thereof may be different from the others. The embodiment in which n2 is 0 may be the same as an embodiment in which n2 is 7 and seven R²'s are all hydrogens.

When n3 is an integer of 2 or greater, a plurality of R³'s may all be the same or at least one thereof may be different from the others. The embodiment in which n3 is 0 may be the same as an embodiment in which n3 is 3 and three R³'s are all hydrogens. When n4 is an integer of 2 or greater, a plurality of R⁴'s may all be the same or at least one thereof may be different from the others. The embodiment in which n4 is 0 may be the same as an embodiment in which n4 is 7 and seven R⁴'s are all hydrogens.

In one or more embodiments, the monoamine compound represented by Formula 1 may include a chemical structure in which any hydrogen atom is optionally substituted with deuterium. For example, in one or more embodiments, in Formula 1, R⁴ may be deuterium, and the monoamine compound of one or more embodiments may include a naphthalenyl group substituted with deuterium. In one or more embodiments, in Formula 1, L³ may be a phenylene group substituted with deuterium, and the monoamine compound of one or more embodiments may include a phenylene group substituted with deuterium. However, this is presented as an example, and embodiments of the present disclosure are not limited thereto.

In one or more embodiments, the monoamine compound represented by Formula 1 may be represented by any one selected from among Formulas 1-A1 to 1-A3. Formulas 1-A1 to 1-A3 may show embodiments in which a bonding position of a naphthalenyl group including R¹ and a bonding position of a naphthalenyl group including R² in Formula 1 are specified and L¹ is a direct linkage. In Formulas 1-A1 to 1-A3, the description of Formula 1 also applies to L², L³, Ar^{a}, R¹ to R⁴, and n1 to n4.

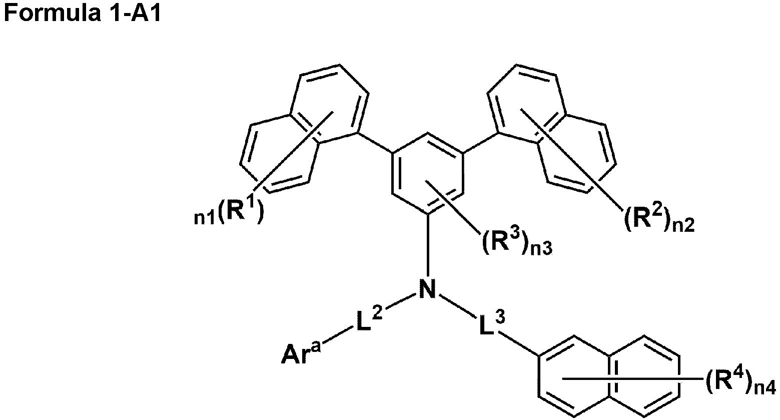

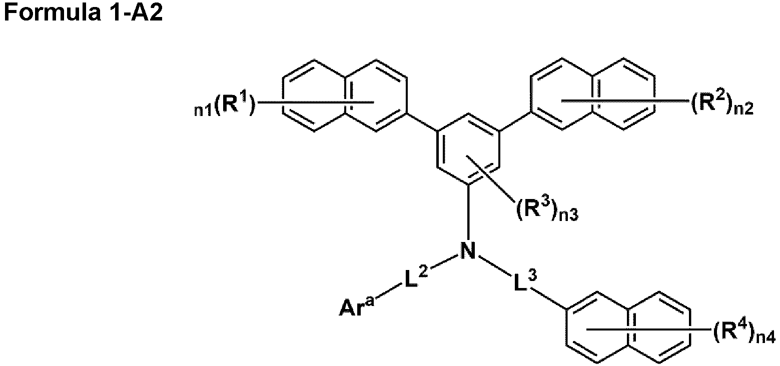

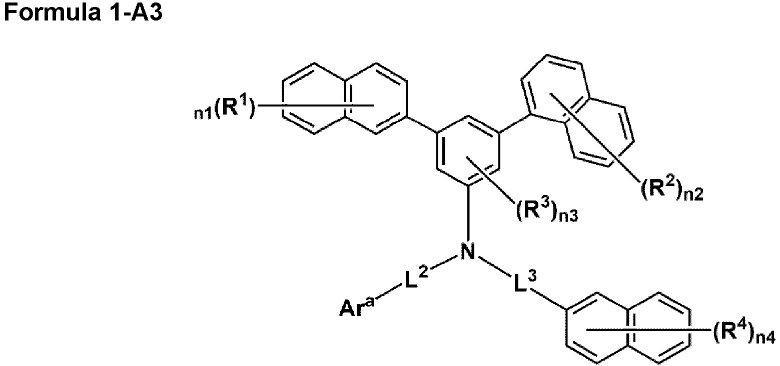

In one or more embodiments, the monoamine compound represented by Formula 1 may be represented by any one selected from among Formulas 1-B1 to 1-B3. Formulas 1-B1 to 1-B3 may indicate embodiments in which L³ in Formula 1 is specified.

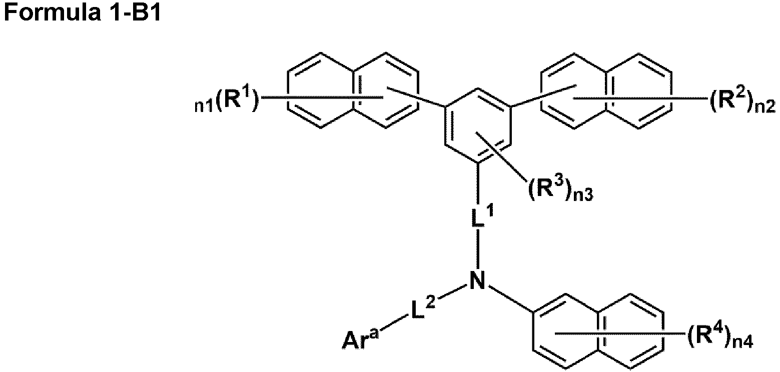

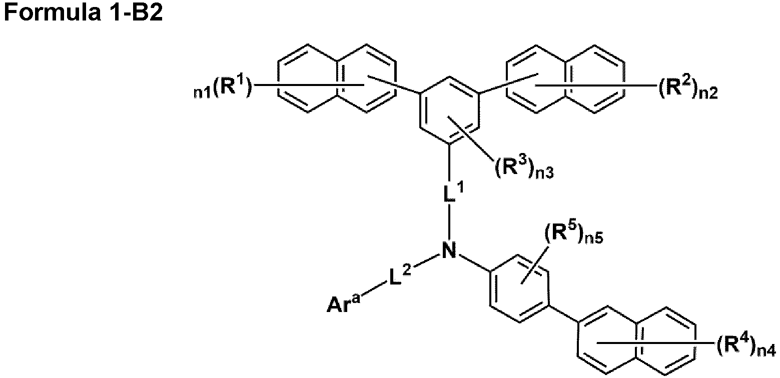

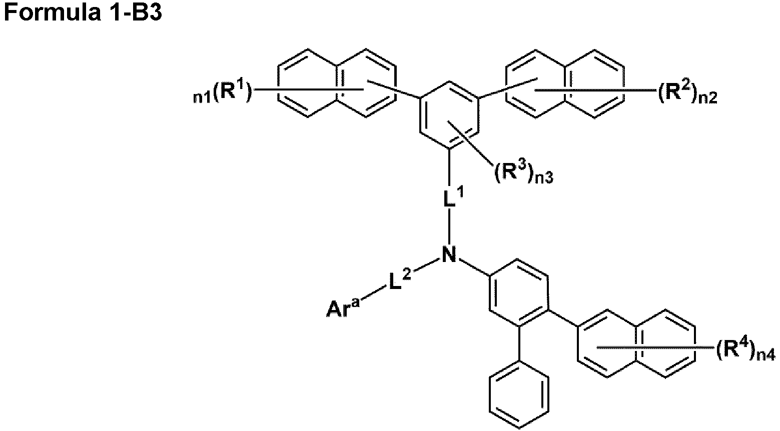

In Formulas 1-B1 to 1-B3, the description of Formula 1 also applies to L¹, L², Ar^{a}, R¹ to R⁴, and n1 to n4. In Formula 1-B2, n5 is an integer of 0 to 4, and R⁵ is hydrogen or deuterium. When n5 is an integer of 2 or greater, a plurality of R⁵'s may all be the same or at least one thereof may be different from the others. The embodiment in which n5 is 0 may be the same as an embodiment in which n5 is 4 and four R⁵'s are all hydrogens.

In one or more embodiments, the monoamine compound represented by Formula 1 may be any one selected from among compounds of Compound Group 1. The monoamine compound of one or more embodiments may be any one selected from among the compounds of Compound Group 1. The light emitting element ED of one or more embodiments may include at least one selected from among the compounds of Compound Group 1. In Compound Group 1, D is deuterium.

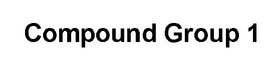

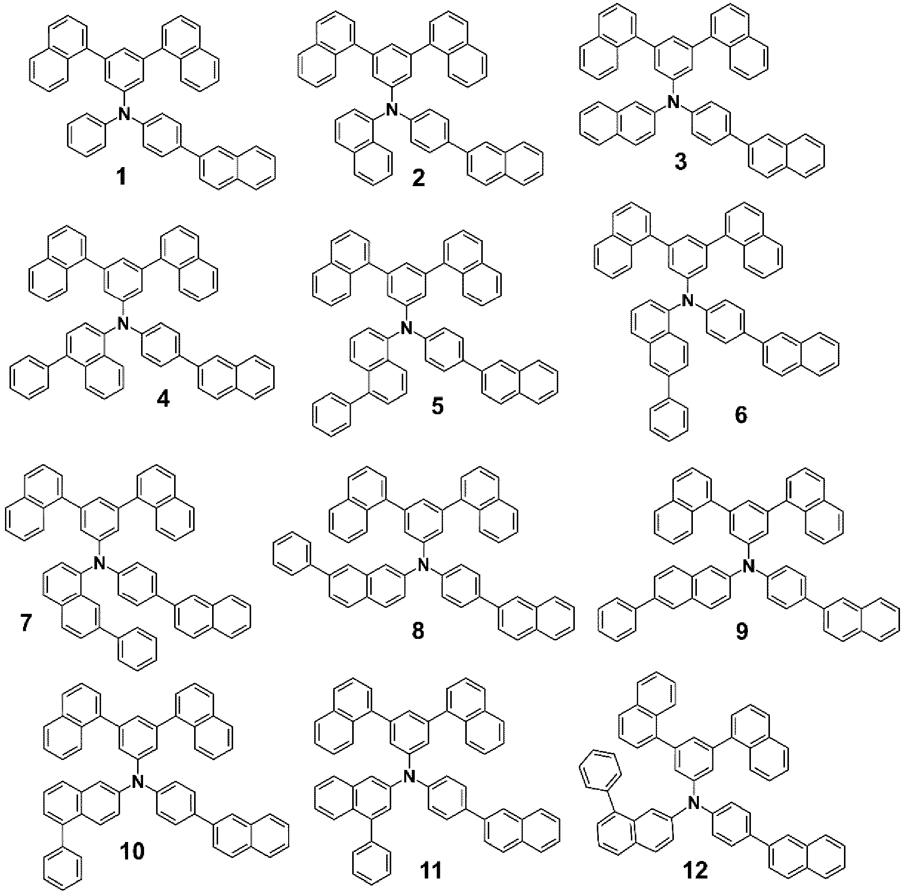

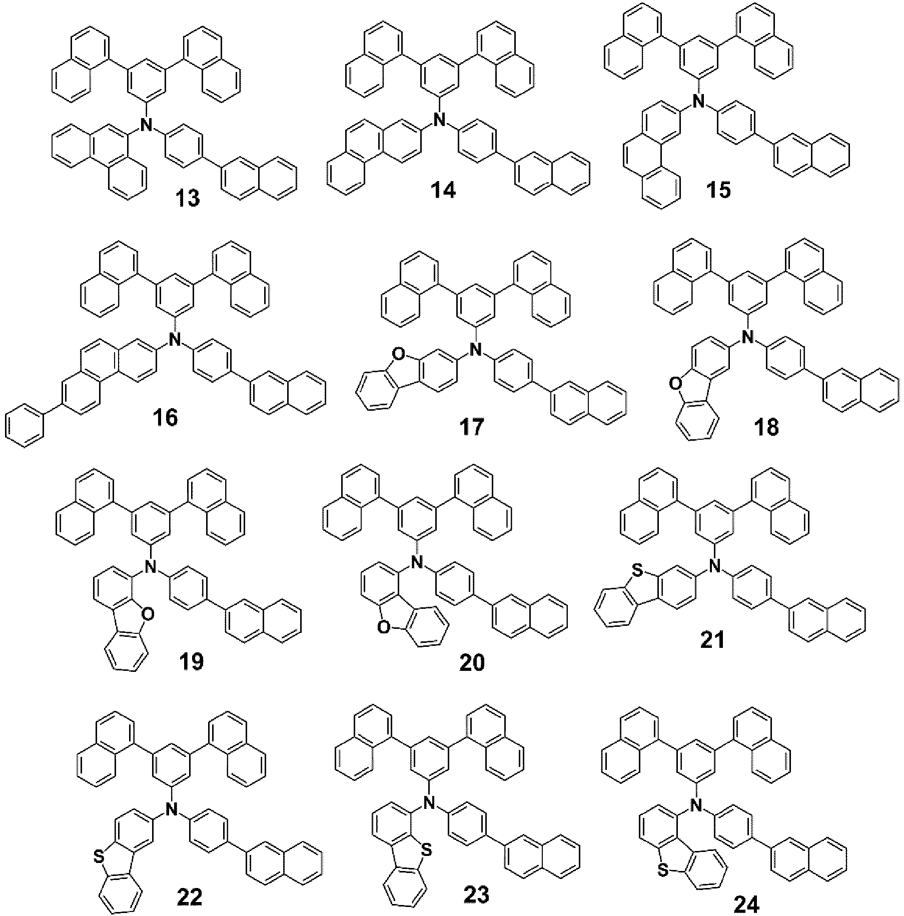

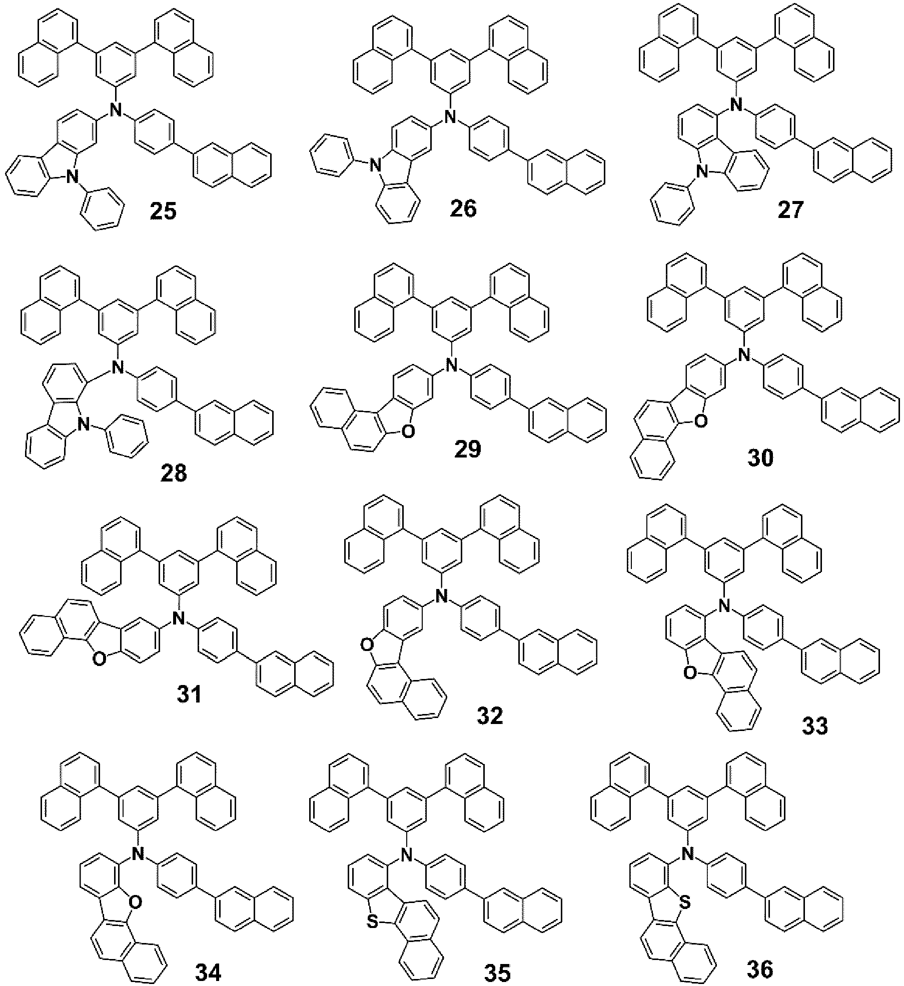

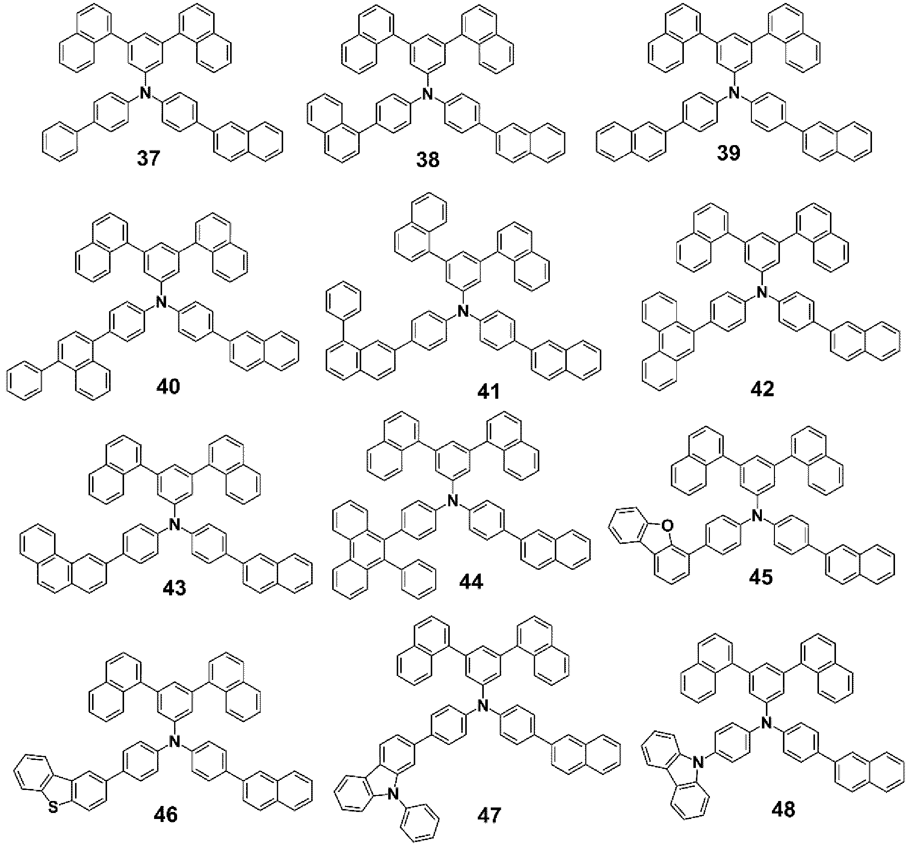

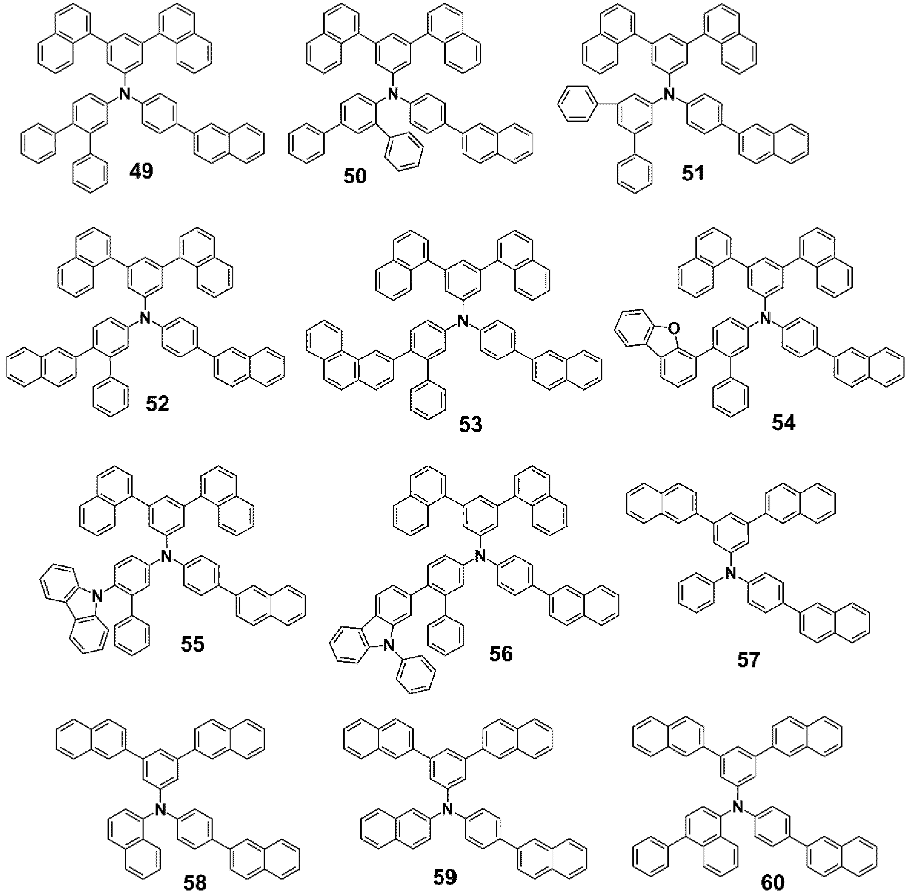

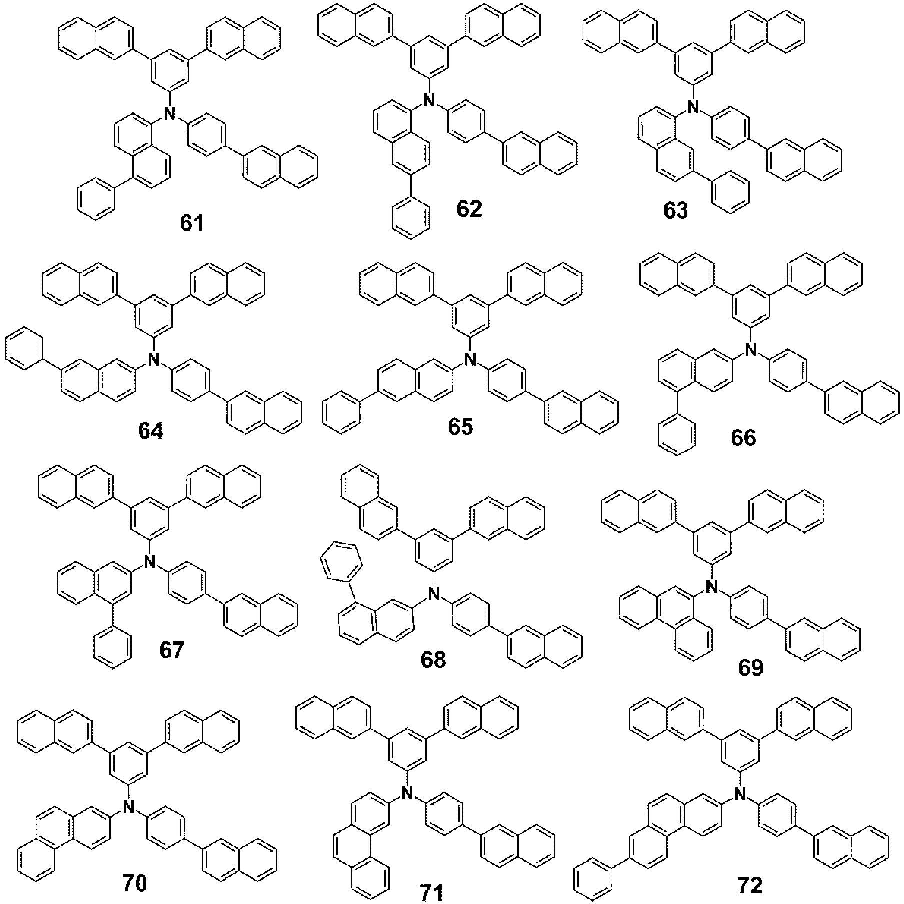

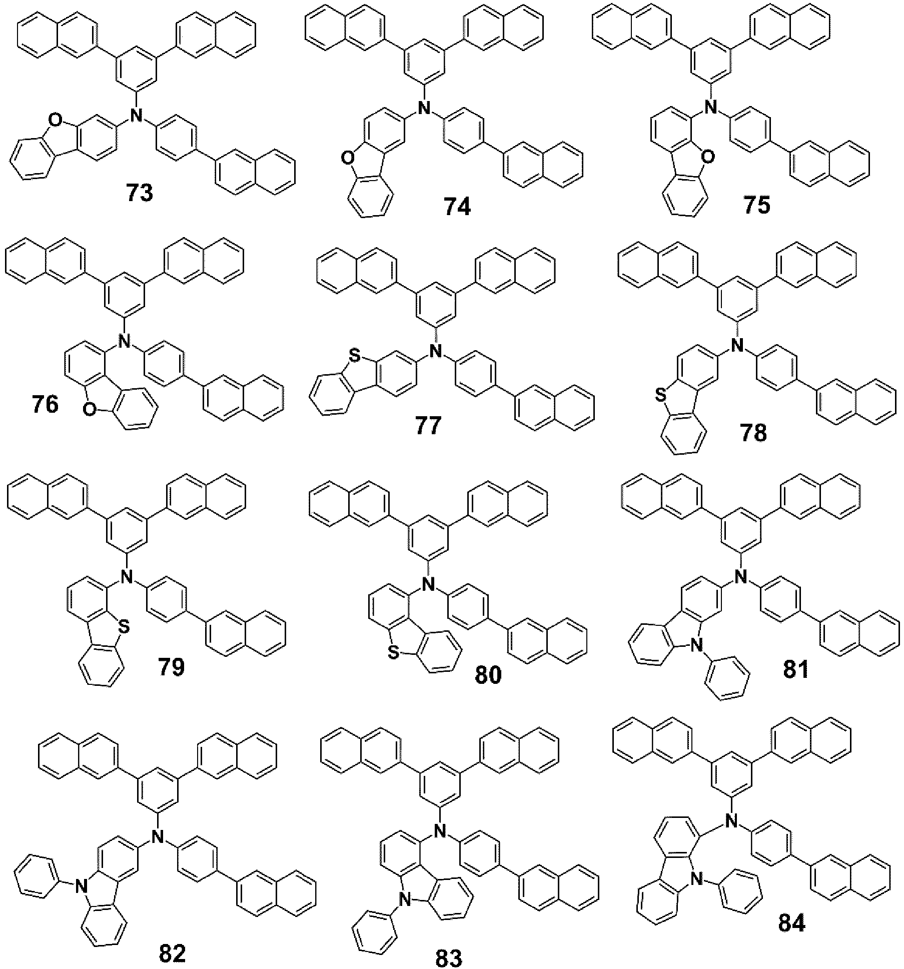

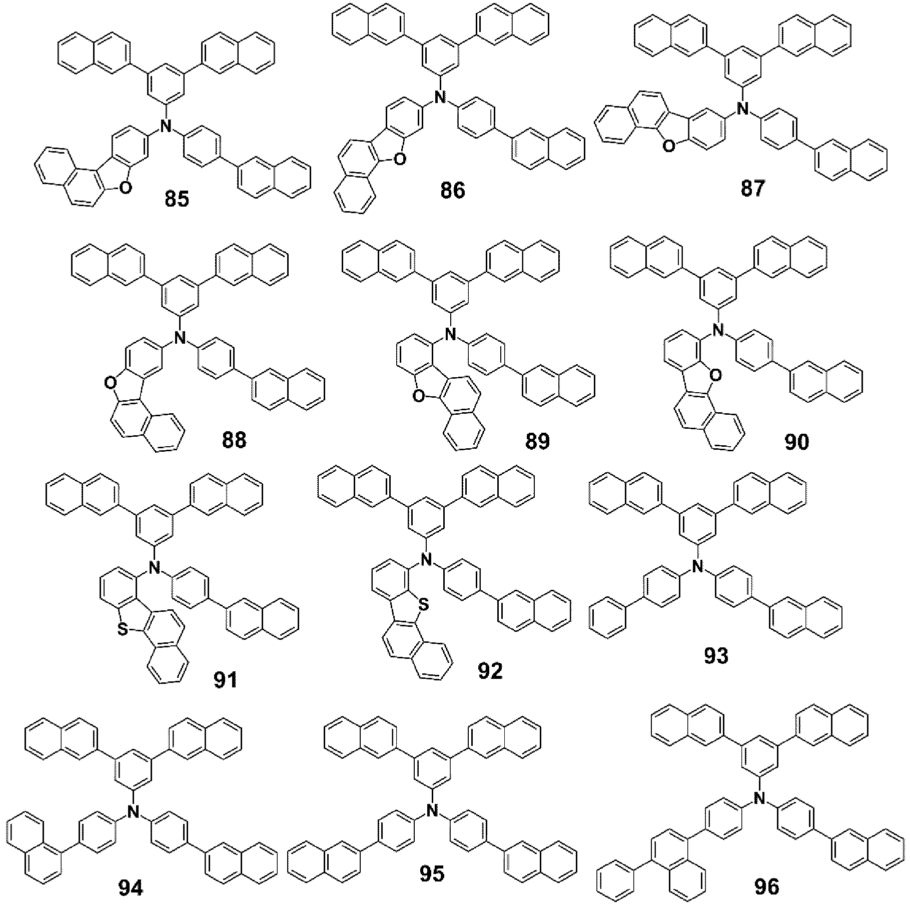

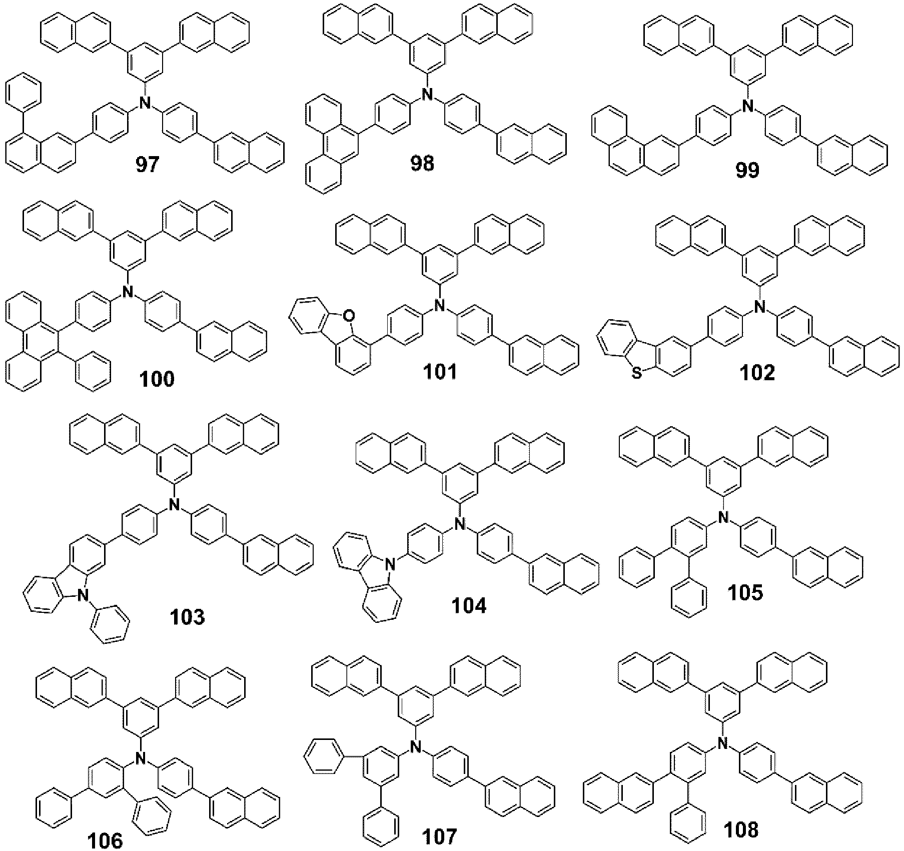

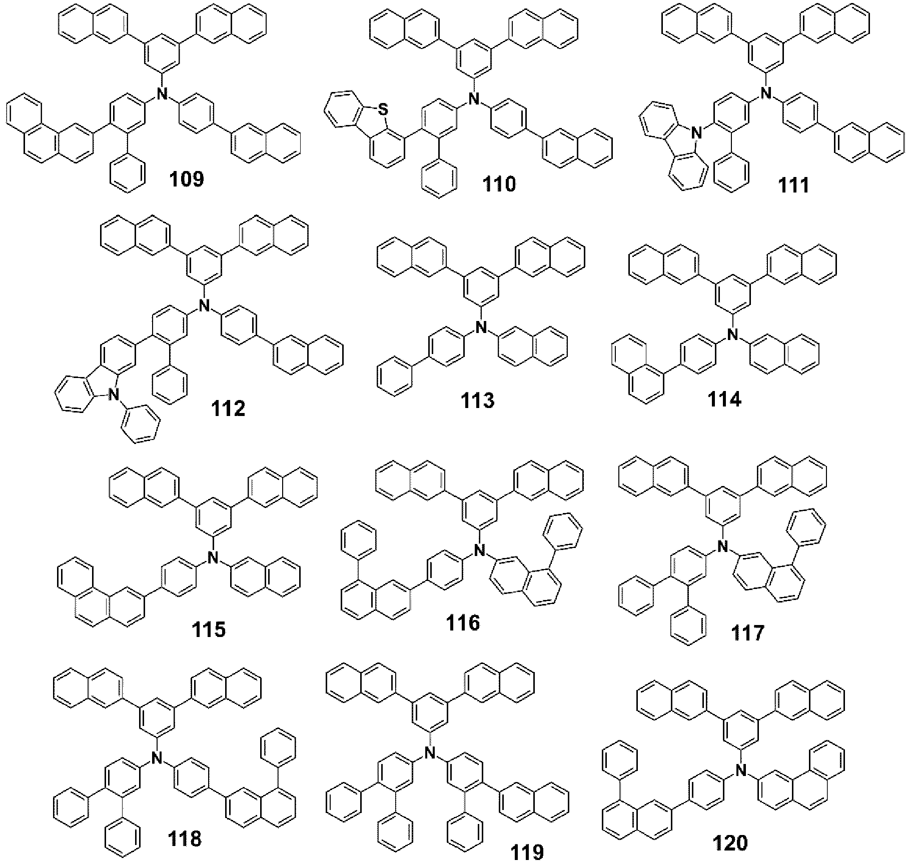

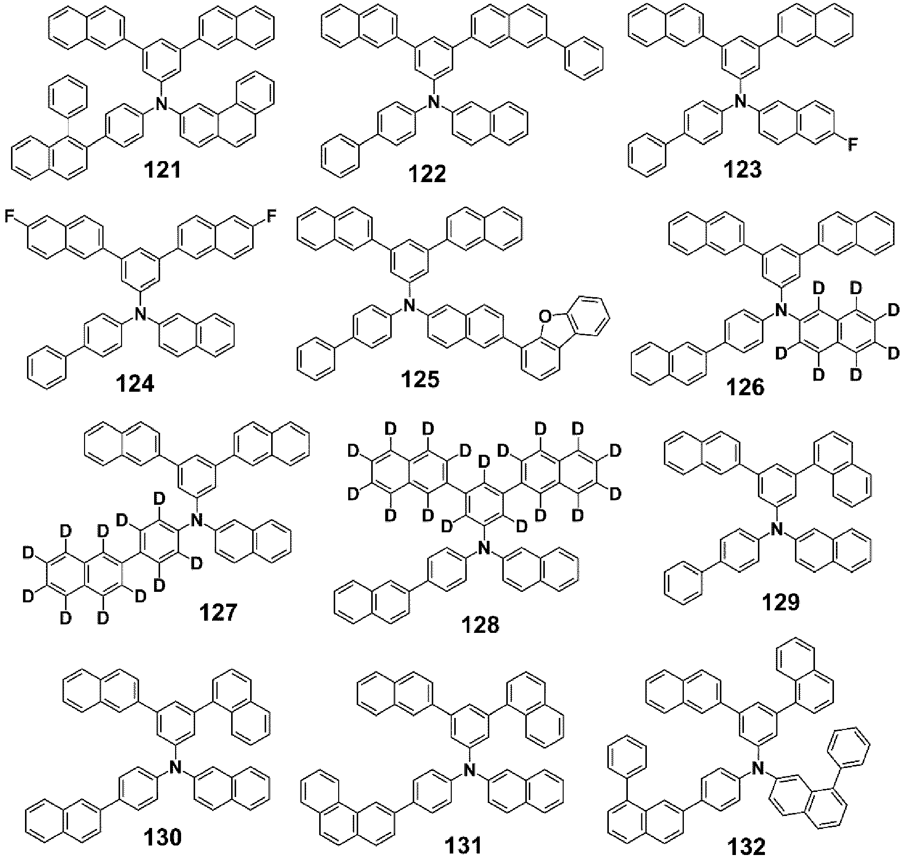

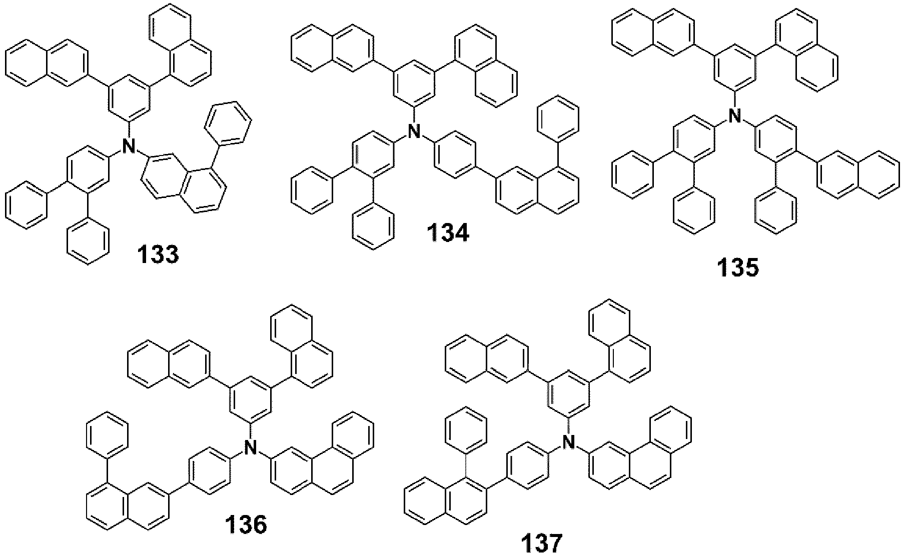

The monoamine compound of one or more embodiments includes first to third substituents directly or indirectly bonded to the amine group of the monoamine compound. The first substituent is a 3,5-dinaphthalenylphenyl group, and the second substituent is a naphthalenyl group. The third substituent corresponds to Ar^{a} of Formula 1 described above. In Formula 1 described above, the naphthalenyl group including R¹, the naphthalenyl group including R², and the phenyl group including R³ form a 3,5-dinaphthalenylphenyl group. In Formula 1 described above, the naphthalenyl group including R⁴ corresponds to the second substituent. The monoamine compound of one or more embodiments including the dinaphthalenylphenyl group and the naphthalenyl group may exhibit excellent or suitable hole transport properties as the hole transport properties are appropriately or suitably controlled or selected to improve charge balance. In addition, the monoamine compound of one or more embodiments including the first to third substituents may suppress or reduce excessive increase in deposition temperature, and may prevent or reduce degradation in materials during a deposition process. Accordingly, the monoamine compound of one or more embodiments including the first to third substituents may exhibit excellent or suitable material stability. The light emitting element ED of one or more embodiments including the monoamine compound of one or more embodiments may exhibit high luminous efficiency and long-life characteristics.

The hole transport region HTR may include at least one selected from among a hole injection layer HIL, a hole transport layer HTL, a buffer layer, a light emitting auxiliary layer, and an electron blocking layer EBL. The hole transport region HTR may have, for example, a thickness of about 50 Å to about 15,000 Å.

The hole transport region HTR may have a single layer formed of a single material, a single layer formed of a plurality of different materials, or a multilayer structure having a plurality of layers formed of a plurality of different materials.

For example, in one or more embodiments, the hole transport region HTR may have a single-layer structure formed of a hole injection layer HIL or a hole transport layer HTL, or a single-layer structure formed of a hole injection material and/or a hole transport material. For example, in one or more embodiments, the hole transport region HTR may have a single-layer structure formed of a plurality of different materials, or a structure in which a hole injection layer HIL/hole transport layer HTL, a hole injection layer HIL/hole transport layer HTL/light emitting auxiliary layer, a hole injection layer HIL/light emitting auxiliary layer, a hole transport layer HTL/light emitting auxiliary layer, or a hole injection layer HIL/hole transport layer HTL/electron blocking layer EBL are stacked in order from the first electrode EL1, but embodiment is of the present disclosure are not limited thereto.

The hole transport region HTR may be formed using one or more suitable methods such as a vacuum deposition method, a spin coating method, a casting method, a Langmuir-Blodgett (LB) method, an inkjet printing method, a laser printing method, and/or a laser induced thermal imaging (LITI) method.

The hole transport region HTR may further include one or more compounds, which will be described later, in addition to the monoamine compound of one or more embodiments.

In one or more embodiments, the hole transport region HTR may include a compound represented by Formula H-1.

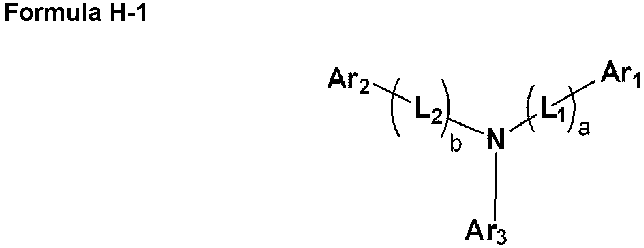

In Formula H-1, L₁ and L₂ may each independently be a direct linkage, a substituted or unsubstituted arylene group having 6 to 30 ring-forming carbon atoms, or a substituted or unsubstituted heteroarylene group having 2 to 30 ring-forming carbon atoms. a and b may each independently be an integer of 0 to 10. In one or more embodiments, if (e.g., when) a or b is an integer of 2 or greater, a plurality of L₁'s and/or a plurality of L₂'s may each independently be a substituted or unsubstituted arylene group having 6 to 30 ring-forming carbon atoms or a substituted or unsubstituted heteroarylene group having 2 to 30 ring-forming carbon atoms.

In Formula H-1, Ar₁ and Ar₂ may each independently be a substituted or unsubstituted aryl group having 6 to 30 ring-forming carbon atoms or a substituted or unsubstituted heteroaryl group having 2 to 30 ring-forming carbon atoms. In addition, in Formula H-1, Ar₃ is a substituted or unsubstituted aryl group having 6 to 30 ring-forming carbon atoms or a substituted or unsubstituted heteroaryl group having 2 to 30 ring-forming carbon atoms.

In one or more embodiments, the compound represented by Formula H-1 may be a monoamine compound. In one or more embodiments, the compound represented by Formula H-1 may be a diamine compound in which at least one selected from among Ar₁ to Ar₃ includes an amine group as a substituent. In one or more embodiments, the compound represented by Formula H-1 may be a carbazole-based compound including a substituted or unsubstituted carbazole group in at least one of Ar₁ or Ar₂, or a fluorene-based compound including a substituted or unsubstituted fluorene group in at least one of Ar₁ or Ar₂.

The compound represented by Formula H-1 may be any one selected from among compounds of Compound Group H. However, the compounds listed in Compound Group H are presented as an example, and the compound represented by Formula H-1 is not limited to the those listed in Compound Group H.

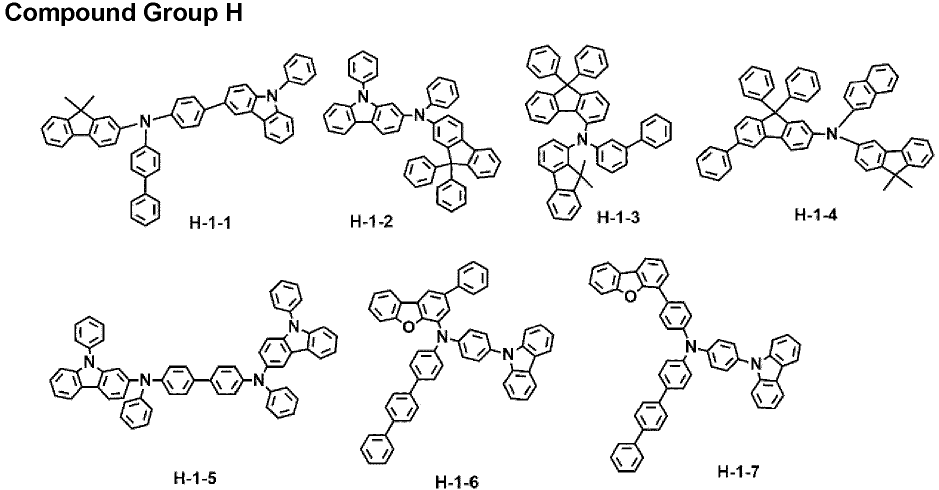

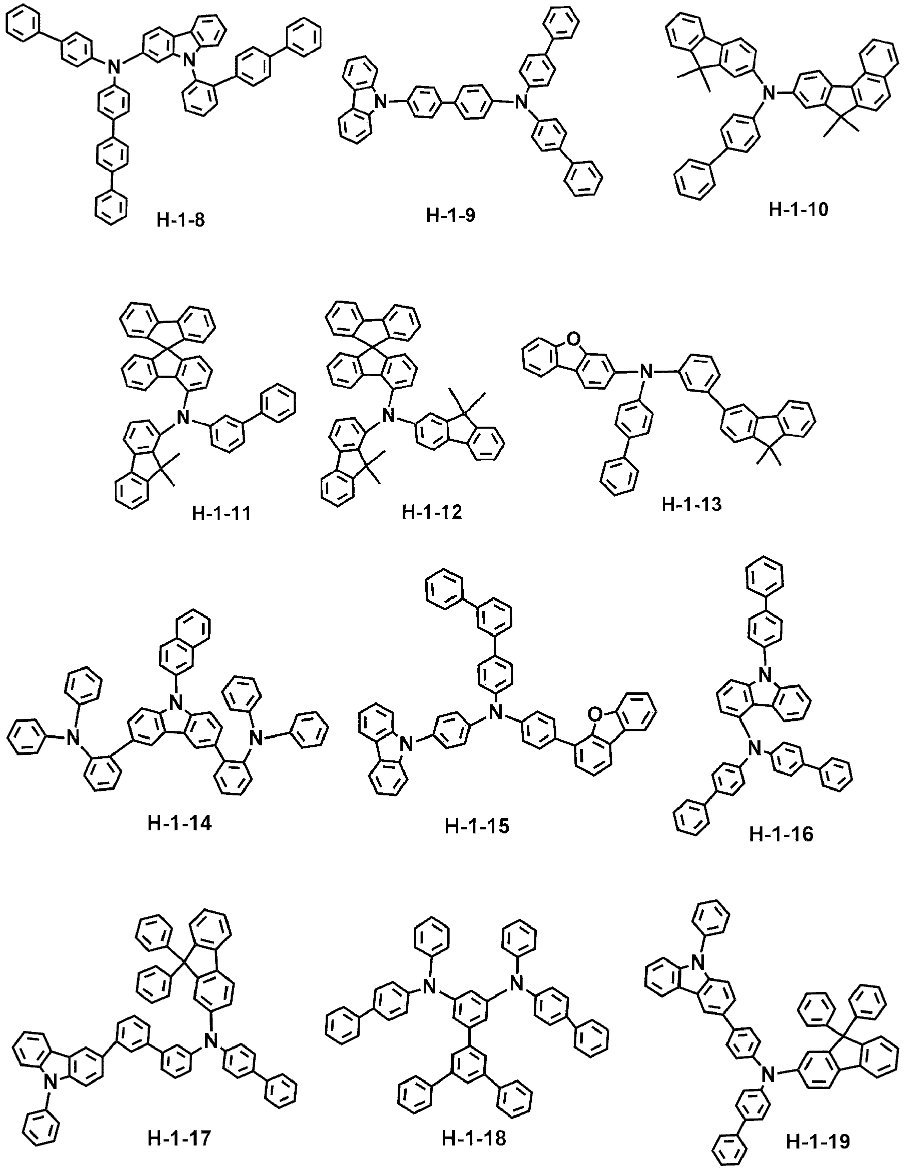

In one or more embodiments, the hole transport region HTR may include one or more selected from among a phthalocyanine compound such as copper phthalocyanine, N¹,N^{1'}-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4'4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonicacid (PANI/CSA), polyaniline/poly(4-styrenesulfonate) (PANI/PSS), N,N'-di(naphthalen-1-yl)-N,N'-diphenyl-benzidine (NPB), triphenylamine-containing polyetherketone (TPAPEK), 4-isopropyl-4'-methyldiphenyliodonium tetrakis(pentafluorophenyl)borate, dipyrazino[2,3-f: 2',3'-h]quinoxaline-2,3,6,7,10,11-hexacarbonitrile (HATCN), and/or the like.

In one or more embodiments, the hole transport region HTR may include one or more selected from among carbazole-based derivatives such as N-phenyl carbazole and polyvinyl carbazole, fluorene-based derivatives, N,N'-bis(3-methylphenyl)-N,N'-diphenyl-[1,1-biphenyl]-4,4'-diamine (TPD), triphenylamine-based derivatives such as 4,4',4"-tris(N-carbazolyl)triphenylamine (TCTA), N,N'-di(naphthalen-1-yl)-N,N'-diphenyl-benzidine (NPB), 4,4'-cyclohexylidene bis[N,N-bis(4-methylphenyl]benzenamine] (TAPC), 4,4'-bis[N,N'-(3-tolyl)amino]-3,3'-dimethylbiphenyl (HMTPD), and 1,3-bis(N-carbazolyl)benzene (mCP), and/or the like.

In one or more embodiments, the hole transport region HTR may include one or more selected from among 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol- 9-yl)benzene (mDCP), and/or the like.

The hole transport region HTR may include one or more of the compounds of the hole transport region described above in at least one selected from among the hole injection layer HIL, the hole transport layer HTL, and the electron blocking layer EBL.

The hole transport region HTR may have a thickness of about 100 Å to about 10000 Å, for example, about 100 Å to about 5000 Å. If (e.g., when) the hole transport region HTR includes a hole injection layer HIL, the hole injection layer HIL may have a thickness of, for example, about 30 Å to about 1000 Å. If (e.g., when) the hole transport region HTR includes a hole transport layer HTL, the hole transport layer HTL may have a thickness of about 30 Å to about 1000 Å. If (when) the hole transport region HTR includes an electron blocking layer EBL, the electron blocking layer EBL may have a thickness of, for example, about 10 Å to about 1000 Å. When the thicknesses of the hole transport region HTR, the hole injection layer HIL, the hole transport layer HTL, and the electron blocking layer EBL satisfy the above-described ranges, satisfactory hole transport properties may be obtained without a substantial increase in driving voltage.

In one or more embodiments, the hole transport region HTR may further include, in addition to one or more of the above-described materials, a charge generation material to increase conductivity (e.g., electric conductivity). The charge generation material may be uniformly (e.g., substantially uniformly) or non-uniformly dispersed in the hole transport region HTR. The charge generation material may be, for example, a p-dopant. The p-dopant may include at least one selected from among halogenated metal compounds (e.g., metal halides), quinone derivatives, metal oxides, and cyano group-containing compounds, but embodiments of the present disclosure are not limited thereto. For example, in one or more embodiments, the p-dopant may include one or more selected from among halogenated metal compounds such as Cul and Rbl, quinone derivatives such as tetracyanoquinodimethane (TCNQ) and 2,3,5,6-tetrafluoro-7,7',8,8'-tetracyanoquinodimethane (F4-TCNQ), metal oxides such as tungsten oxides and molybdenum oxides, cyano group-containing compounds such as dipyrazino[2,3-f: 2',3'-h] quinoxaline-2,3,6,7,10,11-hexacarbonitrile (HAT-CN) and 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), and/or the like, but embodiments of the present disclosure are not limited thereto.

As described above, the hole transport region HTR may further include at least one of a buffer layer or an electron blocking layer EBL in addition to the hole injection layer HIL and the hole transport layer HTL. The buffer layer may compensate a resonance distance according to wavelengths of light emitted from the emission layer EML, and may thus increase light emitting efficiency. Materials which may be included in the hole transport region HTR may be used as materials included in the buffer layer. The electron blocking layer EBL is a layer that serves to prevent or reduce electrons from being injected from the electron transport region ETR to the hole transport region HTR.

The emission layer EML may be provided on the hole transport region HTR. The emission layer EML may have, for example, a thickness of about 100 Å to about 1000 Å or about 100 Å to about 300 Å. The emission layer EML may have a single layer formed of a single material, a single layer formed of a plurality of different materials, or a multilayer structure having a plurality of layers formed of a plurality of different materials.

In the light emitting element ED according to one or more embodiments, the emission layer EML may include at least one of an anthracene derivative, a pyrene derivative, a fluoranthene derivative, a chrysene derivative, a dihydrobenzanthracene derivative, or a triphenylene derivative. For example, in one or more embodiments, the emission layer EML may include an anthracene derivative or a pyrene derivative.

In the light emitting element ED of one or more embodiments shown in FIGS. 3 to 6, the emission layer EML may include a host and a dopant. For example, in one or more embodiments, the emission layer EML may include a compound represented by Formula E-1. The compound represented by Formula E-1 may be used as a fluorescent host material.

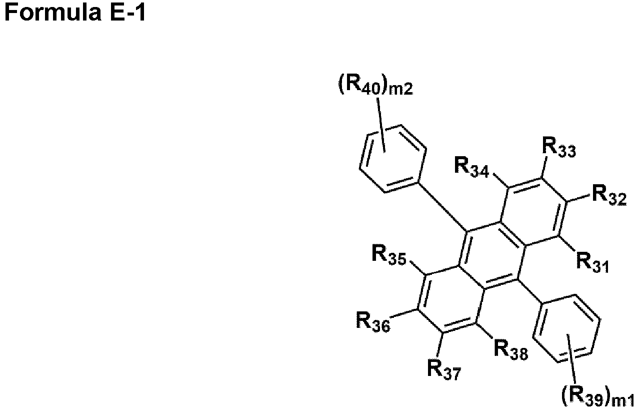

In Formula E-1, m1 and m2 are each independently an integer of 0 to 5. When m1 is an integer of 2 or greater, a plurality of R₃₉'s may all be the same or at least one thereof may be different from the others. When m1 is 0, the embodiment may be the same as an embodiment in which m1 is 5 and five R₃₉'s are all hydrogens. When m2 is an integer of 2 or greater, a plurality of R₄₀'s may all be the same or at least one thereof may be different from the others. When m2 is 0, the embodiment may be the same as an embodiment in which m2 is 5 and five R₄₀'s are all hydrogens.

In Formula E-1, R₃₁ to R₄₀ are each independently hydrogen, deuterium, a halogen, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring.

The compound represented by Formula E-1 may be any one selected from among compounds E1 to E21.

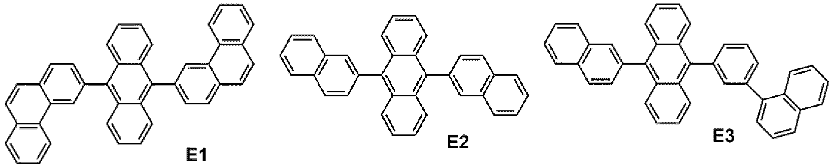

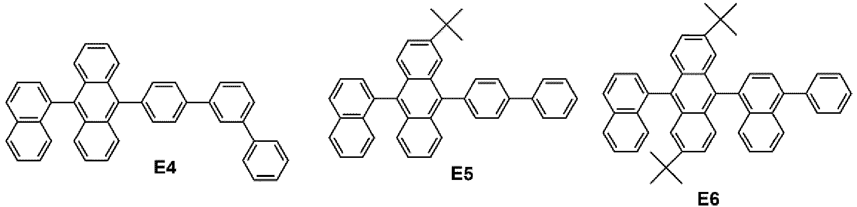

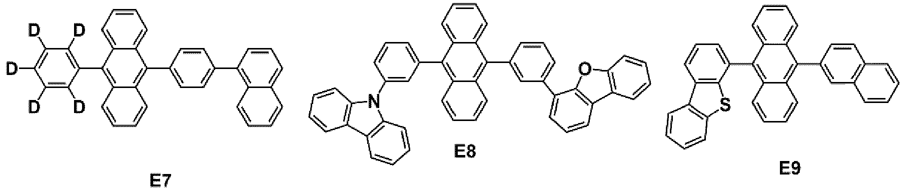

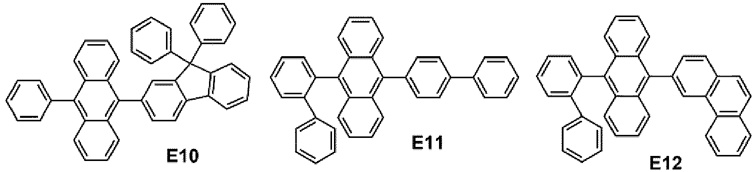

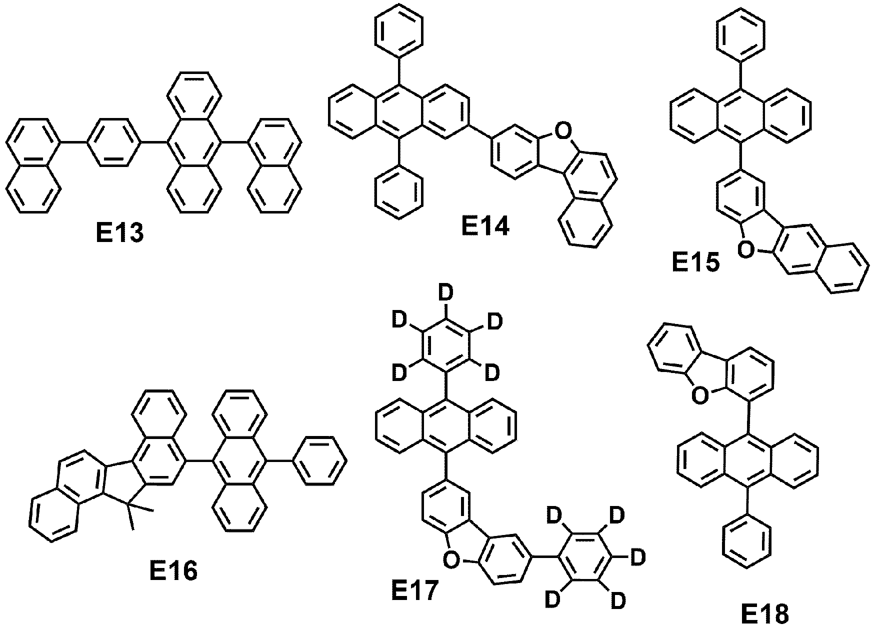

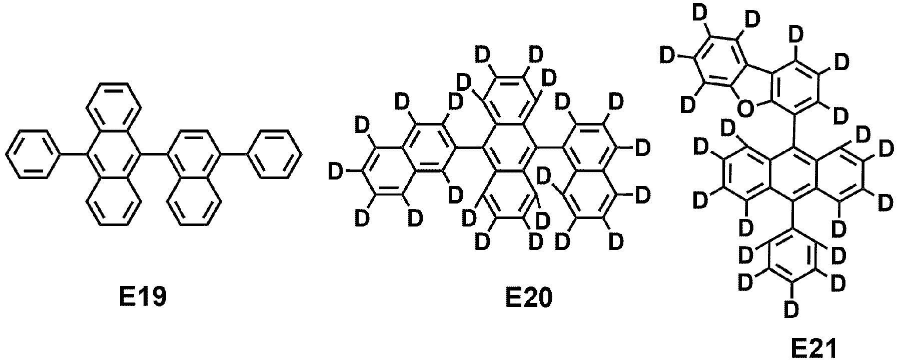

In one or more embodiments, the emission layer EML may include a compound represented by Formula E-2a or Formula E-2b. The compound represented by Formula E-2a or Formula E-2b may be used as a host material for a phosphorescent element.

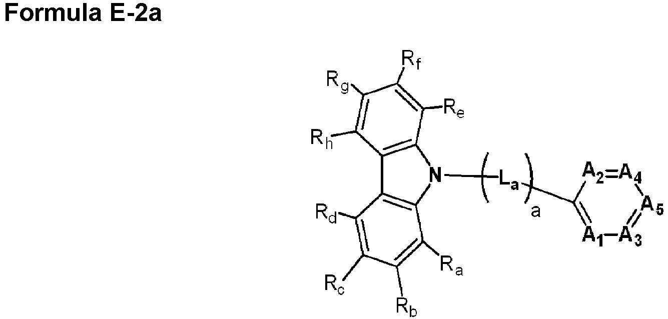

In Formula E-2a, a may be an integer of 0 to 10, and Lₐ may be a direct linkage, a substituted or unsubstituted arylene group having 6 to 30 ring-forming carbon atoms, or a substituted or unsubstituted heteroarylene group having 2 to 30 ring-forming carbon atoms. In one or more embodiments, if (e.g., when) a is an integer of 2 or greater, a plurality of Lₐ's may each independently be a substituted or unsubstituted arylene group having 6 to 30 ring-forming carbon atoms or a substituted or unsubstituted heteroarylene group having 2 to 30 ring-forming carbon atoms.

In addition, in Formula E-2a, A₁ to As may each independently be N or CRᵢ. Rₐ to Rᵢ may each independently be hydrogen, deuterium, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, a substituted or unsubstituted heteroaryl group having 2 to 30 ring-forming carbon atoms, and/or bonded to an adjacent group to form a ring. In one or more embodiments, one or more selected from among Rₐ to Rᵢ may each independently be bonded to an adjacent group to form a hydrocarbon ring or a heterocycle containing N, O, S, and/or the like as a ring-forming atom.

In one or more embodiments, in Formula E-2a, two or three selected from among A₁ to A₅ may be N, and the others may be CRᵢ.

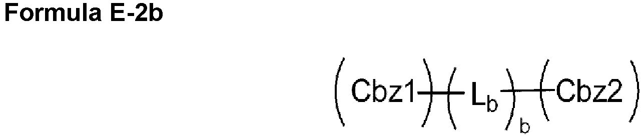

In Formula E-2b, Cbz1 and Cbz2 may each independently be an unsubstituted carbazole group or an aryl-substituted carbazole group having 6 to 30 ring-forming carbon atoms. L_{b} may be a direct linkage, a substituted or unsubstituted arylene group having 6 to 30 ring-forming carbon atoms, or a substituted or unsubstituted heteroarylene group having 2 to 30 ring-forming carbon atoms, b may be an integer of 0 to 10, and if (e.g., when) b is an integer of 2 or greater, a plurality of L_{b}'s may each independently be a substituted or unsubstituted arylene group having 6 to 30 ring-forming carbon atoms or a substituted or unsubstituted heteroarylene group having 2 to 30 ring-forming carbon atoms.

The compound represented by Formula E-2a or Formula E-2b may be any one selected from among compounds in Compound Group E-2. However, the compounds listed in Compound Group E-2 are presented as an example, and the compound represented by Formula E-2a or Formula E-2b is not limited to those listed in Compound Group E-2.

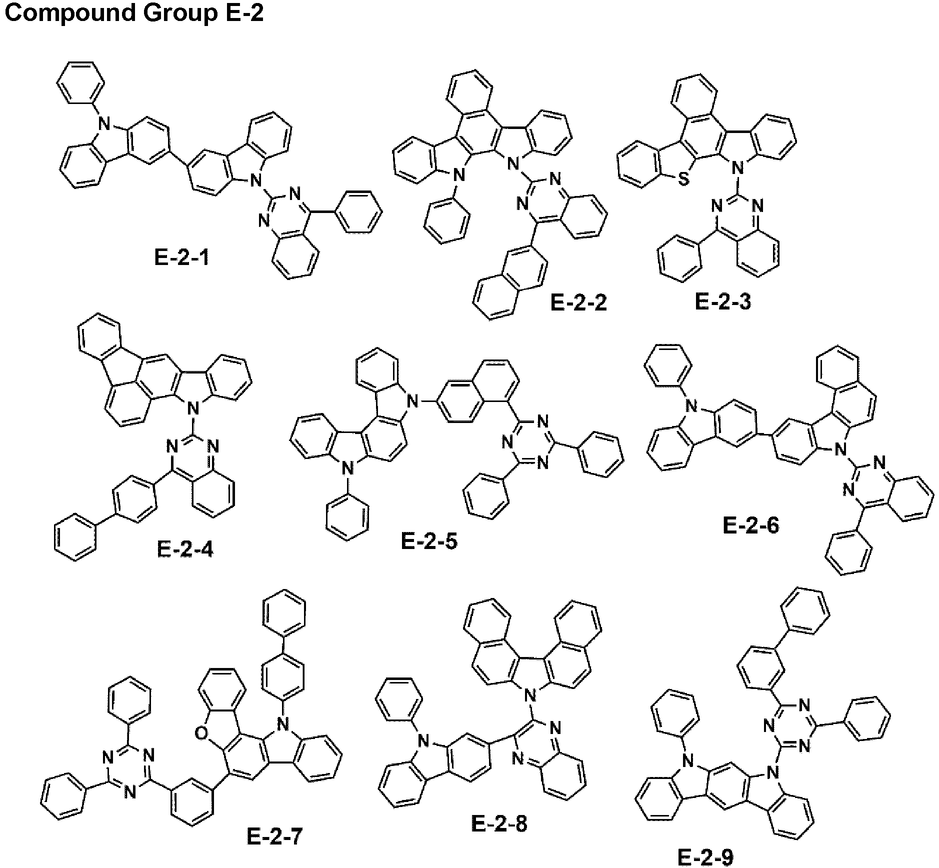

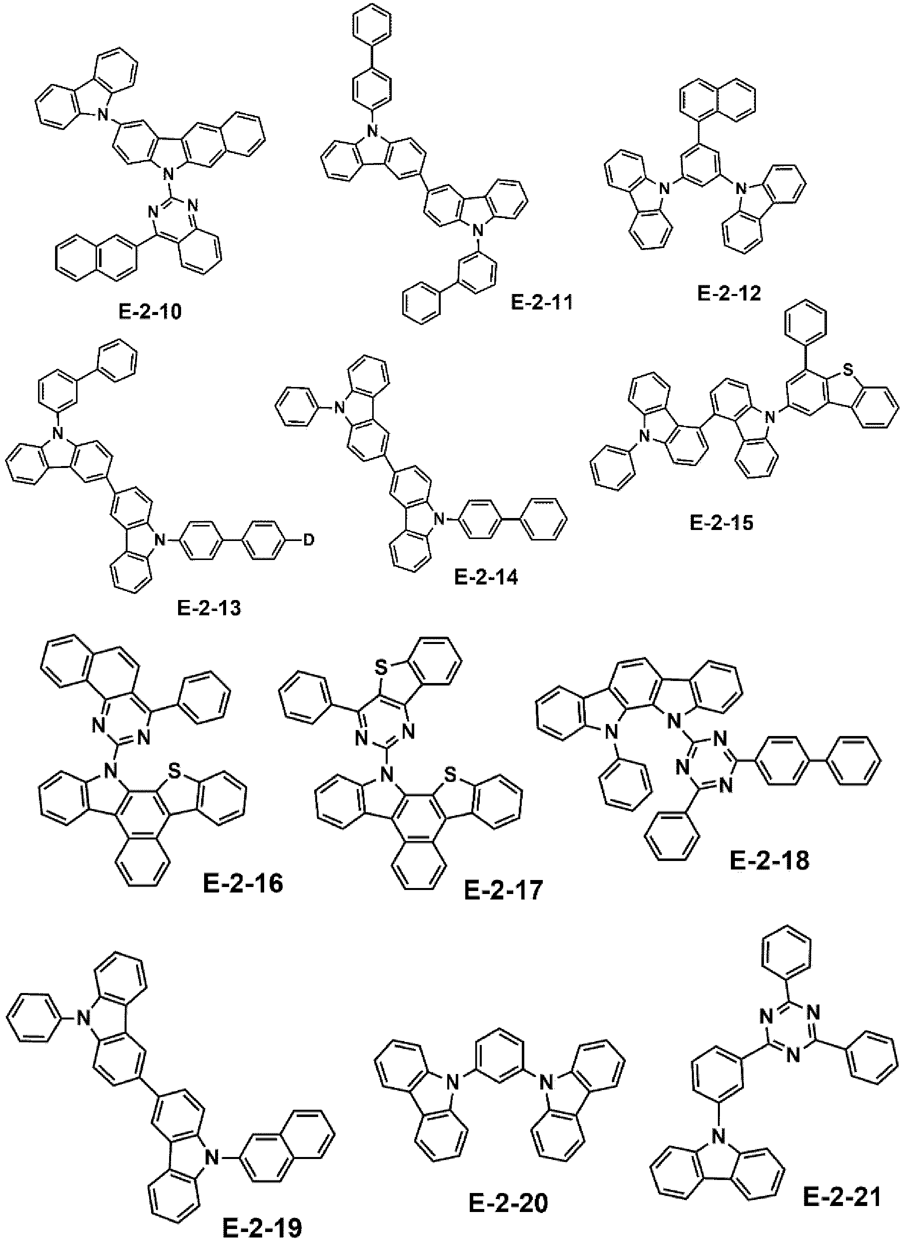

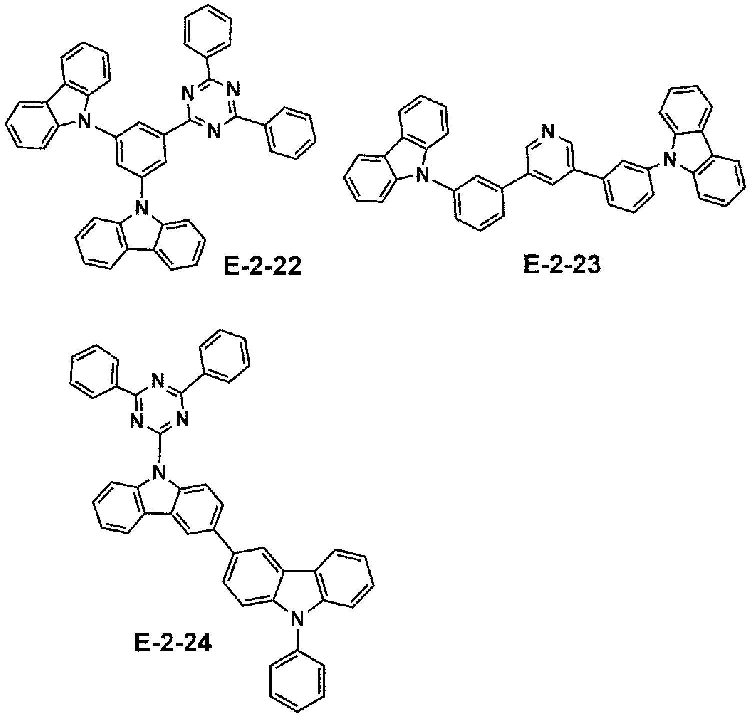

In one or more embodiments, the emission layer EML may further include a general material suitable in the art as a host material. For example, the emission layer EML may include, as a host material, at least one selected from among bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1'-biphenyl (CBP), 1,3-bis(carbazolyl-9-yl)benzene (mCP), 2,8-bis(diphenylphosphoryl)dibenzofuran (PPF), 4,4',4"-tris(carbazol-9-yl)-triphenylamine (TCTA), and 1,3,5-tris(1-phenyl-1H-benzo[d]imidazol-2-yl)benzene (TPBi). However, embodiments of the present disclosure are not limited thereto, for example, tris(8-hydroxyquinolinato)aluminium (Alq₃), 9,10-di(naphthalen-2-yl)anthracene (ADN), 3-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetrasiloxane (DPSiO₄), and/or the like may be used as a host material.

In one or more embodiments, the emission layer EML may include a compound represented by Formula M-a or Formula M-b. The compound represented by Formula M-a or Formula M-b may be used as a phosphorescent dopant material.

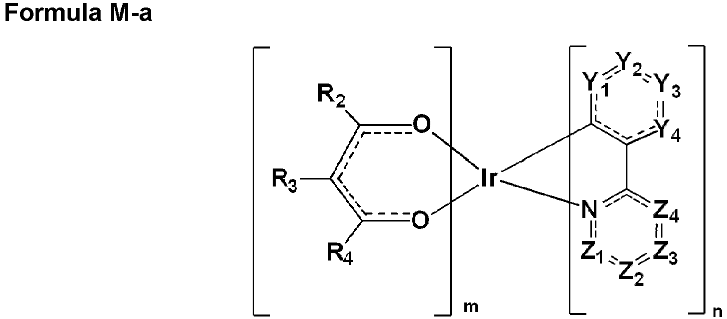

In Formula M-a, Y₁ to Y₄ and Z₁ to Z₄ may each independently be CR₁ or N, and R₁ to R₄ may each independently be hydrogen, deuterium, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring. In Formula M-a, m is 0 or 1, and n is 2 or 3. In Formula M-a, if (e.g., when) m is 0, n is 3, and if (e.g., when) m is 1, n is 2.

The compound represented by Formula M-a may be used as a phosphorescent dopant.

The compound represented by Formula M-a may be any one selected from among compounds M-a1 to M-a25. However, the compounds M-a1 to M-a25 are presented as an example, and the compound represented by Formula M-a is not limited to those represented by the compounds M-a1 to M-a25.

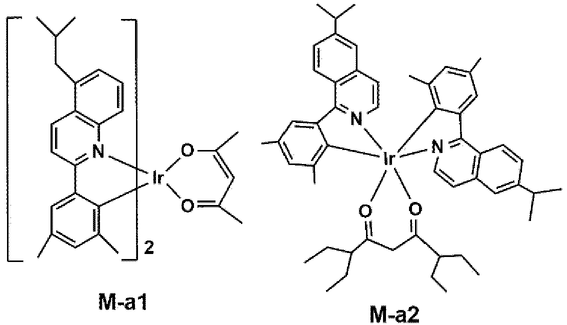

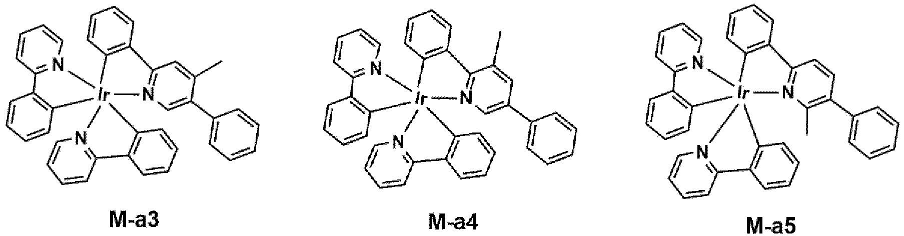

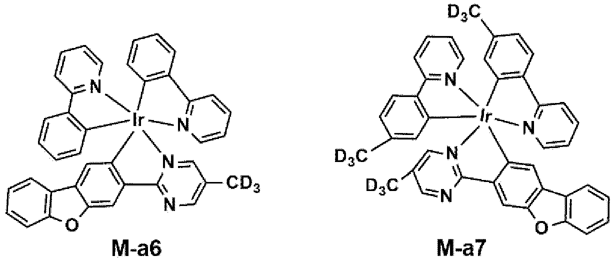

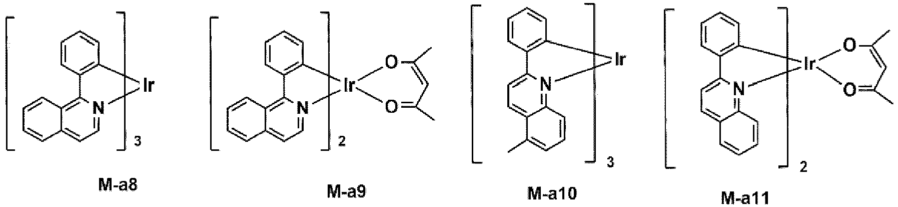

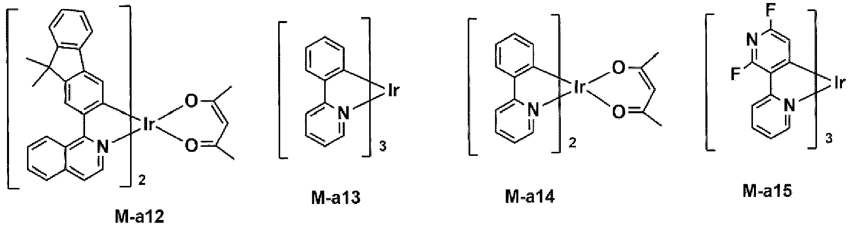

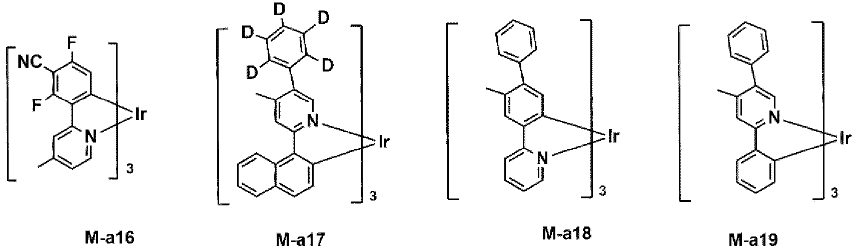

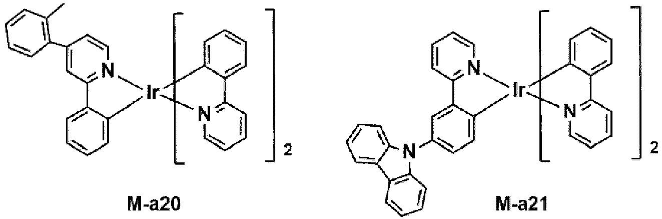

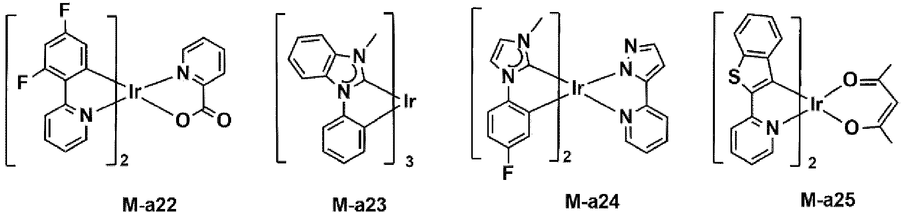

The compounds M-a1 and M-a2 may be used as a red dopant material, and the compounds M-a3 to M-a5 may be used as a green dopant material.

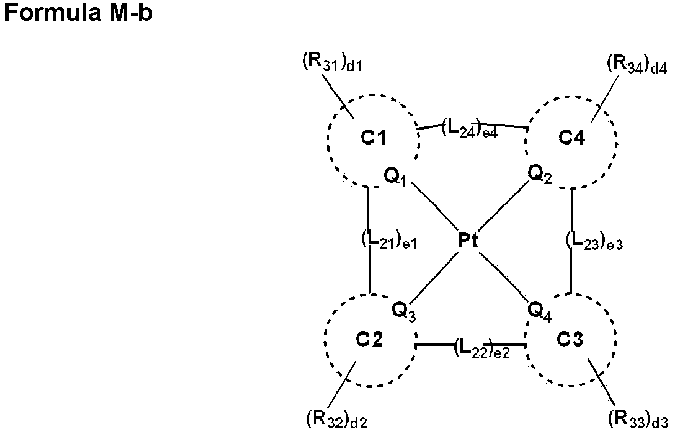

In Formula M-b, Q₁ to Q₄ may each independently be C or N. C1 to C4 may each independently be a substituted or unsubstituted hydrocarbon ring group having 5 to 30 ring-forming carbon atoms, a substituted or unsubstituted aryl group having 6 to 30 ring-forming carbon atoms, or a substituted or unsubstituted heterocycle group having 2 to 30 ring-forming carbon atoms.

L₂₁ to L₂₄ may each independently be a direct linkage, 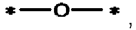 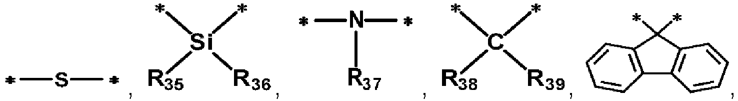 a substituted or unsubstituted alkylene group having 1 to 20 carbon atoms, a substituted or unsubstituted arylene group having 6 to 30 ring-forming carbon atoms, or a substituted or unsubstituted heteroarylene group having 2 to 30 ring-forming carbon atoms. e1 to e4 may each independently be 0 or 1.

R₃₁ to R₃₉ may each independently be hydrogen, deuterium, a halogen, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring. d1 to d4 may each independently be an integer of 0 to 4. When d1 is an integer of 2 or greater, a plurality of R₃₁'s may all be the same or at least one thereof may be different from the others. When d2 is an integer of 2 or greater, a plurality of R₃₂'s may all be the same or at least one thereof may be different from the others. When d3 is an integer of 2 or greater, a plurality of R₃₃'s may all be the same or at least one thereof may be different from the others. When d4 is an integer of 2 or greater, a plurality of R₃₄'s may all be the same or at least one thereof may be different from the others.

The compound represented by Formula M-b may be used as a blue phosphorescent dopant or a green phosphorescent dopant. The compound represented by Formula M-b may be any one selected from compounds listed below. However, those compounds are presented as an example, and the compound represented by Formula M-b is not limited to those represented by the compounds listed below. In compounds AD-01 to AD-53, D indicates deuterium.

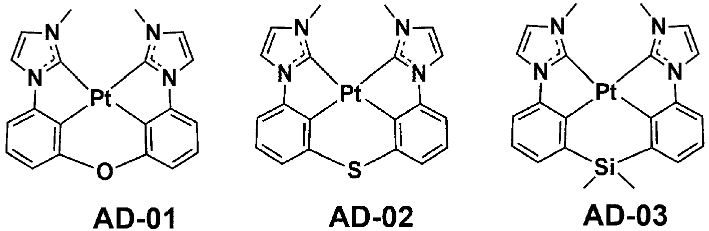

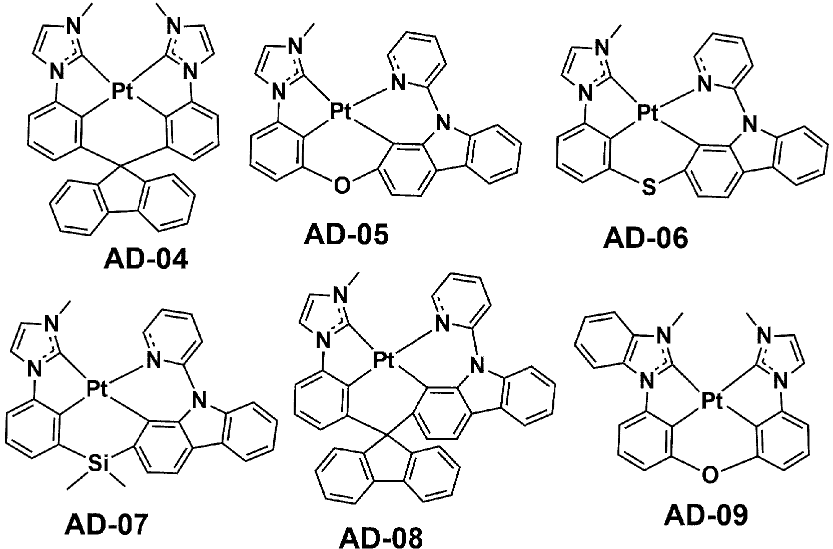

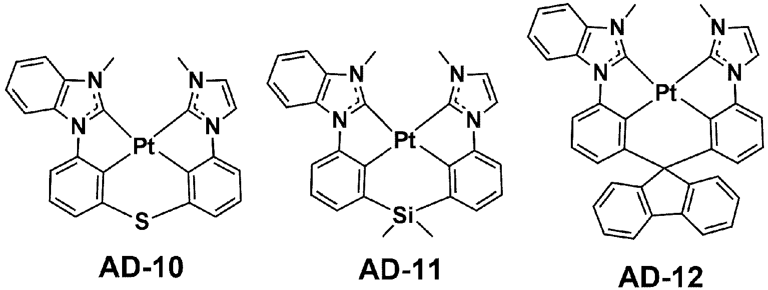

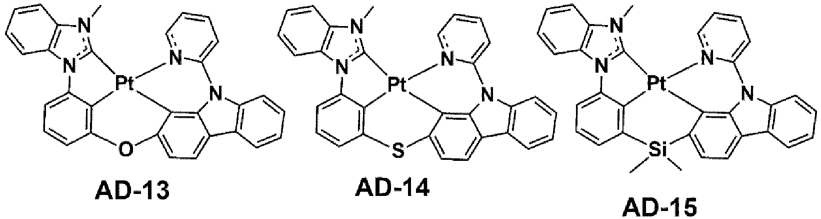

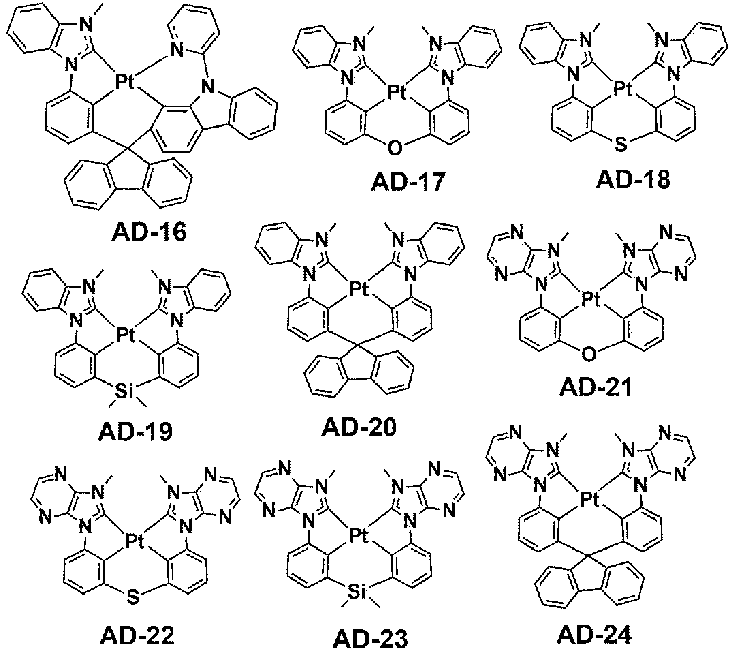

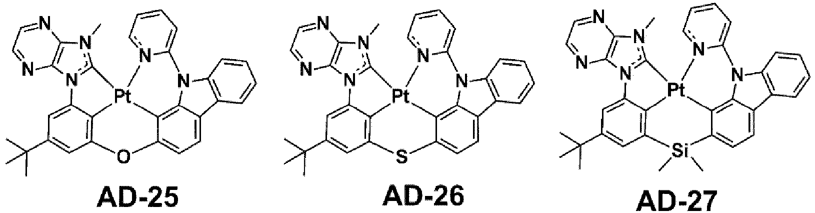

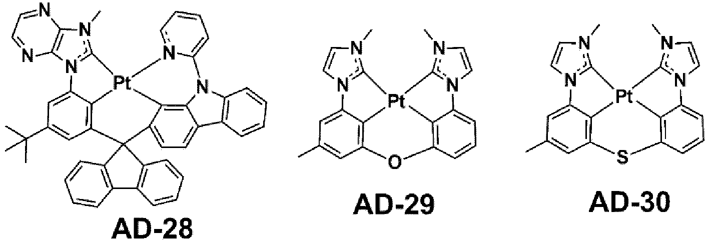

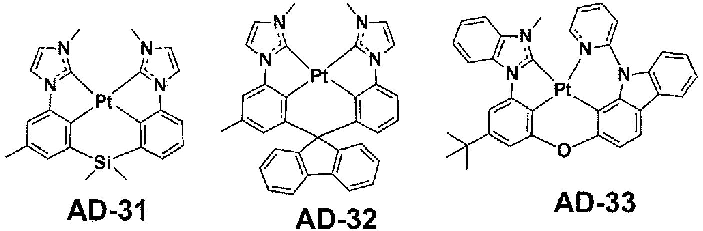

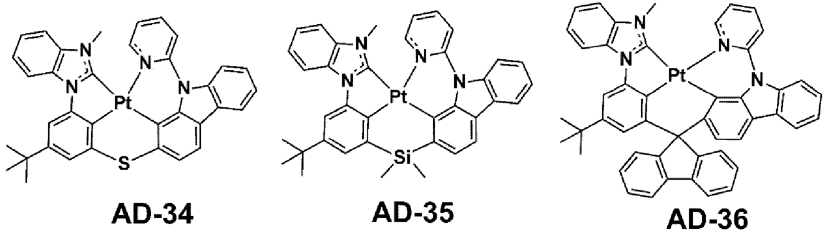

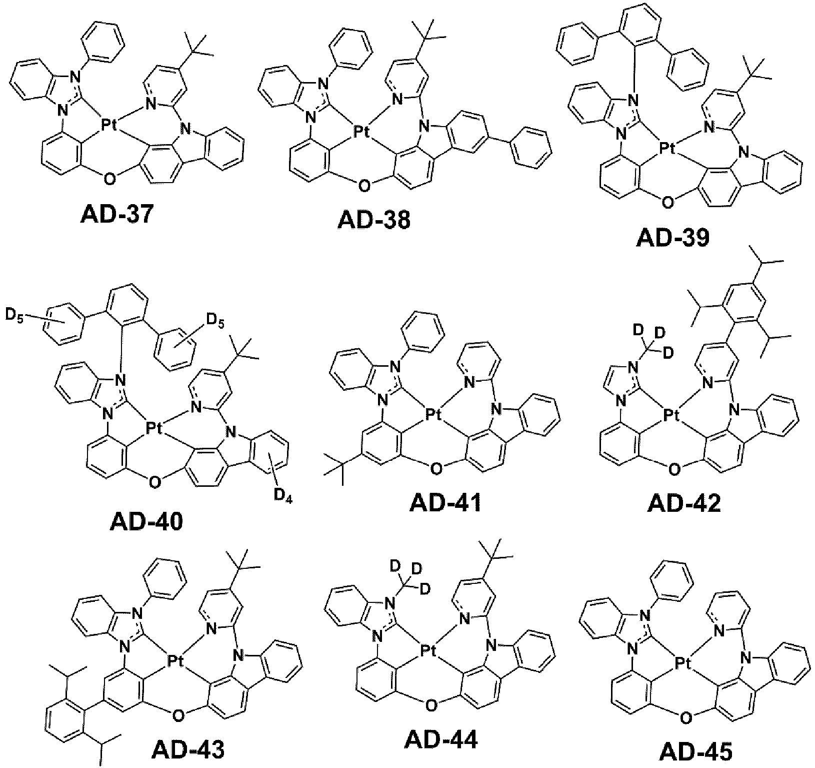

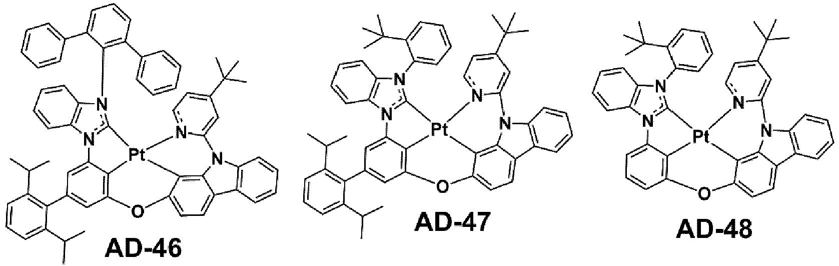

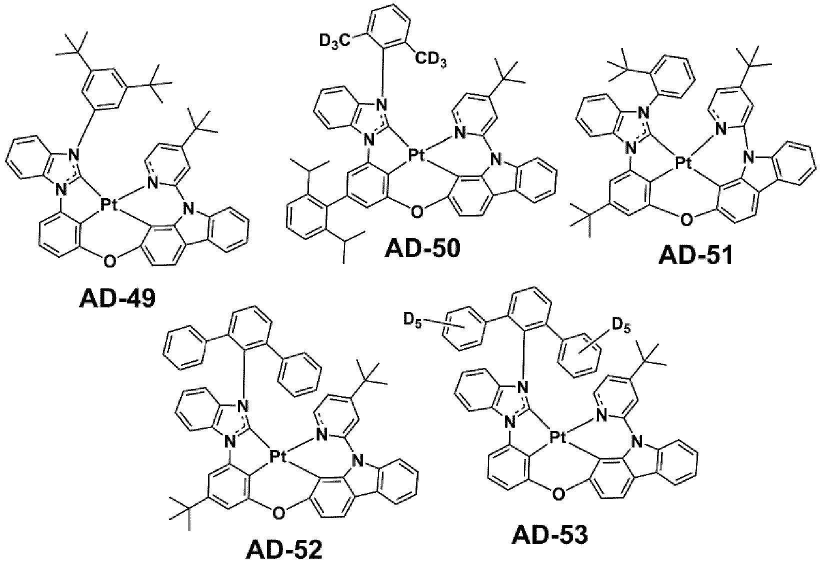

In one or more embodiments, the emission layer EML may include a compound represented by any one selected from Formulas F-a to F-c. The compounds represented by any one selected from Formulas F-a to F-c may be used as a fluorescence dopant material.

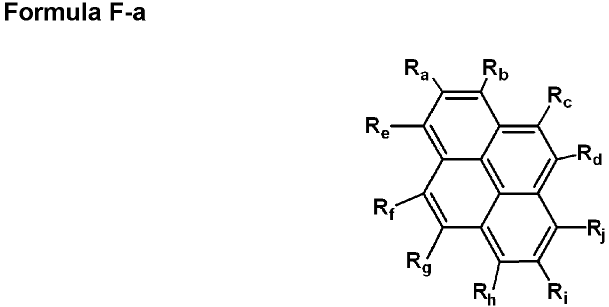

In Formula F-a, two selected from among Rₐ to Rⱼ may each independently be substituted with *-NAr₁Ar₂. The others among Rₐ to Rⱼ which are not substituted with *-NAr₁,Ar₂ may each independently be hydrogen, deuterium, a halogen, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In *-NAr₁Ar₂, Ar₁ and Ar₂ may each independently be a substituted or unsubstituted aryl group having 6 to 30 ring-forming carbon atoms or a substituted or unsubstituted heteroaryl group having 2 to 30 ring-forming carbon atoms. For example, in one or more embodiments, at least one of Ar₁ or Ar₂ may be a heteroaryl group containing O or S as a ring-forming atom.

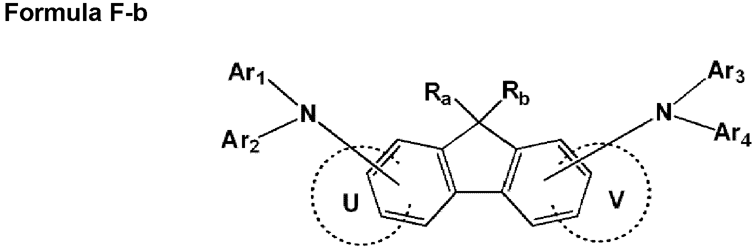

In Formula F-b, Rₐ and R_{b} may each independently be hydrogen, deuterium, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or independently bonded to an adjacent group to form a ring. Ar₁ to Ar₄ may each independently be a substituted or unsubstituted aryl group having 6 to 30 ring-forming carbon atoms or a substituted or unsubstituted heteroaryl group having 2 to 30 ring-forming carbon atoms.

In Formula F-b, U and V may each independently be a substituted or unsubstituted hydrocarbon ring having 5 to 30 ring-forming carbon atoms or a substituted or unsubstituted heterocycle having 2 to 30 ring-forming carbon atoms. At least one of Ar₁ to Ar₄ may be a heteroaryl group containing O or S as a ring-forming atom.

In Formula F-b, the number of rings represented by U and V may each independently be 0 or 1. For example, In Formula F-b, if (e.g., when) the number of U or V is 1, one ring forms a part of a fused ring in a portion indicated by U or V, and if (e.g., when) the number of U or V is 0, it refers to that no ring indicated by U or V is present. For example, if (e.g., when) the number of U is 0 and the number of V is 1, or if (e.g., when) the number of U is 1 and the number of V is 0, a fused ring having a fluorene core of Formula F-b may be a cyclic compound having four rings. In one or more embodiments, if (e.g., when) both (e.g., simultaneously) U and V are 0, the fused ring of Formula F-b may be a cyclic compound having three rings. In one or more embodiments, if (e.g., when) both (e.g., simultaneously) U and V are 1, the fused ring having a fluorene core of Formula F-b may be a cyclic compound having five rings.

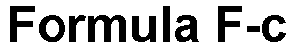

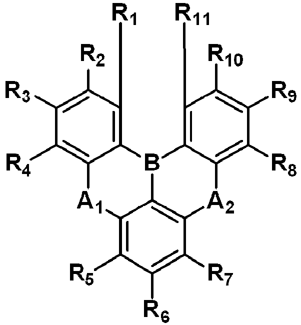

In Formula F-c, A₁ and A₂ may each independently be O, S, Se, or NRₘ, and Rₘ may be hydrogen, deuterium, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ may each independently be hydrogen, deuterium, a halogen, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or independently bonded to an adjacent group to form a ring.

In Formula F-c, A₁ and A₂ may each independently be bonded to substituents of neighboring rings to form a fused ring. For example, if (e.g., when) A₁ and A₂ may each independently be NRₘ, A₁ may be bonded to R₄ or R₅ to form a ring. In addition, A₂ may be bonded to R₇ or R₈ to form a ring.

In one or more embodiments, the emission layer EML may include, as a suitable dopant material, one or more selected from among styryl derivatives (e.g., 1,4-bis[2-(3-N- ethylcarbazolyl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi), 4,4'-bis[2-(4-(N,N-diphenylamino)phenyl)vinyl]biphenyl (DPAVBi)), perylene and derivatives thereof (e.g., 2,5,8,11-tetra-t-butylperylene (TBP)), pyrene and derivatives thereof (e.g., 1,1'-dipyrene, 1,4-dipyrenylbenzene, 1,4-bis(N,N'-diphenylamino)pyrene), and/or the like.

In one or more embodiments, the emission layer EML may include a suitable phosphorescent dopant material. For example, as a phosphorescent dopant, a metal complex including iridium (Ir), platinum (Pt), osmium (Os), gold (Au), titanium (Ti), zirconium (Zr), hafnium (Hf), europium (Eu), terbium (Tb), or thulium (Tm) may be used. For example, iridium(III) bis(4,6-difluorophenylpyridinato-N,C2')picolinate (FIrpic), bis(2,4-difluorophenylpyridinato)-tetrakis(1-pyrazolyl)borate iridium(III) (Fir6), platinum octaethyl porphyrin (PtOEP), and/or the like may be used as a phosphorescent dopant. However, embodiments of the present disclosure are not limited thereto.

In one or more embodiments, the emission layer EML may include a quantum dot material. The quantum dot may have a core/shell structure. The core of a quantum dot may be selected from among a Group II-VI compound, a Group I-II-VI compound, a Group II-IV-VI compound, a Group I-II-IV-VI compound, a Group II-IV-V compound, a Group III-VI compound, a Group I-III-VI element, a Group III-V compound, a Group III-II-V compound, a Group IV-VI compound, a Group IV element, a Group IV compound, and a combination thereof.

The Group II-VI compound may be selected from the group consisting of: a binary compound selected from the group consisting of CdSe, CdTe, CdS, ZnS, ZnSe, ZnTe, ZnO, HgS, HgSe, HgTe, MgSe, MgS, and a mixture thereof; a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof; and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, and a mixture thereof.

In one or more embodiments, the Group II-VI compound may further include a Group I metal and/or a Group IV element. The Group I-II-VI compound may be selected from among CuSnS and CuZnS, and the Group II-IV-VI compound may be selected from among ZnSnS and/or the like. The Group I-II-IV-VI compound may be selected from among quaternary compounds selected from the group consisting of Cu₂ZnSnS₂, Cu₂ZnSnS₄, Cu₂ZnSnSe₄, Ag₂ZnSnS₂, and a mixture thereof.

The Group II-IV-V compound may be selected from among a ternary compound selected from among the group consisting of ZnSnP, ZnSnP₂, ZnSnAs₂, ZnGeP₂, ZnGeAs₂, CdSnP₂, and CdGeP₂ and a mixture thereof.

The Group III-VI compound may include a binary compound such as GaS, Ga₂S₃, GaSe, Ga₂Se₃, GaTe, InTe, InS, InSe, In₂S₃, and/or In₂Se₃, a ternary compound such as InGaS₃ and/or InGaSe₃, or any combination thereof.

The Group I-III-VI compound may be selected from among a ternary compound selected from the group consisting of AgInS, AgInS₂, CuInS, CuInS₂, AgGaS₂, CuGaS₂ CuGaO₂, AgGaO₂, AgAlO₂, and a mixture thereof, and/or a quaternary compound such as AgInGaS₂ and/or CuInGaS₂.

The Group III-V compound may be selected from the group consisting of a binary compound selected from the group consisting of GaN, GaP, GaAs, GaSb, AlN, AlP, AlAs, AlSb, InN, InP, InAs, InSb, and a mixture thereof, a ternary compound selected from the group consisting of GaNP, GaNAs, GaNSb, GaPAs, GaPSb, AINP, AlNAs, AlNSb, AlPAs, AlPSb, InGaP, InAlP, InNP, InNAs, InNSb, InPAs, InPSb, and a mixture thereof, and a quaternary compound selected from the group consisting of GaAlNP, GaAlNAs, GaAlNSb, GaAlPAs, GaAlPSb, GaInNP, GaInNAs, GaInNSb, GaInPAs, GaInPSb, InAlNP, InAINAs, InAINSb, InAlPAs, InAlPSb, and a mixture thereof. In one or more embodiments, the Group III-V compound may further include a Group II metal. For example, InZnP and/or the like may be selected as a Group III-II-V compound.

The Group IV-VI compound may be selected from the group consisting of a binary compound selected from the group consisting of SnS, SnSe, SnTe, PbS, PbSe, PbTe, and a mixture thereof, a ternary compound selected from the group consisting of SnSeS, SnSeTe, SnSTe, PbSeS, PbSeTe, PbSTe, SnPbS, SnPbSe, SnPbTe, and a mixture thereof, and a quaternary compound selected from the group consisting of SnPbSSe, SnPbSeTe, SnPbSTe, and a mixture thereof. The Group IV element may be selected from the group consisting of Si, Ge, and a mixture thereof. The Group IV compound may be a binary compound selected from the group consisting of SiC, SiGe, and a mixture thereof.

Each element included in a multi-element compound such as the binary compound, ternary compound, and quaternary compound may be present in particles at a substantially uniform concentration or a non-uniform concentration. For example, Formula above indicates the types (kinds) of elements included in a compound, and element ratios in the compound may be different. For example, AgInGaS₂ may indicate AgInₓGa₁₋ₓS₂ (x is a real number between 0 and 1).

In one or more embodiments, the quantum dot may have a single structure having a substantially uniform concentration of each element included in the corresponding quantum dot or a dual structure of a core-shell. For example, materials included in the core may be different from materials included in the shell.

The shell of the quantum dot may serve as a protection layer to prevent or reduce the chemical deformation of the core so as to keep semiconductor properties, and/or a charging layer to impart electrophoresis properties to the quantum dot. The shell may be a single layer or a plurality of layers. An interface between the core and the shell may have a concentration gradient in which the concentration of an element present in the shell becomes lower towards the center.

In one or more embodiments, the quantum dots may have the above-described core/shell structure including a core having nano-crystals and a shell around (e.g., surrounding) the core. Examples of the shell of the quantum dot may be a metal or non-metal oxide, a semiconductor compound, or any combination thereof.

For example, the metal or non-metal oxide as a shell may be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, NiO, or a ternary compound such as MgAl₂O₄, CoFe₂O₄, NiFe₂O₄, and/or CoMn₂O₄, but embodiments of the disclosure are not limited thereto.

In addition, the semiconductor compound suitable as a shell may be, for example, CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and/or the like, but embodiments of the disclosure are not limited thereto.

Each element included in a multi-element compound such as the binary compound and the ternary compound may be present in particles at a substantially uniform concentration or a non-uniform concentration. For example, Formula above indicates the types (kinds) of elements included in a compound, and element ratios in the compound may be different.

The quantum dots may have, in a light emitting wavelength spectrum (e.g., in an emission spectrum), a full width at half maximum (FWHM) of about 45 nm or less, about 40 nm or less, or about 30 nm or less, and in this range, the color purity or the color reproducibility of the quantum dot may be improved. In addition, light emitted through the quantum dot is emitted in all directions, and thus a wide viewing angle may be improved.

In addition, the form of the quantum dots is not particularly limited as long as it is a form generally used in the art, for example, a quantum dot in the form of spherical nanoparticles, pyramidal nanoparticles, multi-arm nanoparticles, cubic nanoparticles, nanotubes, nanowires, nanofibers, nanoplatelets, and/or the like may be used.

As the size of the quantum dot or the ratio of elements in the quantum dot compound is regulated, the energy band gap of the quantum dot may be accordingly controlled or selected to obtain light of one or more suitable wavelengths from a quantum dot emission layer. Therefore, by using the quantum dots as described above (using quantum dots of different sizes and/or having different element ratios in the quantum dot compound), a light emitting element emitting light of one or more suitable wavelengths may be obtained. For example, the size of the quantum dots or the ratio of elements in the quantum dot compound may be regulated to enable the quantum dots to emit red, green, and/or blue light. In one or more embodiments, the quantum dots may be configured to emit white light by combining light of one or more suitable colors.

In the light emitting element ED according to one or more embodiments shown in FIGS. 3 to 6, the electron transport region ETR may be provided on the emission layer EML. The electron transport region ETR may include at least one selected from among a hole blocking layer HBL, an electron transport layer ETL, and an electron injection layer EIL, but embodiments of the present disclosure are not limited thereto.

The electron transport region ETR may have a single layer formed of a single material, a single layer formed of a plurality of different materials, or a multilayer structure having a plurality of layers formed of a plurality of different materials.

For example, in one or more embodiments, the electron transport region ETR may have a single layer structure of an electron injection layer EIL or an electron transport layer ETL, or may have a single layer structure formed of an electron injection material and/or an electron transport material. In one or more embodiments, the electron transport region ETR may have a single layer structure formed of a plurality of different materials, or may have a structure in which an electron transport layer ETL/electron injection layer EIL, or a hole blocking layer HBL/electron transport layer ETL/electron injection layer EIL are stacked in order (e.g., in the stated order) from the emission layer EML, but embodiments of the present disclosure are not limited thereto. The electron transport region ETR may have a thickness of, for example, about 100 Å to about 1500 Å.

The electron transport region ETR may be formed using one or more suitable methods such as a vacuum deposition method, a spin coating method, a casting method, a Langmuir-Blodgett (LB) method, an inkjet printing method, a laser printing method, and/or a laser induced thermal imaging (LITI) method.

In one or more embodiments, the electron transport region ETR may include a compound represented by Formula ET-2.

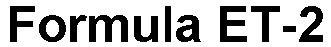

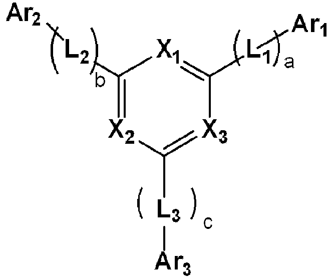

In Formula ET-2, at least one selected from among X₁ to X₃ may be N and the rest are CRₐ. Rₐ may be hydrogen, deuterium, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁ to Ar₃ may each independently be hydrogen, deuterium, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-2, a to c may each independently be an integer of 0 to 10. In Formula ET-2, L₁ to L₃ may each independently be a direct linkage, a substituted or unsubstituted arylene group having 6 to 30 ring-forming carbon atoms, or a substituted or unsubstituted heteroarylene group having 2 to 30 ring-forming carbon atoms. In one or more embodiments, if (e.g., when) a to c are an integer of 2 or greater, L₁'s to L₃'s may each independently be a substituted or unsubstituted arylene group having 6 to 30 ring-forming carbon atoms or a substituted or unsubstituted heteroarylene group having 2 to 30 ring-forming carbon atoms.

In one or more embodiments, the electron transport region ETR may include an anthracene-based compound. However, embodiments of the present disclosure are not limited thereto, and the electron transport region ETR may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(biphenyl-4-yl)-4-phenyl-5-tert-butylphenyl-1,2,4-triazole (TAZ), 4-(naphthalen-1-yl)-3,5-diphenyl-4H-1,2,4-triazole (NTAZ), 2-(biphenyl-4-yl)-5-(4-tert-butylphenyl)-1,3,4-oxadiazole (tBu-PBD), bis(2-methyl-8-quinolinolato-N1,O8)-(1,1'-biphenyl-4-olato)aluminium (BAlq), berylliumbis(benzoquinolin-10-olate) (Bebq₂), 9,10-di(naphthalen-2-yl)anthracene (ADN), 1,3-bis[3,5-di(pyridin-3-yl)phenyl]benzene (BmPyPhB), or a mixture thereof.

In one or more embodiments, the electron transport region ETR may include at least one selected from among compounds ET1 to ET36.

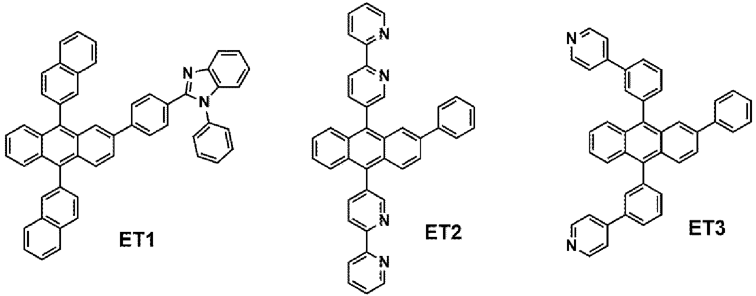

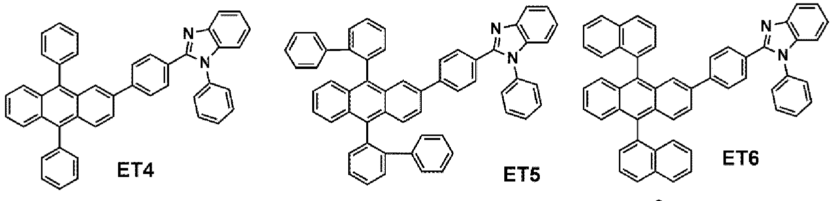

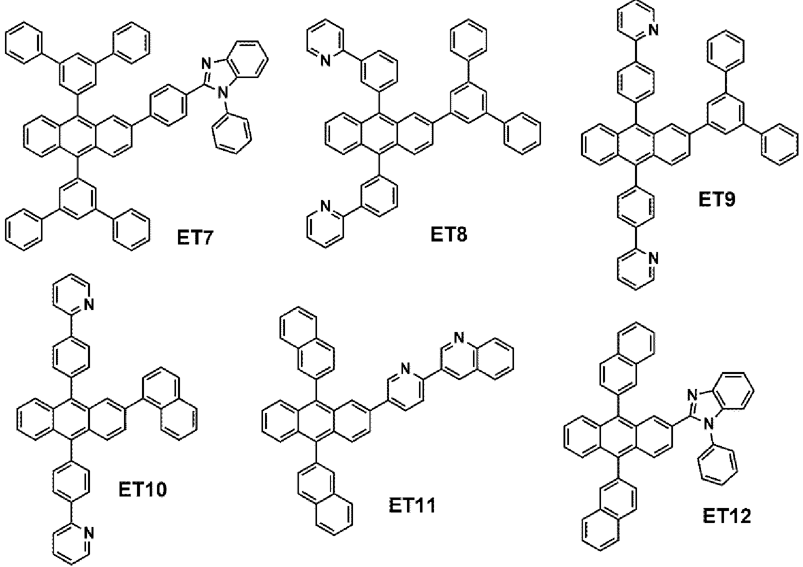

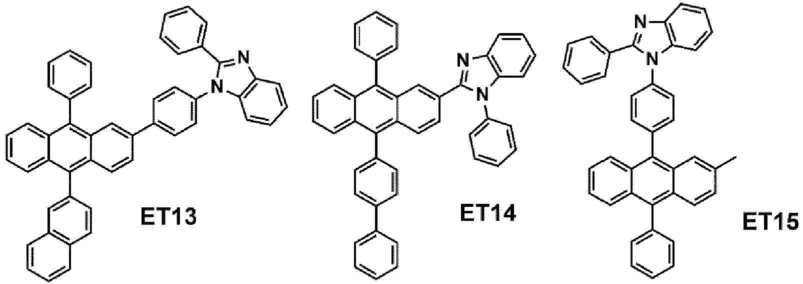

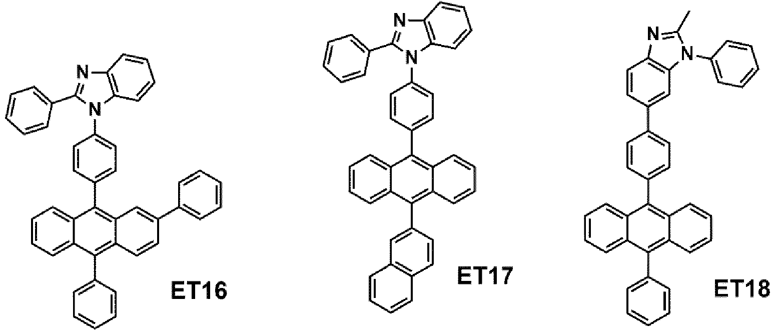

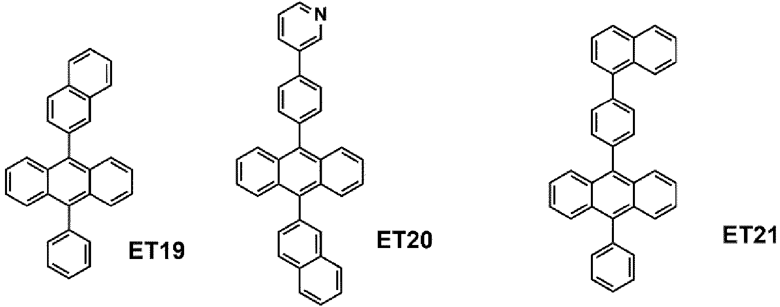

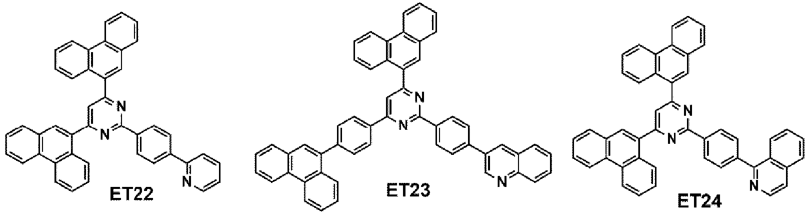

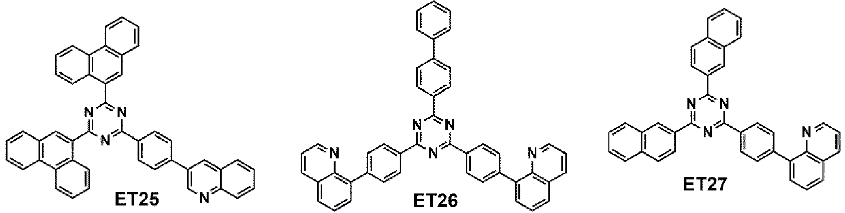

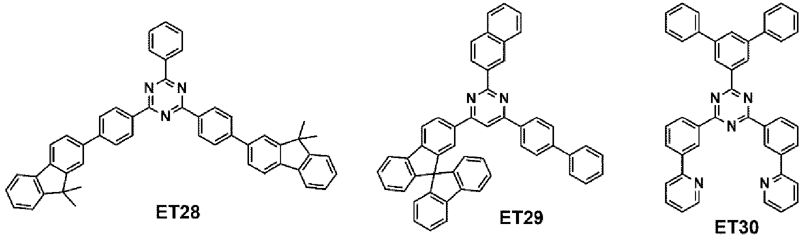

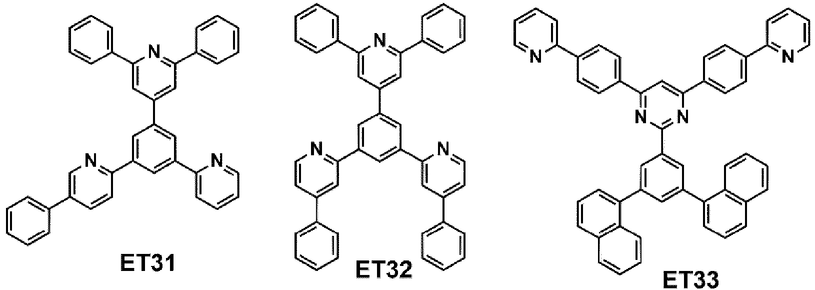

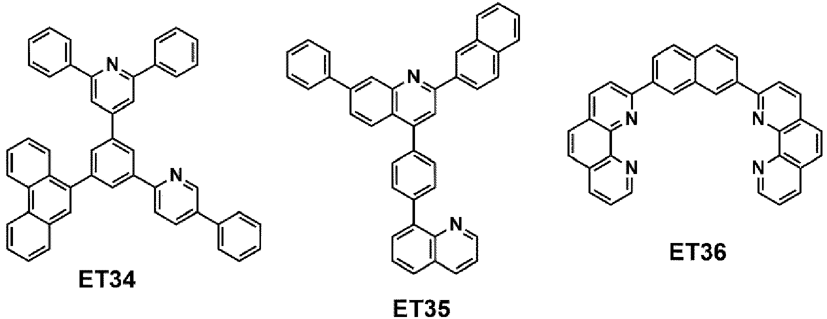

In one or more embodiments, the electron transport region ETR may include one or more halogenated metals (e.g., metal halides) such as LiF, NaCl, CsF, RbCl, RbI, Cul, and/or KI, one or more lanthanide metals such as Yb, or co-deposition materials of a halogenated metal and a lanthanide metal. For example, in one or more embodiments, the electron transport region ETR may include KI:Yb, RbI:Yb, LiF:Yb, and/or the like as the co-deposition material. In one or more embodiments, for the electron transport region ETR, a metal oxide such as Li₂O and BaO, lithium-8-hydroxyquinolinolate (Liq), and/or the like may be used, but embodiments of the present disclosure are limited thereto. The electron transport region ETR may also be formed of a mixture material of an electron transport material and an insulating organo-metal salt. The organo-metal salt may be a material having an energy band gap of about 4 eV or greater. For example, the organo-metal salt may include, for example, one or more selected from among metal acetates, metal benzoates, metal acetoacetates, metal acetylacetonates, or metal stearates.

In one or more embodiments, the electron transport region ETR may further include, for example, at least one of 2,9-dimethyl-4,7-diphenyl-1,10-phenanthroline (BCP), diphenyl(4-(triphenylsilyl)phenyl)phosphine oxide (TSPO1), or 4,7-diphenyl-1,10-phenanthroline (Bphen) in addition to one or more of the materials described above, but embodiments of the present disclosure are not limited thereto.

The electron transport region ETR may include one or more of the compounds of the electron transport region described above in at least one selected from among the electron injection layer EIL, the electron transport layer ETL, and the hole blocking layer HBL.

If (e.g., when) the electron transport region ETR includes the electron transport layer ETL, the electron transport layer ETL may have a thickness of about 100 Å to about 1000 Å, for example, about 150 Å to about 500 Å. When the thickness of the electron transport layer ETL satisfies the above-described ranges, satisfactory electron transport properties may be obtained without a substantial increase in driving voltage. If (e.g., when) the electron transport region ETR includes the electron injection layer EIL, the electron injection layer EIL may have a thickness of about 1 Å to about 100 Å, for example, about 3 Å to about 90 Å. When the thickness of the electron injection layer EIL satisfies the above-described ranges, satisfactory electron injection properties may be obtained without a substantial increase in driving voltage.

The second electrode EL2 may be provided on the electron transport region ETR. The second electrode EL2 may be a common electrode. The second electrode EL2 may be a cathode or an anode, but embodiments of the present disclosure are not limited thereto. For example, if (e.g., when) the first electrode EL1 is an anode, the second electrode EL2 may be a cathode, and if (e.g., when) the first electrode EL1 is a cathode, the second electrode EL2 may be an anode.

The second electrode EL2 may be a transmissive electrode, a transflective electrode, or a reflective electrode. When the second electrode EL2 is a transmissive electrode, the second electrode EL2 may be formed of a transparent metal oxide, for example, indium tin oxide (ITO), indium zinc oxide (IZO), zinc oxide (ZnO), indium tin zinc oxide (ITZO), and/or the like.

When the second electrode EL2 is a transflective electrode or a reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, Na, a compound thereof, or a mixture thereof (e.g., AgMg, AgYb, MgYb, AgLi, or AgNa). In one or more embodiments, the second electrode EL2 may have a multilayer structure including a reflective film or a transflective film formed of one or more of the above-described materials, and a transparent conductive film formed of indium tin oxide (ITO), indium zinc oxide (IZO), zinc oxide (ZnO), indium tin zinc oxide (ITZO), and/or the like. For example, in one or more embodiments, the second electrode EL2 may include one of the above-described metal materials, a combination of two or more metal materials selected from among the above-described metal materials, or an oxide of the above-described metal materials.

In one or more embodiments, the second electrode EL2 may be connected with an auxiliary electrode. If (e.g., when) the second electrode EL2 is connected with the auxiliary electrode, the resistance of the second electrode EL2 may decrease.

In one or more embodiments, a capping layer CPL may be further arranged on the second electrode EL2 of the light emitting element ED. The capping layer CPL may include a multilayer or a single layer.

In one or more embodiments, the capping layer CPL may be an organic layer or an inorganic layer. For example, if (e.g., when) the capping layer CPL includes an inorganic material, the inorganic material may include an alkali metal compound such as LiF, an alkaline earth metal compound such as MgF₂, SiON, SiNₓ, SiO_{y}, and/or the like.

In one or more embodiments, if (e.g., when) the capping layer CPL includes an organic material, the organic material may include 2,2'-dimethyl-N,N'-di-[(1-naphthyl)-N,N'-diphenyl]-1,1'-biphenyl-4,4'-diamine (α-NPD), NPB, TPD, m-MTDATA, Alq₃ CuPc, N4,N4,N4',N4'-tetra(biphenyl-4-yl) biphenyl-4,4'-diamine (TPD15), 4,4',4"-tris(carbazol-9-yl) triphenylamine (TCTA), and/or the like or may include one or more of epoxy resins and/or acrylates such as methacrylates. However, embodiments of the present disclosure are not limited thereto, for example, the capping layer CPL may include at least one selected from among compounds P1 to P5.

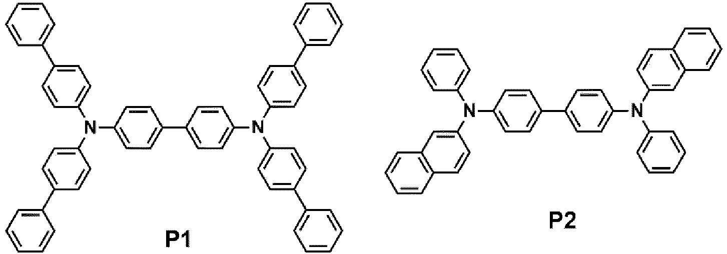

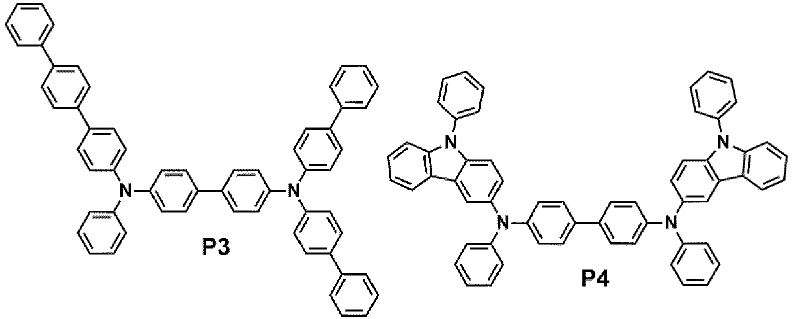

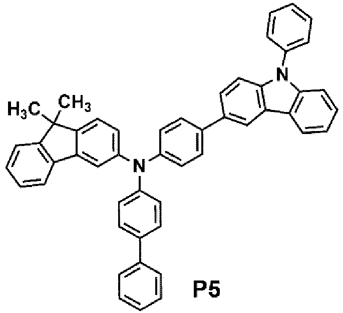

In one or more embodiments, the capping layer CPL may have a refractive index of about 1.6 or greater. For example, the capping layer CPL may have a refractive index of about 1.6 or greater in a wavelength range of about 550 nm to about 660 nm.

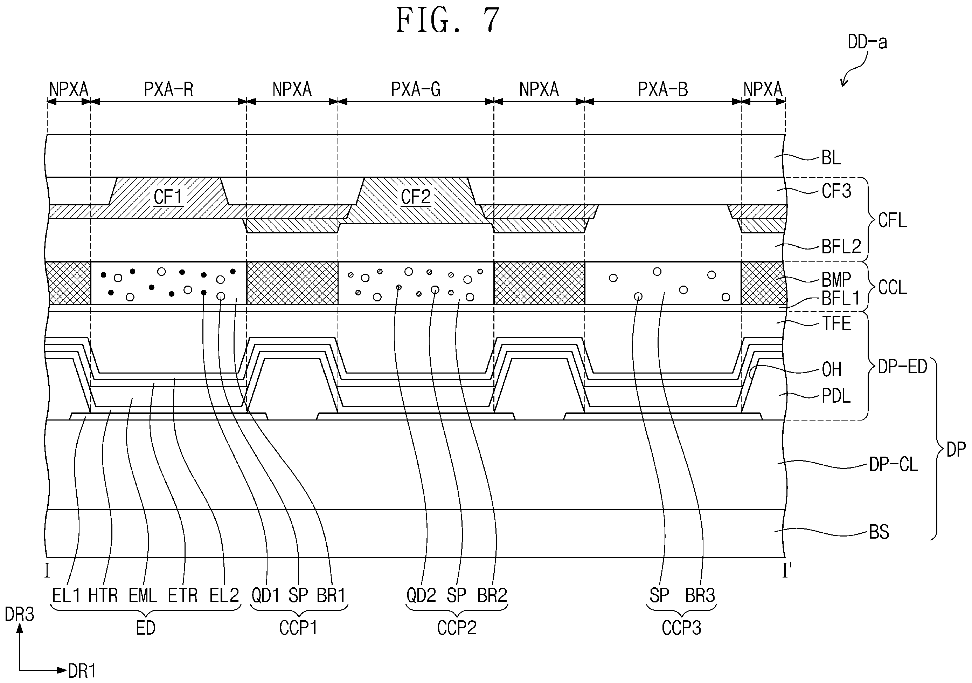

FIGS. 7 to 10 are each a cross-sectional view of a display device according to one or more embodiments of the present disclosure. Hereinafter, in the description of the display device according to one or more embodiments with reference to FIGS. 7 to 10, content overlapping ones described above with reference to FIGS. 1 to 6 will not be described again, only the differences will be mainly described.

Referring to FIG. 7, a display device DD-a according to one or more embodiments may include a display panel DP having a display element layer DP-ED, a light control layer CCL arranged on the display panel DP, and a color filter layer CFL. In one or more embodiments shown in FIG. 7, the display panel DP may include a base layer BS, a circuit layer DP-CL provided on the base layer BS, and a display element layer DP-ED, and the display element layer DP-ED may include a light emitting element ED.

The light emitting element ED includes a first electrode EL1, a hole transport region HTR arranged on the first electrode EL1, an emission layer EML arranged on the hole transport region HTR, an electron transport region ETR arranged on the emission layer EML, and a second electrode EL2 arranged on the electron transport region ETR. In one or more embodiments, a structure of the light emitting element ED shown in FIG. 7 may be the same as the structure of one of the light emitting elements of FIGS. 3 to 6 described above. The light emitting element ED shown in FIG. 7 includes a monoamine compound of one or more embodiments of the present disclosure. The light emitting element ED including the monoamine compound of one or more embodiments may exhibit high luminous efficiency and long-life characteristics.

Referring to FIG. 7, the emission layer EML may be arranged in openings OH defined in pixel defining films PDL. For example, the emission layer EML separated by the pixel defining films PDL and provided corresponding to each of light emitting regions PXA-R, PXA-G, and PXA-B may be to emit light in substantially the same wavelength ranges. In the display device DD-a of one or more embodiments, the emission layer EML may be to emit blue light. In one or more embodiments, the emission layer EML may be provided as a common layer throughout the light emitting regions PXA-R, PXA-G, and PXA-B.

The light control layer CCL may be arranged on the display panel DP. The light control layer CCL may include a light converter. The light converter may be a quantum dot or a phosphor. The light converter may wavelength-convert the provided light and emit the wavelength-converted light. For example, the light control layer CCL may be a layer containing quantum dots or phosphors.

The light control layer CCL may include a plurality of light control units CCP1, CCP2, and CCP3. The light control units CCP1, CCP2, and CCP3 may be spaced apart from one another.

Referring to FIG. 7, a division pattern BMP may be arranged between the light control units CCP1, CCP2, and CCP3 spaced and/or apart from one another, but embodiments of the present disclosure are not limited thereto. In FIG. 7, the division pattern BMP is shown to nonoverlap the light control units CCP1, CCP2, and CCP3, but, in one or more embodiments, edges of the light control units CCP1, CCP2, and CCP3 may each overlap at least a portion of the division pattern BMP.

The light control layer CCL may include a first light control unit CCP1 including a first quantum dot QD1 for converting first color light provided from the light emitting element ED into second color light, a second light control unit CCP2 including a second quantum dot QD2 for converting the first color light into third color light, and a third light control unit CCP3 for transmitting the first color light.

In one or more embodiments, the first light control unit CCP1 may provide red light, which is the second color light, and the second light control unit CCP2 may provide green light, which is the third color light. The third light control unit CCP3 may be to transmit and provide blue light, which is the first color light provided from the light emitting element ED. For example, the first quantum dot QD1 may be a red quantum dot to emit red light, and the second quantum dot QD2 may be a green quantum dot to emit green light. The same descriptions above on quantum dots may be applied to the quantum dots QD1 and QD2.

In one or more embodiments, the light control layer CCL may further include scatterers SP. The first light control unit CCP1 may include the first quantum dot QD1 and the scatterers SP, the second light control unit CCP2 may include the second quantum dot QD2 and the scatterers SP, and the third light control unit CCP3 may not include (e.g., may exclude) a quantum dot but may include the scatterers SP.

The scatterers SP may be inorganic particles. For example, the scatterers SP may include at least one selected from among TiO₂, ZnO, Al₂O₃, SiO₂, and hollow silica. In one or more embodiments, the scatterers SP may include any one selected from among TiO₂, ZnO, Al₂O₃, SiO₂, and hollow silica, or may be a mixture of two or more materials selected from among TiO₂, ZnO, Al₂O₃, SiO₂, and hollow silica.

The first light control unit CCP1, the second light control unit CCP2, and the third light control unit CCP3 may respectively include base resins BR1, BR2, and BR3 for dispersing the quantum dots QD1 and QD2 and the scatterers SP accordingly. In one or more embodiments, the first light control unit CCP1 may include the first quantum dot QD1 and the scatterers SP each dispersed in a first base resin BR1, the second light control unit CCP2 may include the second quantum dot QD2 and the scatterers SP each dispersed in a second base resin BR2, and the third light control unit CCP3 may include the scatterers SP dispersed in a third base resin BR3.

The base resins BR1, BR2, and BR3 are a medium in which the quantum dots QD1 and QD2 and the scatterers SP are dispersed accordingly, and may each be formed of one or more suitable resin compositions, which may be generally referred to as a binder. For example, the base resins BR1, BR2, and BR3 may each independently be an acrylic-based resin, a urethane-based resin, a silicone-based resin, an epoxy-based resin, and/or the like. Base resins BR1, BR2, and BR3 may each be transparent resins. In one or more embodiments, the first base resin BR1, the second base resin BR2, and the third base resin BR3 may be the same as or different from one another.

In one or more embodiments, the light control layer CCL may include a barrier layer BFL1. The barrier layer BFL1 may serve to prevent or reduce moisture and/or oxygen (hereinafter referred to as "moisture/oxygen") from being introduced. The barrier layer BFL1 may be arranged on the light control units CCP1, CCP2, and CCP3 to prevent or reduce the light control units CCP1, CCP2, and CCP3 from being exposed to moisture/oxygen. In one or more embodiments, the barrier layer BFL1 may cover the light control units CCP1, CCP2, and CCP3. In one or more embodiments, a barrier layer BFL2 may be provided between the light control units CCP1, CCP2, and CCP3 and the filters CF1, CF2, and CF3.

The barrier layers BFL1 and BFL2 may each include at least one inorganic layer. For example, in one or more embodiments, the barrier layers BFL1 and BFL2 may each be formed of an inorganic material. For example, the barrier layers BFL1 and BFL2 may each independently be formed by including silicon nitride, aluminium nitride, zirconium nitride, titanium nitride, hafnium nitride, tantalum nitride, silicon oxide, aluminium oxide, titanium oxide, tin oxide, cerium oxide, silicon oxynitride, or a metal thin film in which light transmittance is secured, and/or the like. In one or more embodiments, the barrier layers BFL1 and BFL2 may each independently further include an organic film. The barrier layers BFL1 and BFL2 may each independently be formed of a single layer or a plurality of layers.

In the display device DD-a of one or more embodiments, the color filter layer CFL may be arranged on the light control layer CCL. For example, in one or more embodiments, the color filter layer CFL may be directly arranged on the light control layer CCL. In these embodiments, the barrier layer BFL2 may not be provided.

The color filter layer CFL may include filters CF1, CF2, and CF3. For example, in one or more embodiments, the color filter layer CFL may include a first filter CF1 transmitting the second color light, a second filter CF2 transmitting the third color light, and a third filter CF3 transmitting the first color light. For example, the first filter CF1 may be a red filter, the second filter CF2 may be a green filter, and the third filter CF3 may be a blue filter. The filters CF1, CF2, and CF3 may each include a polymer photosensitive resin, a pigment and/or a dye. For example, in one or more embodiments, the first filter CF1 may include a red pigment and/or a red dye, the second filter CF2 may include a green pigment and/or a green dye, and the third filter CF3 may include a blue pigment and/or a blue dye.

Embodiments of the present disclosure are not limited thereto, for example, the third filter CF3 may not include (e.g., may exclude) any pigment or any dye. The third filter CF3 may include a polymer photosensitive resin, but not include any pigment or any dye. The third filter CF3 may be transparent. The third filter CF3 may be formed of a transparent photosensitive resin.

In one or more embodiments, the first filter CF1 and the second filter CF2 may be yellow filters. The first filter CF1 and the second filter CF2 may not be separated and may be provided as a single body.

In one or more embodiments, the color filter layer CFL may further include a light blocking unit. The light blocking unit may be a black matrix. The light blocking unit may be formed including an organic light blocking material and/or an inorganic light blocking material, each including a black pigment and/or a black dye. The light blocking unit may prevent or reduce light leakage, and separate boundaries between adjacent filters CF1, CF2, and CF3. In one or more embodiments, the light blocking unit may be formed with a blue filter.

The first to third filters CF1, CF2, and CF3 may be arranged corresponding to the red light emitting region PXA-R, the green light emitting region PXA-G, and the blue light emitting region PXA-B, respectively.

A base substrate BL may be arranged on the color filter layer CFL. The base substrate BL may be a member providing a base surface on which the color filter layer CFL and the light control layer CCL are arranged. The base substrate BL may be a glass substrate, a metal substrate, a plastic substrate, and/or the like. However, embodiments of the present disclosure are not limited thereto, and the base substrate BL may be an inorganic layer, an organic layer, or a composite material layer. In one or more embodiments, the base substrate BL may not be provided.

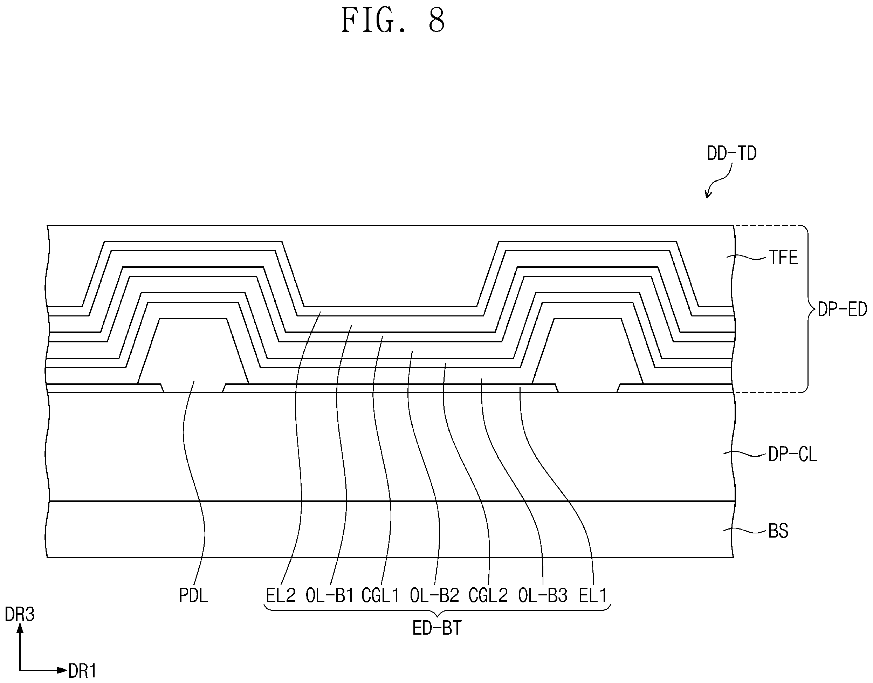

FIG. 8 is a cross-sectional view showing a portion of a display device according to one or more embodiments of the present disclosure. In a display device DD-TD of one or more embodiments, a light emitting element ED-BT may include a plurality of light emitting structures OL-B1, OL-B2, and OL-B3. The light emitting element ED-BT may include a first electrode EL1 and a second electrode EL2 opposite to (e.g., facing) each other, and the plurality of light emitting structures OL-B1, OL-B2, and OL-B3 provided by being sequentially stacked in a thickness direction between the first electrode EL1 and the second electrode EL2. The light emitting structures OL-B1, OL-B2, and OL-B3 each may include an emission layer EML (FIG. 7), a hole transport region HTR and an electron transport region ETR arranged with the emission layer EML (FIG. 7) therebetween. For example, the light emitting element ED-BT included in the display device DD-TD of one or more embodiments may be a light emitting element having a tandem structure including a plurality of emission layers.

The light emitting element ED-BT shown in FIG. 8 includes a monoamine compound of one or more embodiments. The light emitting element ED-BT including the monoamine compound of one or more embodiments may exhibit high luminous efficiency and long-life characteristics.

In one or more embodiments shown in FIG. 8, light emitted from each of the light emitting structures OL-B1, OL-B2, and OL-B3 may all be blue light. However, embodiments of the present disclosure are not limited thereto, for example, in one or more embodiments, wavelength ranges of light emitted from each of the light emitting structures OL-B1, OL-B2, and OL-B3 may be different from one another. For example, in one or more embodiments, the light emitting element ED-BT including the plurality of light emitting structures OL-B1, OL-B2, and OL-B3 emitting light in different wavelength ranges may be to emit white light (e.g., combined white light).

Charge generation layers CGL1 and CGL2 may be respectively arranged between neighboring light emitting structures OL-B1, OL-B2, and OL-B3. The charge generation layers CGL1 and CGL2 may include a p-type (kind) charge generation layer and/or an n-type (kind) charge generation layer.

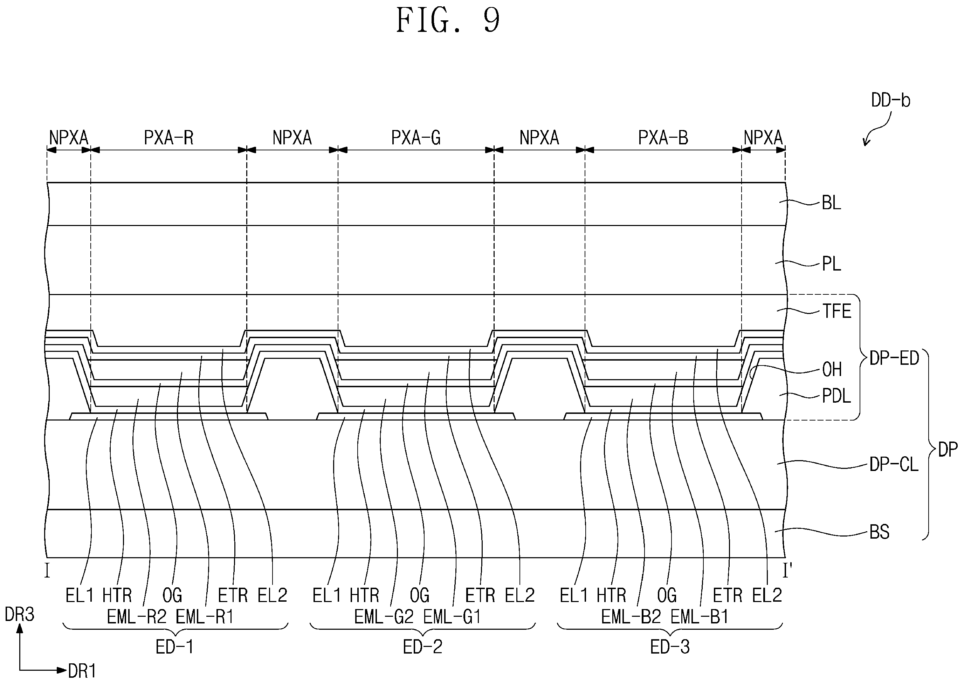

Referring to FIG. 9, a display device DD-b according to one or more embodiments may include light emitting elements ED-1, ED-2, and ED-3 in each of which two emission layers are stacked. Compared to the display device DD shown in FIG. 2, the difference is that in one or more embodiments shown in FIG. 9, the first to third light emitting elements ED-1, ED-2, and ED-3 each include two emission layers stacked in a thickness direction. In each of the first to third light emitting elements ED-1, ED-2, and ED-3, the two emission layers may be to emit light in substantially the same wavelength range. At least one selected from among the first to third light emitting elements ED-1, ED-2, and ED-3 includes the monoamine compound of one or more embodiments. The light emitting element (at least one of ED-1, ED-2, or ED-3) including the monoamine compound of one or more embodiments may exhibit high luminous efficiency and long-life characteristics.

In one or more embodiments, the first light emitting element ED-1 may include a first red emission layer EML-R1 and a second red emission layer EML-R2. The second light emitting element ED-2 may include a first green emission layer EML-G1 and a second green emission layer EML-G2. In addition, the third light emitting element ED-3 may include a first blue emission layer EML-B1 and a second blue emission layer EML-B2. A light emitting auxiliary portion OG may be arranged between the first red emission layer EML-R1 and the second red emission layer EML-R2, between the first green emission layer EML-G1 and the second green emission layer EML-G2, and between the first blue emission layer EML-B1 and the second blue emission layer EML-B2.

The light emitting auxiliary portion OG may include a single layer or multiple layers. The light emitting auxiliary portion OG may include a charge generation layer. For example, in one or more embodiments, the light emitting auxiliary portion OG may include an electron transport region, a charge generation layer, and a hole transport region that are sequentially stacked (e.g., in the stated order). The light emitting auxiliary portion OG may be provided as a common layer throughout the first to third light emitting elements ED-1, ED-2, and ED-3. However, embodiments of the present disclosure are not limited thereto, and the light emitting auxiliary portion OG may be provided to be patterned inside openings OH defined in pixel defining films PDL.

The first red emission layer EML-R1, the first green emission layer EML-G1, and the first blue emission layer EML-B1 may each be arranged between the light emitting auxiliary portion OG and the electron transport region ETR. The second red emission layer EML-R2, the second green emission layer EML-G2, and the second blue emission layer EML-B2 may each be arranged between the hole transport region HTR and the light emitting auxiliary portion OG.

For example, in one or more embodiments, the light emitting element ED-1 may include a first electrode EL1, a hole transport region HTR, a second red emission layer EML-R2, an emission auxiliary portion OG, a first red emission layer EML-R1, an electron transport region ETR, and a second electrode EL2, which are sequentially stacked (e.g., in the stated order). The second light emitting element ED-2 may include a first electrode EL1, a hole transport region HTR, a second green emission layer EML-G2, an emission auxiliary portion OG, a first green emission layer EML-G1, an electron transport region ETR, and a second electrode EL2, which are sequentially stacked (e.g., in the stated order). The third light emitting element ED-3 may include a first electrode EL1, a hole transport region HTR, a second blue emission layer EML-B2, an emission auxiliary portion OG, a first blue emission layer EML-B1, an electron transport region ETR, and a second electrode EL2, which are sequentially stacked (e.g., in the stated order).

In one or more embodiments, an optical auxiliary layer PL may be arranged on the display element layer DP-ED. The optical auxiliary layer PL may include a polarizing layer. The optical auxiliary layer PL may be arranged on the display panel DP to control reflected light in the display panel DP due to external light. In one or more embodiments, the optical auxiliary layer PL may not be provided in the display device DD-b.

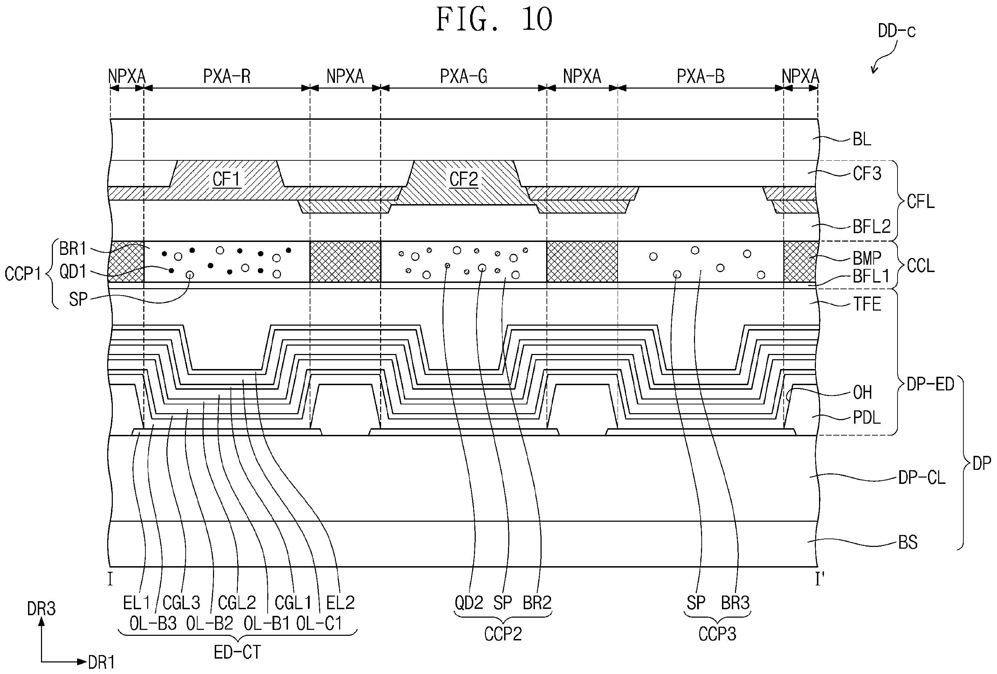

Different from FIG. 8 and FIG. 9, a display device DD-c of FIG. 10 is shown to include four light emitting structures OL-B1, OL-B2, OL-B3, and OL-C1. The light emitting element ED-CT may include a first electrode EL1 and a second electrode EL2 opposite to (e.g., facing) each other, and the first to fourth light emitting structures OL-B1, OL-B2, OL-B3, and OL-C1 arranged between the first electrode EL1 and the second electrode EL2. The third light emitting structure OL-B3, the second light emitting structure OL-B2, the first light emitting structure OL-B1, and the fourth light emitting structure OL-C1 are sequentially stacked in a thickness direction between the first electrode EL1 and the second electrode EL2. A first charge generation layer CGL1 may be arranged between the first light emitting structure OL-B1 and the fourth light emitting structure OL-C1. A second charge generation layer CGL2 may be arranged between the second light emitting structure OL-B2 and the first light emitting structure OL-B1. A third charge generation layer CGL3 may be arranged between the third light emitting structure OL-B3 and the second light emitting structure OL-B2. The light emitting element ED-CT shown in FIG. 10 includes a monoamine compound of one or more embodiments. The light emitting element ED-CT including the monoamine compound of one or more embodiments may exhibit high luminous efficiency and long-life characteristics.

In one or more embodiments, among the four light emitting structures, the first to third light emitting structures OL-B1, OL-B2, and OL-B3 may be to emit blue light, and the fourth light emitting structure OL-C1 may be to emit green light. However, embodiments of the present disclosure are not limited thereto, for example, the first to fourth light emitting structures OL-B1, OL-B2, OL-B3, and OL-C1 may be to emit light having different wavelength ranges.

The charge generation layers CGL3, CGL2 and CGL1 separately arranged between neighboring light emitting structures OL-B3, OL-B2, OL-B1, and OL-C1 may include a p-type (kind) charge generation layer and/or an n-type (kind) charge generation layer.

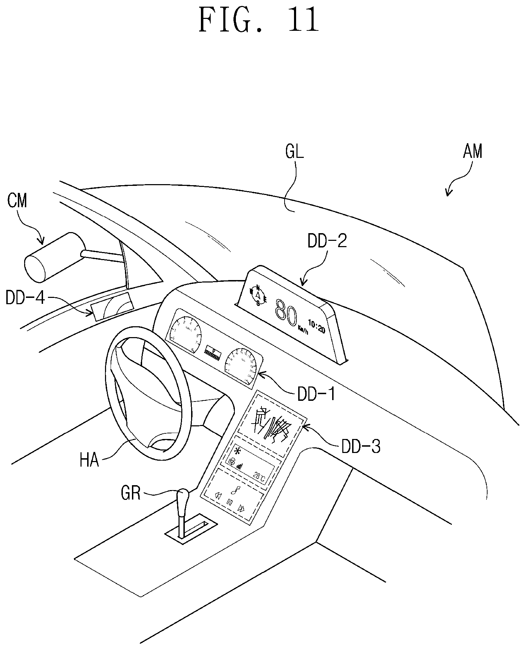

FIG. 11 is a diagram showing a vehicle AM in which first to fourth display devices DD-1, DD-2, DD-3, and DD-4 are arranged. At least one selected from among the first to fourth display devices DD-1, DD-2, DD-3, and DD-4 may have the same configuration as those of the display devices DD, DD-TD, DD-a, DD-b, and DD-c of embodiments described with reference to FIGS. 1, 2, and 7 to 10.

FIG. 11 shows a car as the vehicle AM, but this is presented as an example, and the first to fourth display devices DD-1, DD-2, DD-3, and DD-4 may be arranged on other means of transportation, such as bicycles, motorcycles, trains, ships, and/or airplanes. In one or more embodiments, at least one selected from among the first to fourth display devices DD-1, DD-2, DD-3, and DD-4 having the same configuration as those of the display devices DD, DD-TD, DD-a, DD-b, and DD-c of one or more embodiments may be adopted for personal computers, laptop computers, personal digital terminals, game consoles, portable electronic devices, televisions, monitors, outdoor billboards, and/or the like. In addition, these are merely presented as examples, and thus the display device may be adopted for other electronic apparatuses without departing from the disclosure.

At least one selected from among the first to fourth display devices DD-1, DD-2, DD-3, or DD-4 may include the light emitting element ED described with reference to FIGS. 3 to 6. At least one selected from among the first to fourth display devices DD-1, DD-2, DD-3, and DD-4 includes a monoamine compound of one or more embodiments. The display device (at least one of DD-1, DD-2, DD-3, or DD-4) including the monoamine compound of one or more embodiments may exhibit excellent or suitable display efficiency and display lifespan.

Referring to FIG. 11, the vehicle AM may include a steering wheel HA and a gear GR for operating the vehicle AM, and may have a front window GL arranged to face a driver.

The first display device DD-1 may be arranged in a first region overlapping the steering wheel HA. For example, the first display device DD-1 may be a digital cluster displaying first information of the vehicle AM. The first information may include a first scale indicating driving speed of the vehicle AM, a second scale indicating engine revolutions (i.e., revolutions per minute (RPM)), and an image indicating fuel gauge, and/or the like. The first scale and the second scale may be displayed as digital images.

The second display device DD-2 may be arranged in a second region opposite to (e.g., facing) a driver seat and overlapping the front window GL. The driver seat may be a seat in which the steering wheel HA faces. For example, the second display device DD-2 may be a head up display (HUD) displaying second information of the vehicle AM. The second display device DD-2 may be optically transparent. The second information includes a digital number (DN) indicating driving speed of the vehicle AM and may further include information such as current time. In one or more embodiments, the second information of the second display device DD-2 may be projected and displayed on the front window GL.

The third display device DD-3 may be arranged in a third region adjacent to the gear GR. For example, the third display device DD-3 may be a center information display (CID) for the vehicle, which is arranged between a driver seat and a front passenger seat, and displays third information. The passenger seat may be a seat spaced apart from the driver seat with the gear GR therebetween. The third information may include information about road conditions (e.g., navigation information), music or radio play, dynamic video play, temperature inside the vehicle AM, and/or the like.

The fourth display device DD-4 may be arranged in a fourth region spaced apart from the steering wheel HA and the gear GR and adjacent to a side of the vehicle AM. For example, the fourth display device DD-4 may be a digital side mirror displaying fourth information. The fourth display device DD-4 may display images of conditions outside the vehicle AM, which are taken by a camera module CM arranged outside the vehicle AM. The fourth information may include images of conditions outside the vehicle AM.

The first to fourth information described above are presented as an example, and the first to fourth display devices DD-1, DD-2, DD-3, and DD-4 may further display information about inside or outside the vehicle. The first to fourth information may include different information. However, embodiments of the present disclosure are not limited thereto, and some of the first to fourth information may include the same information.

In one or more embodiments, an electronic apparatus may include a display device including a plurality of light emitting elements, and a control portion configured to control the display device. The electronic apparatus of one or more embodiments may be a device that is activated in response to electrical signals. The electronic apparatus may include display devices of one or more embodiments of the present disclosure. For example, in one or more embodiments, the electronic apparatus may include large-sized display devices such as televisions, monitors, or outdoor billboards, as well as small- and medium-sized display devices such as personal computers, laptop computers, personal digital terminals, in-vehicle display devices, game consoles, portable electronic devices, or cameras. In one or more embodiments, the electronic apparatus may include at least one of a flat panel display, a curved display, a computer monitor, a medical monitor, a television, a billboard, an indoor light, an outdoor light, a light for signaling, a head-up display, a fully transparent display, a partially transparent display, a flexible display, a rollable display, a foldable display, a stretchable display, a laser printer, a telephone, a portable phone, a tablet personal computer, a phablet, a personal digital assistant, a wearable device, a laptop computer, a digital camera, a camcorder, a viewfinder, a micro display, a three-dimension (3D) display, a virtual reality display, an augmented reality display, a vehicle, a video wall with multiple displays tiled together, a theater screen, a stadium screen, a phototherapy device, or a signboard.

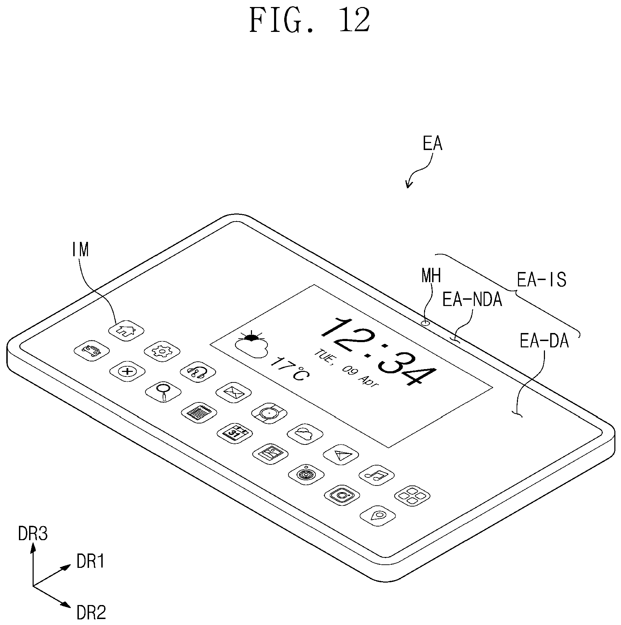

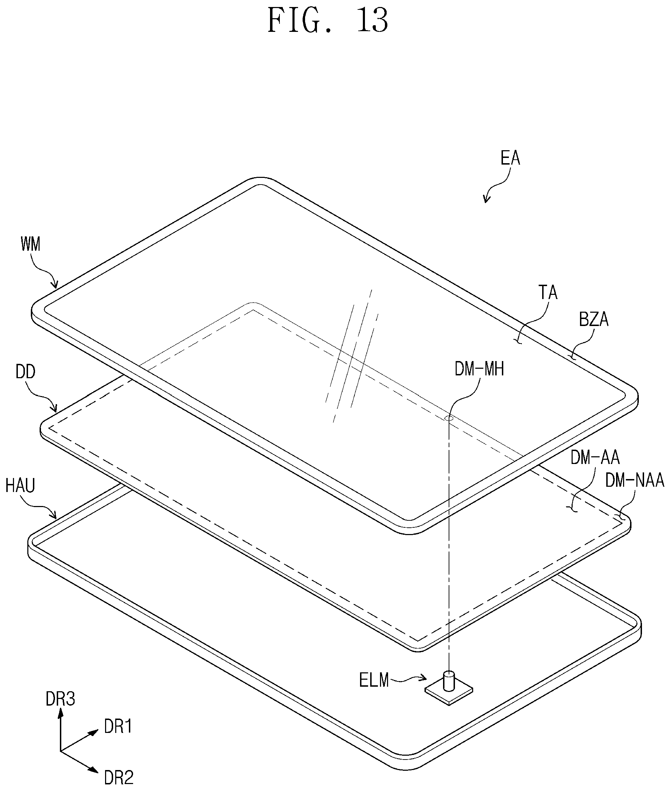

FIG. 12 is a perspective view showing an electronic apparatus of one or more embodiments of the present disclosure. FIG. 13 is an exploded perspective view showing an electronic apparatus of one or more embodiments.

An electronic apparatus EA may display an image IM through a display surface EA-IS. The image IM may include still images as well as dynamic images. The display surface EA-IS may be parallel to a plane defined by a first direction axis DR1 and a second direction axis DR2. FIG. 12 shows the electronic apparatus EA provided with the flat display surface EA-IS, but embodiments of the present disclosure are not limited thereto. For example, the electronic apparatus EA may include a curved display surface or a three-dimensional display surface. The three-dimensional display surface may include a plurality of display regions indicating different directions.

The display surface EA-IS may include a display region EA-DA and a non-display region EA-NDA. The display surface EA-IS may further include a sub region MH. The electronic apparatus EA may display the image IM through the display region EA-DA.

The non-display region EA-NDA may have a set or predetermined color. The non-display region EA-NDA may be adjacent to the display region EA-DA. The non-display region EA-NDA may surround the display region EA-DA. Accordingly, the shape of the display region EA-DA may be defined substantially by the non-display region EA-NDA. However, FIG. 12 is presented as an example, and the non-display region EA-NDA may be arranged adjacent to only one side of the display region EA-DA, or may not be provided.

The sub region MH may detect an external subject received through the display surface EA-IS, or provide sound signals such as voice to the outside through the display surface EA-IS. A light signal such as visible light or infrared light may travel to the sub region MH.

In one or more embodiments, the sub region MH may be arranged within the display region EA-DA. However, this is presented as an example, and the arrangement of the sub region MH is not limited to any specific arrangement. For example, the sub region MH may be surrounded by the non-display region EA-NDA, or may be surrounded by the display region EA-DA and the non-display region EA-NDA. Although one sub region MH is shown in FIG. 12, a plurality of sub regions MH may be provided.

Various electronic modules ELM (see FIG. 13) may be arranged to correspond to the sub regions MH. For example, the electronic modules ELM (FIG. 13) may include at least one of a camera, a speaker, a light detection sensor, or a heat detection sensor. In one or more embodiments, the electronic apparatus EA may include an electronic module ELM (FIG. 13) that captures external images via visible light passing through the sub regions MH or determines the accessibility of external objects via infrared light. The electronic modules ELM (FIG. 13) may include a plurality of components, and are not limited to any one embodiment.

Referring to FIG. 13, the electronic apparatus EA may include a display device DD. In addition, the electronic apparatus EA may further include an electronic module ELM, a window member WM, and a housing HAU.

The window member WM may cover an entire outer portion of the electronic apparatus EA. The window member WM may include a transmission region TA and a bezel region BZA. A front surface of the window member WM including the transmission region TA and the bezel region BZA may serve as a front surface of the electronic apparatus EA. The transmission region TA may correspond to the display region EA-DA of the electronic apparatus EA shown in FIG. 12, and the bezel region BZA may correspond to the non-display region EA-NDA of the electronic apparatus EA shown in FIG. 12.

The transmission region TA may be an optically transparent region. The bezel region BZA may be a region having a relatively lower light transmittance than the transmission region TA. The bezel region BZA may have a set or predetermined color. The bezel region BZA may be adjacent to the transmission region TA and may surround the transmission region TA. The bezel region BZA may define a shape of the transmission region TA. However, embodiments of the present disclosure are not limited to what is shown, for example, the bezel region BA may be arranged adjacent to only one side of the transmission region TA, and a portion thereof may not be provided.

The housing HAU may include a material having a relatively greater rigidity. For example, in one or more embodiments, the housing HAU may include frames and/or plates formed of glass, plastic, or metal. The frames and/or plates may be provided in plurality. The housing HAU may provide a set or predetermined place/space for accommodation. The display device DD may be accommodated in the accommodation place/space to be protected from external shocks.

The display device DD may have the same configuration as at least one of the display devices DD, DD-TD, DD-a, DD-b, or DD-c of one or more embodiments described with reference to FIGS. 1, 2, and 7 to 10. The display device DD may include the light emitting element ED described with reference to FIGS. 3 to 6. Accordingly, the electronic apparatus EA including the display device DD according to one or more embodiments may exhibit excellent or suitable reliability.

The display device DD may define an active region DM-AA, a peripheral region DM-NAA, and a module region DM-MH. The active region DM-AA may overlap the display region EA-DA shown in FIG. 12, and the peripheral region DM-NAA may overlap the non-display region EA-NDA shown in FIG. 12. The module region DM-MH may overlap the sub region MH shown in FIG. 12.

The active region DM-AA may be a region activated according to electrical signals. The peripheral region DM-NAA may be a region adjacent to at least one side of the active region DM-AA. The active region DM-AA may include a non-light emitting region NPXA and light emitting regions PXA-R, PXA-G, and PXA-R shown in FIG. 1. The peripheral region DM-NAA may be around the active region DM-AA. However, embodiments of the present disclosure are not limited thereto, for example, in one or more embodiments, a portion of the peripheral region DM-NAA may not be provided unlike what is shown. A driving circuit, a driving line, and/or the like for driving the active region DM-AA may be arranged in the peripheral region DM-NAA.

Light signals such as visible light or infrared light may travel through the module region DM-MH. In one or more embodiments, the module region DM-MH may be arranged in the active region DM-AA. In one or more embodiments, the module region DM-MH may be surrounded by the peripheral region DM-NAA, or may be surrounded by the active region DM-AA and the peripheral region DM-NAA.

The electronic module ELM may be an electronic component that outputs or receives light signals. The electronic module ELM may include a camera module and/or a proximity sensor. The camera module may capture an external image through the module region DM-MH. However, embodiments of the present disclosure are not limited thereto, for example, in one or more embodiments, the electronic module ELM may further include a built-in module and/or an external module. The built-in module may include a sensor module, an antenna module, and/or an audio output module. The external module may include a light module and/or a communication module.

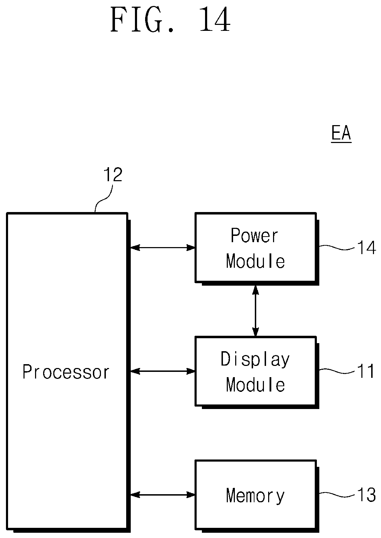

FIG. 14 is a block diagram of an electronic apparatus according to one or more embodiments of the present disclosure. Referring to FIG. 14, an electronic apparatus EA according to one or more embodiments may include a display module 11, a processor 12, a memory 13, and a power module 14.

The processor 12 may include at least one of a central processing unit (CPU), an application processor (AP), a graphic processing unit (GPU), a communication processor (CP), an image signal processor (ISP), or a controller.

The memory 13 may store data information desired or required for the operation of the processor 12 and/or the display module 11. When the processor 12 executes an application stored in the memory 13, image data signals and/or input control signals are transmitted to the display module 11, and the display module 11 may process the received signal and output image information through a display screen.

The power module 14 may include a power supply module such as a power adapter or a battery device, and a power conversion module that converts power supplied by the power supply module to generate power desired or required for the operation of the electronic apparatus EA.

At least one of the components of the electronic apparatus EA described above may be included in the display device according to the above-described embodiments. In one or more embodiments, some of the individual modules functionally included in one module may be included in the display device, and others may be separately provided from the display device. For example, the display device may include a display module 11, and the processor 12, the memory 13, and the power module 14 may be provided in the form of other devices within the electronic apparatus EA, rather than the display device.

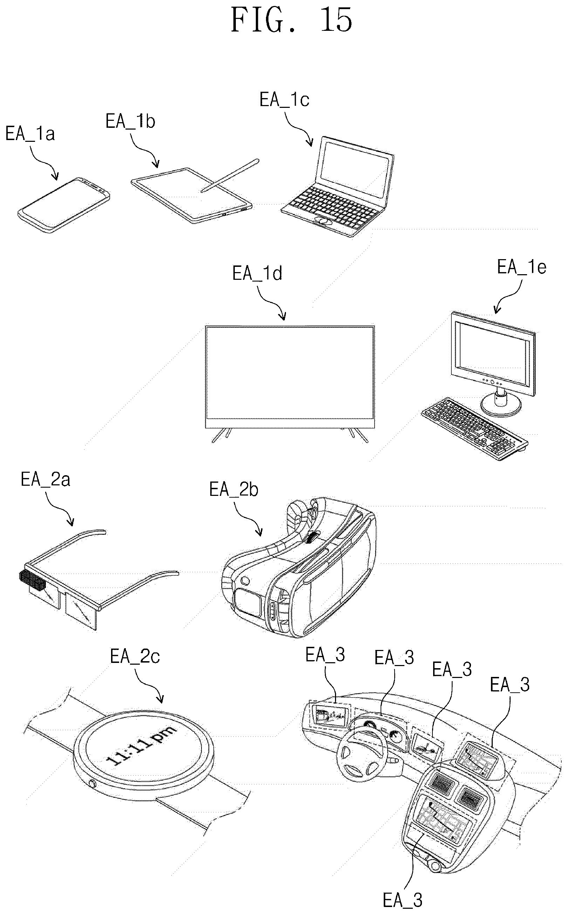

FIG. 15 shows schematic views of electronic apparatuses according to one or more embodiments of the present disclosure. Referring to FIG. 15, one or more suitable electronic apparatuses to which the display device according to one or more embodiments is applied may include an electronic apparatus for displaying images, such as a smart phone EA_1a, a tablet PC EA_1b, a laptop computer EA_1c, a TV EA_1d, and a desk monitor EA_1e, a wearable electronic apparatus including a display module such as a smart glasses EA_2a, a head mounted display EA_2b, and a smart watch EA_2c, and a vehicle electronic apparatus EA_3 including a display module such as a center information display (CID) and a room mirror display arranged on an instrument panel, a center fascia, or a dashboard of a vehicle.

Hereinafter, with reference to Examples and Comparative Examples, a monoamine compound according to one or more embodiments of the present disclosure and a light emitting element of one or more embodiments will be specifically described. In addition, Examples are shown only for the understanding of the disclosure, and the scope of the disclosure is not limited thereto.

### Examples

### 1. Synthesis of a monoamine compound of an embodiment

A process of synthesizing monoamine compounds according to one or more embodiments will be described in more detail by presenting a process of synthesizing monoamine Compounds 3, 8, 18, 39, 71, 94, 37, 44, 104, 81, 29, and 36 as an example. In addition, a process of synthesizing monoamine compounds, which will be described hereinafter, is provided as an example, and thus a process of synthesizing monoamine compounds according to one or more embodiments of the disclosure is not limited to Examples.

### (1) Synthesis of Intermediate Compounds

Intermediate Compounds used may be synthesized through the steps of Reaction Schemes 1-1 to 1-3.

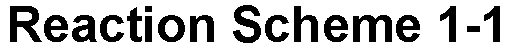

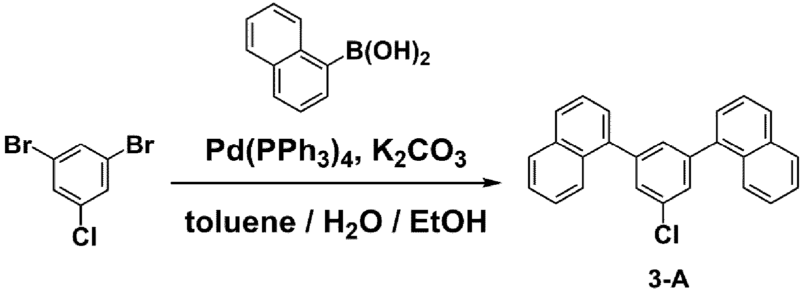

### Synthesis of Intermediate Compound 3-A

In an Ar atmosphere, 1,3-dibromo-5-chlorobenzene (30.0 g), naphthalen-1-ylboronic acid (38.2 g), tetrakis(triphenylphosphine)palladium(0) (Pd(PPh₃)₄, 6.4 g), and potassium carbonate (K₂CO₃, 61.3 g) were added to a 1 L 3-necked flask, and the mixture was dissolved in a mixed solvent of toluene, water, and ethanol (EtOH) (volume ratio: 10:2:1, 500 mL), and stirred and heated at 80°C for 16 hours. After cooling, water was added to the solution and an organic layer was obtained through extraction with dichloromethane (CH₂Cl₂). The obtained organic layer was dried over anhydrous magnesium sulfate (MgSO₄) all at once, and then the solvent was distilled off under reduced pressure. The obtained crude product was purified through recrystallization to obtain 30.8 g of Intermediate Compound 3-A (yield: 76%). Intermediate Compound 3-A had a molecular weight of 364, as measured by Fast atom bombardment-Mass spectroscopy (FAB-MS).

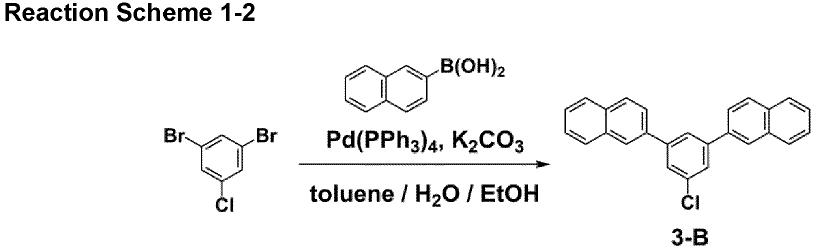

### Synthesis of Intermediate Compound 3-B

35.1 g of Intermediate Compound 3-B (yield: 87%) was obtained in substantially the same manner as in the synthesis of Intermediate Compound 3-A, except that naphthalen-2-ylboronic acid (38.2 g) was used instead of naphthalen-1-ylboronic acid (38.2 g). Intermediate Compound 3-B had a molecular weight of 364, as measured by FAB-MS.

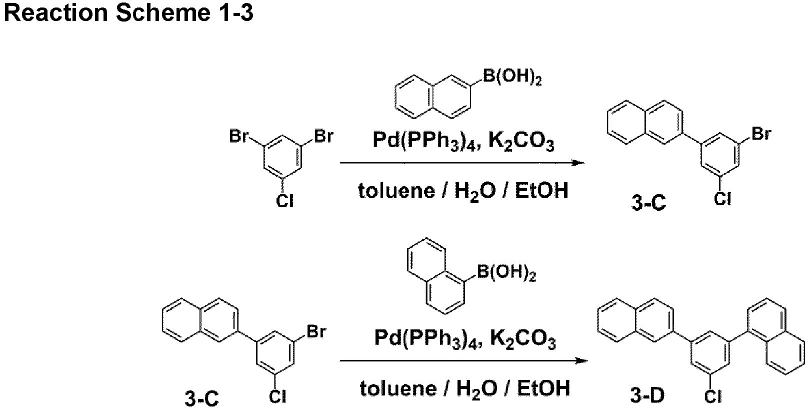

### Synthesis of Intermediate Compound 3-C

In an Ar atmosphere, 1,3-dibromo-5-chlorobenzene (30.0 g), naphthalen-2-ylboronic acid (19.2 g), Pd(PPh₃)₄ (6.4 g), and K₂CO₃ (31.0 g) were added to a 1 L 3-necked flask, and the mixture was dissolved in a mixed solvent of toluene, water, and ethanol (volume ratio: 10:2:1, 500 mL), and stirred and heated at 80°C for 16 hours. After cooling, water was added to the solution and an organic layer was obtained through extraction with CH₂Cl₂. The obtained organic layer was dried over anhydrous MgSO₄ all at once, and then the solvent was distilled off under reduced pressure. The obtained crude product was purified through silica gel column chromatography and recrystallization to obtain 18.5 g of Intermediate Compound 3-C (yield: 52%). Intermediate Compound 3-C had a molecular weight of 317, as measured by FAB-MS.

### Synthesis of Intermediate Compound 3-D

14.8 g of Intermediate Compound 3-D (yield: 86%) was obtained in substantially the same manner as in the synthesis of Intermediate Compound 3-C, except that Intermediate Compound 3-C (15.0 g) was used instead of 1,3-dibromo-5-chlorobenzene (30.0 g) and naphthalen-1-ylboronic acid (8.1 g) was used instead of naphthalen-2-ylboronic acid (19.2 g). Intermediate Compound 3-D had a molecular weight of 364, as measured by FAB-MS.

### (2) Synthesis of monoamine Compound 3

Monoamine Compound 3 according to one or more embodiments may be synthesized by, for example, a process of Reaction Scheme 2.

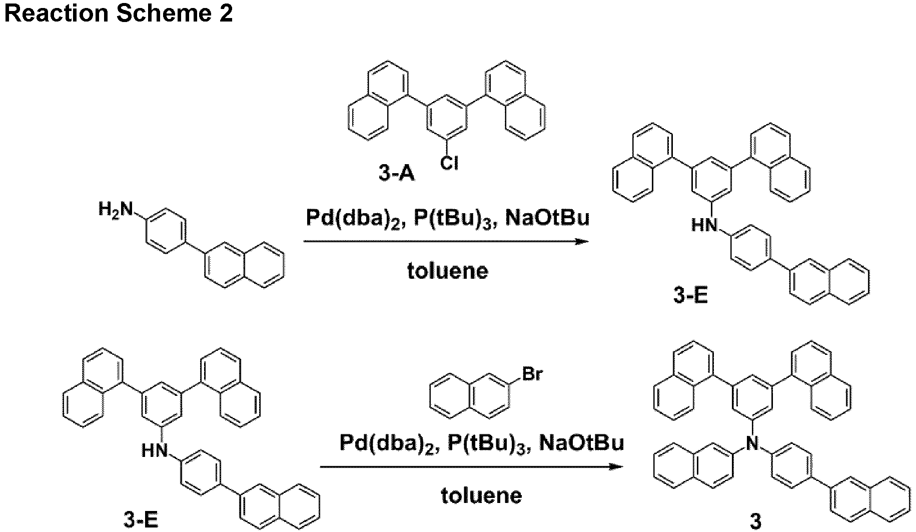

### Synthesis of Intermediate Compound 3-E

In an Ar atmosphere, 4-(naphthalen-2-yl)aniline (10.0 g), Intermediate Compound 3-A (16.6 g), bis(dibenzylideneacetone)palladium(0) (Pd(dba)₂, 0.3 g), and sodium tert-butoxide (NaOtBu, 6.6 g) were added to a 500 mL 3-necked flask, and the mixture was dissolved in toluene (200 mL), and tri-tert-butylphosphine (P(*t*Bu)₃, 2.0 *M* in toluene, 0.5 mL) was added and the mixture was stirred at room temperature for 8 hours. Water was then added to the solution and an organic layer was obtained through extraction with CH₂Cl₂. The obtained organic layer was dried over anhydrous MgSO₄ all at once, and then the solvent was distilled off under reduced pressure. The obtained crude product was purified through silica gel column chromatography and recrystallization to obtain 14.2 g of Intermediate Compound 3-E (yield: 57%). Intermediate Compound 3-E had a molecular weight of 547, as measured by FAB-MS.

### Synthesis of Compound 3

In an Ar atmosphere, Intermediate Compound 3-E (5.0 g), 2-bromonaphthalene (1.9 g), Pd(dba)₂ (0.05 g), and NaOtBu (1.3 g) were added to a 300 mL 3-necked flask, and the mixture was dissolved in toluene (100 mL), and P(*t*Bu)₃ *(2.0 M in* toluene, 0.1 mL) was added, and the mixture was stirred and heated at 100°C for 4 hours. After cooling, water was added to the solution and an organic layer was obtained through extraction with CH₂Cl₂. The obtained organic layer was dried over anhydrous MgSO₄ all at once, and then the solvent was distilled off under reduced pressure. The obtained crude product was purified through silica gel column chromatography to obtain 5.2 g of Compound 3 (yield: 85%). Compound 3 had a molecular weight of 673, as measured by FAB MS.

### (3) Synthesis of monoamine Compound 8

Monoamine Compound 8 according to one or more embodiments may be synthesized by, for example, a process of Reaction Scheme 3.

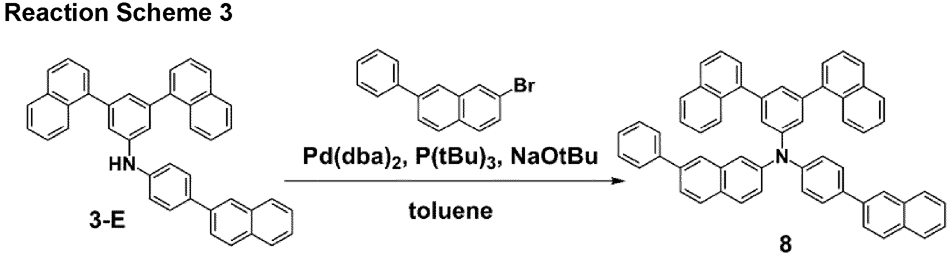

5.5 g of Compound 8 (yield: 81%) was obtained in substantially the same manner as in the synthesis of Compound 3, except that 2-bromo-7-phenylnaphthalene (2.6 g) was used instead of 2-bromonaphthalene (1.9 g). Compound 8 had a molecular weight of 749, as measured by FAB MS.

### (4) Synthesis of monoamine Compound 18

Monoamine Compound 18 according to one or more embodiments may be synthesized by, for example, a process of Reaction Scheme 4.

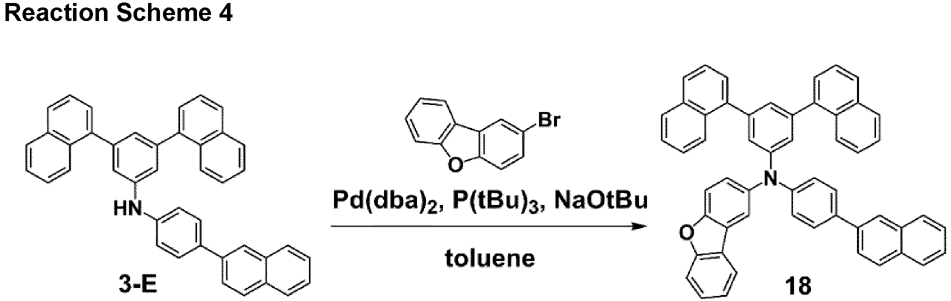

5.4 g of Compound 18 (yield: 84%) was obtained in substantially the same manner as in the synthesis of Compound 3, except that 2-bromodibenzofuran (2.3 g) was used instead of 2-bromonaphthalene (1.9 g). Compound 18 had a molecular weight of 713, as measured by FAB MS.

### (5) Synthesis of monoamine Compound 39

Monoamine Compound 39 according to one or more embodiments may be synthesized by, for example, a process of Reaction Scheme 5.

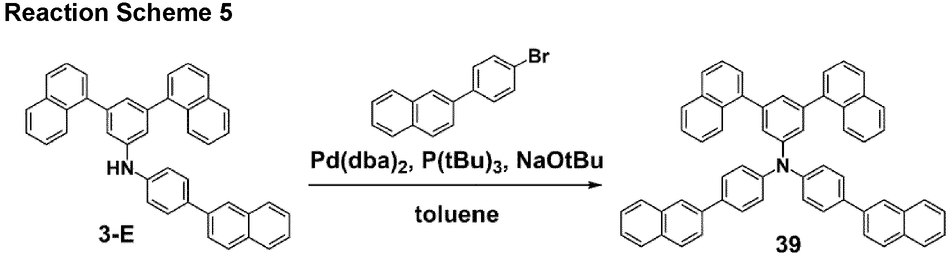

5.4 g of Compound 39 (yield: 80%) was obtained in substantially the same manner as in the synthesis of Compound 3, except that 2-(4-bromophenyl)naphthalene (2.6 g) was used instead of 2-bromonaphthalene (1.9 g). Compound 39 had a molecular weight of 749, as measured by FAB MS.

### (6) Synthesis of monoamine Compound 71

Monoamine Compound 71 according to one or more embodiments may be synthesized by, for example, a process of Reaction Scheme 6.

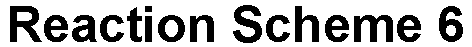

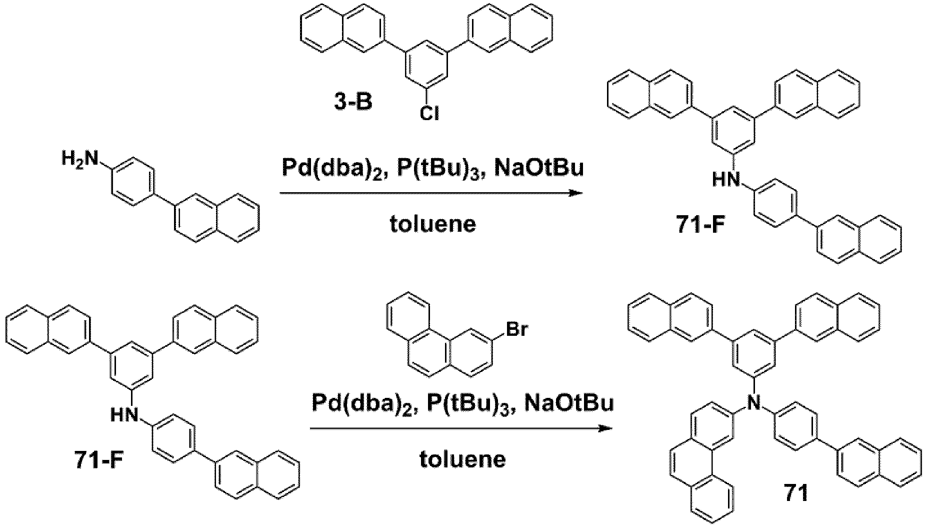

### Synthesis of Intermediate Compound 71-F

12.2 g of Intermediate Compound 71-F (yield: 49%) was obtained in substantially the same manner as in the synthesis of Intermediate Compound 3-E, except that Intermediate Compound 3-B (16.6 g) was used instead of Intermediate Compound 3-A (16.6 g). Intermediate Compound 71-F had a molecular weight of 547, as measured by FAB-MS.

### Synthesis of Compound 71

5.2 g of Compound 71 (yield: 80%) was obtained in substantially the same manner as in the synthesis of Compound 3, except that Intermediate Compound 71-F (5.0 g) was used instead of Intermediate Compound 3-E (5.0 g) and 3-bromophenanthrene (2.4 g) was used instead of 2-bromonaphthalene (1.9 g). Compound 71 had a molecular weight of 723, as measured by FAB MS.

### (7) Synthesis of monoamine Compound 94

Monoamine Compound 94 according to one or more embodiments may be synthesized by, for example, a process of Reaction Scheme 7.

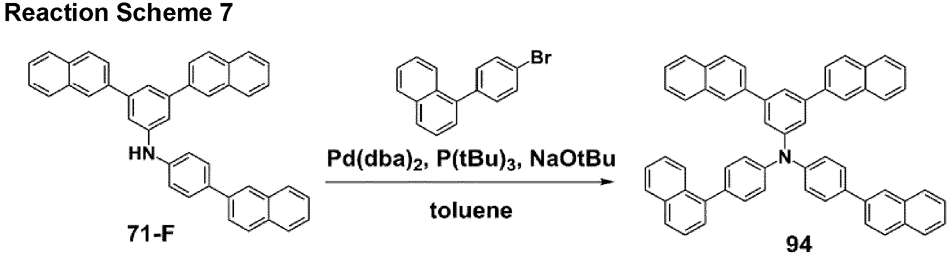

5.1 g of Compound 94 (yield: 75%) was obtained in substantially the same manner as in the synthesis of Compound 3, except that Intermediate Compound 71-F (5.0 g) was used instead of Intermediate Compound 3-E (5.0 g) and 1-(4-bromophenyl)naphthalene (2.6 g) was used instead of 2-bromonaphthalene (1.9 g). Compound 94 had a molecular weight of 749, as measured by FAB MS.

### (8) Synthesis of monoamine Compound 37

Monoamine Compound 37 according to one or more embodiments may be synthesized by, for example, a process of Reaction Scheme 8.

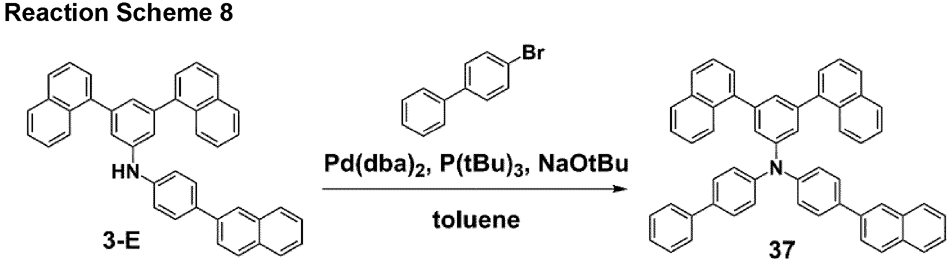

5.2 g of Compound 37 (yield: 81%) was obtained in substantially the same manner as in the synthesis of Compound 3, except that 4-bromobiphenyl (2.2 g) was used instead of 2-bromonaphthalene (1.9 g). Compound 37 had a molecular weight of 699, as measured by FAB MS.

### (9) Synthesis of monoamine Compound 44

Monoamine Compound 44 according to one or more embodiments may be synthesized by, for example, a process of Reaction Scheme 9.

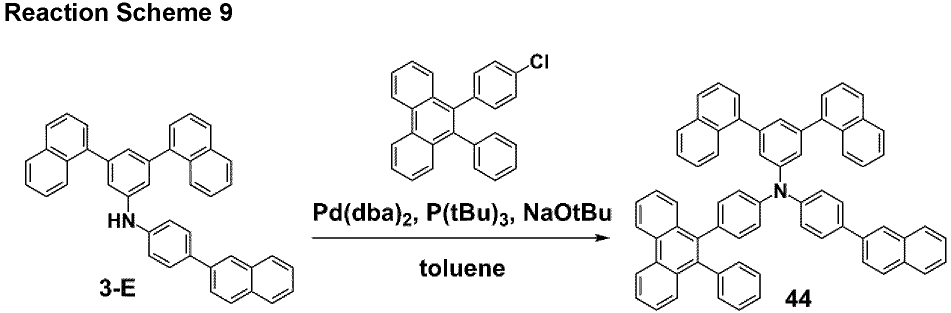

5.4 g of Compound 44 (yield: 68%) was obtained in substantially the same manner as in the synthesis of Compound 3, except that 9-(4-chlorophenyl)-10-phenylphenanthrene (3.4 g) was used instead of 2-bromonaphthalene (1.9 g). Compound 44 had a molecular weight of 876, as measured by FAB MS.

### (10) Synthesis of monoamine Compound 104

Monoamine Compound 104 according to one or more embodiments may be synthesized by, for example, a process of Reaction Scheme 10.

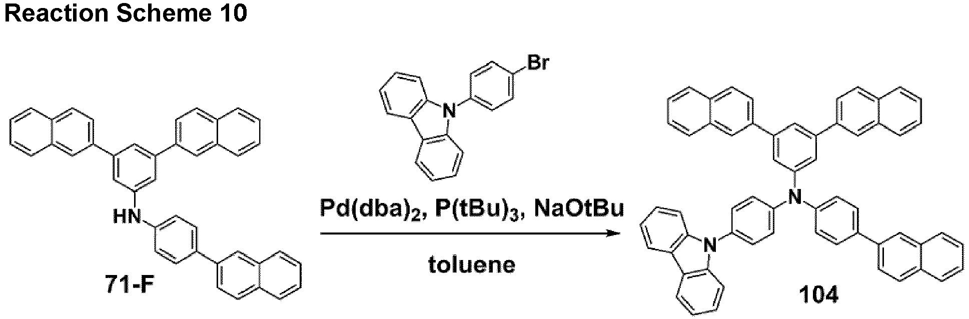

4.7 g of Compound 104 (yield: 65%) was obtained in substantially the same manner as in the synthesis of Compound 3, except that Intermediate Compound 71-F (5.0 g) was used instead of Intermediate Compound 3-E (5.0 g) and 9-(4-bromophenyl)-9H-carbazole (2.9 g) was used instead of 2-bromonaphthalene (1.9 g). Compound 104 had a molecular weight of 788, as measured by FAB MS.

### (11) Synthesis of monoamine Compound 81

Monoamine Compound 81 according to one or more embodiments may be synthesized by, for example, a process of Reaction Scheme 11.

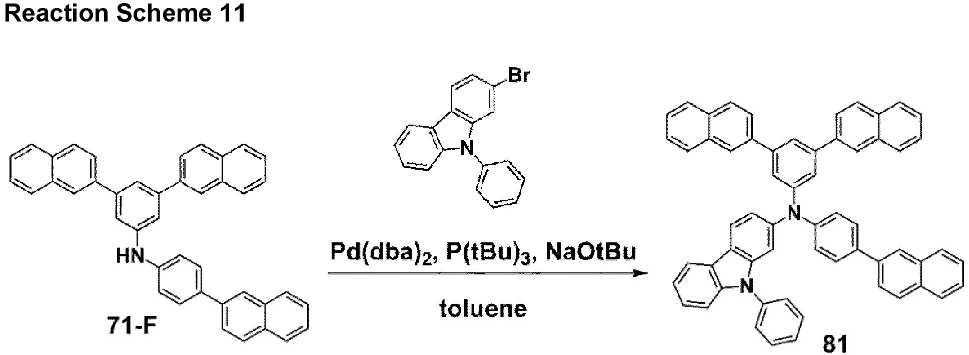

5.0 g of Compound 81 (yield: 70%) was obtained in substantially the same manner as in the synthesis of Compound 3, except that Intermediate Compound 71-F (5.0 g) was used instead of Intermediate Compound 3-E (5.0 g) and 2-bromo-9-phenyl-9H-carbazole (3.0 g) was used instead of 2-bromonaphthalene (1.9 g). Compound 81 had a molecular weight of 788, as measured by FAB MS.

### (12) Synthesis of monoamine Compound 29

Monoamine Compound 29 according to one or more embodiments may be synthesized by, for example, a process of Reaction Scheme 12.

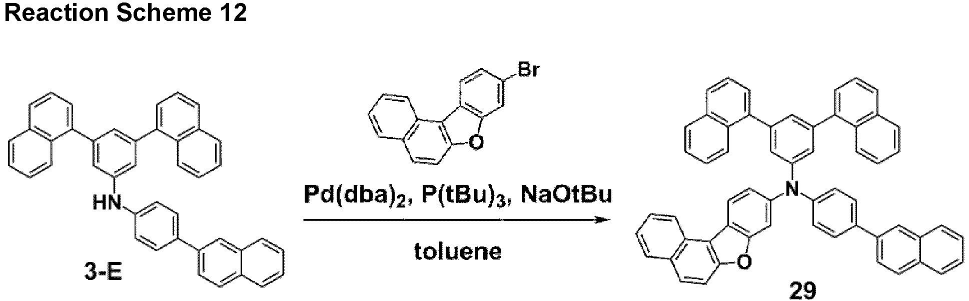

5.4 g of Compound 29 (yield: 77%) was obtained in substantially the same manner as in the synthesis of Compound 3, except that 9-bromonaphthobenzofuran (2.8 g) was used instead of 2-bromonaphthalene (1.9 g). Compound 29 had a molecular weight of 763, as measured by FAB MS.

### (13) Synthesis of monoamine Compound 36

Monoamine Compound 36 according to one or more embodiments may be synthesized by, for example, a process of Reaction Scheme 13.

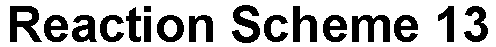

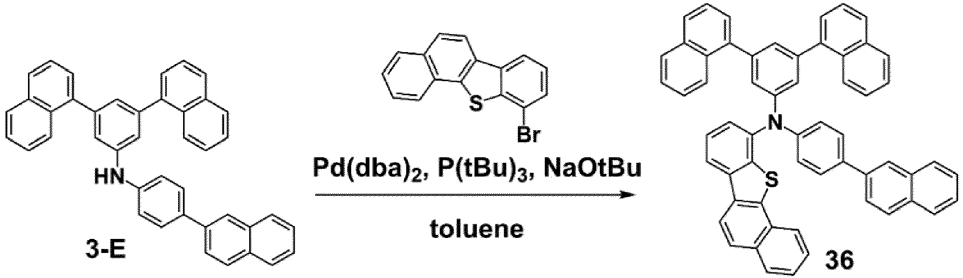

4.0 g of Compound 36 (yield: 50%) was obtained in substantially the same manner as in the synthesis of Compound 3, except that 10-bromobenzonaphthothiophene (2.9 g) was used instead of 2-bromonaphthalene (1.9 g). Compound 36 had a molecular weight of 780, as measured by FAB MS.

### 2. Preparation and Evaluation of Light Emitting Element

### (1) Preparation of light emitting element

Light emitting elements including a respective monoamine compound according to an embodiment or a respective Comparative Example compound in a hole transport layer were prepared through a method. Light emitting elements of Examples 1 to 12 were each prepared using respective monoamine Compounds of an embodiment, 3, 8, 18, 39, 71, 94, 37, 44, 104, 81, 29, and 36 as a material of the hole transport layer. Light emitting elements of Comparative Examples 1 to 14 were each prepared using respectively Comparative Example Compounds X-1 to X-14 as a material of the hole transport layer.

A glass substrate on which an ITO having a thickness of 150 nm was patterned as a first electrode was subjected to ultrasonic cleaning with isopropyl alcohol and pure water each for 5 minutes. The glass substrate was irradiated with UV for 30 minutes, and ozone-treated. Thereafter, a hole injection layer was formed through the deposition of 2-TNATA with a thickness of 60 nm. On the hole injection layer, a hole transport layer was formed through the deposition of respective Example compounds or respective Comparative Example compounds with a thickness of 30 nm.

On the hole transport layer, an emission layer was formed through the co-deposition of TBP and ADN with a thickness of 25 nm. TBP and ADN were subjected to the co-deposition at a weight ratio of 3:97. Thereafter, an electron transport region was formed through the sequential deposition of Alq₃ with a thickness of 25 nm and LiF with a thickness of 1 nm. Then, a second electrode was formed through the deposition of Al with a thickness of 100 nm. The hole injection layer, the hole transport layer, the emission layer, the electron transport region, and the second electrode were each formed using a vacuum deposition device.

### Materials used for preparation of light emitting elements

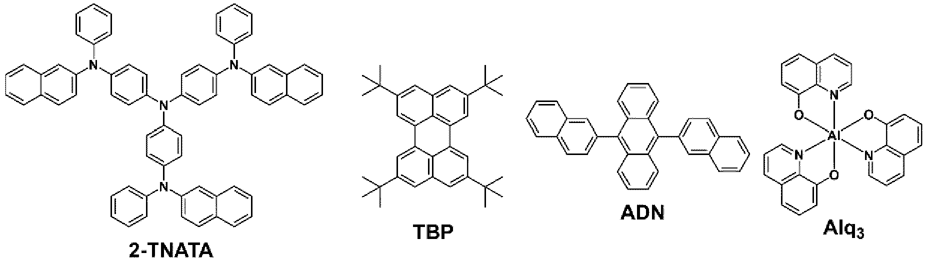

### Example Compound

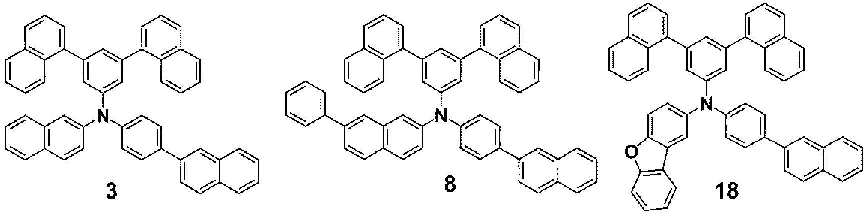

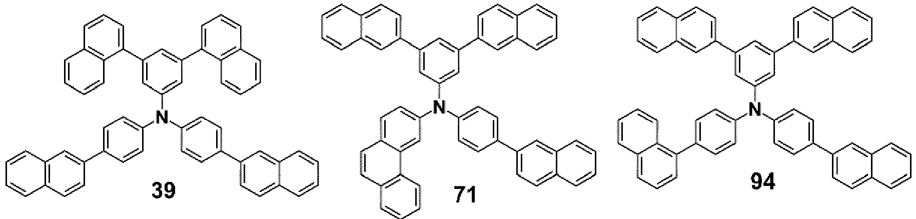

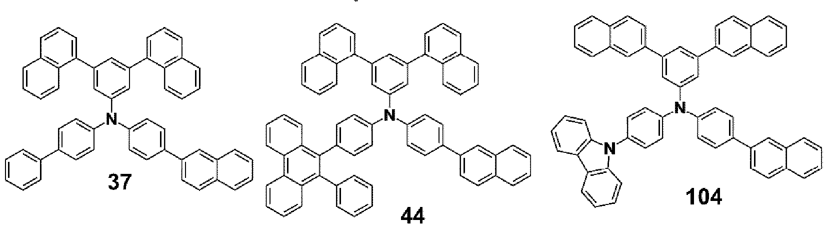

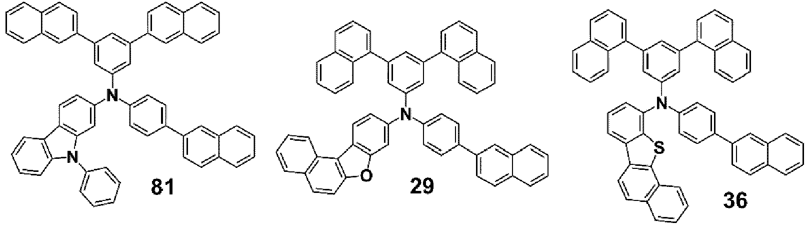

### Comparative Example Compound

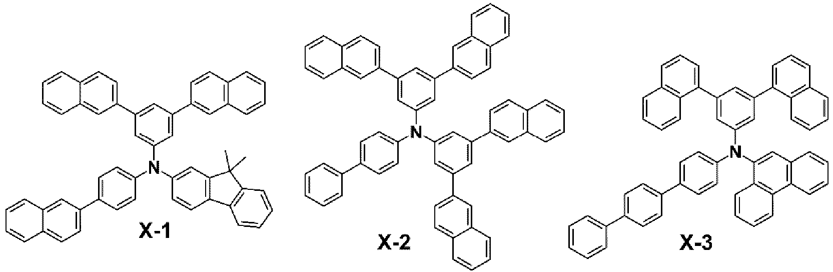

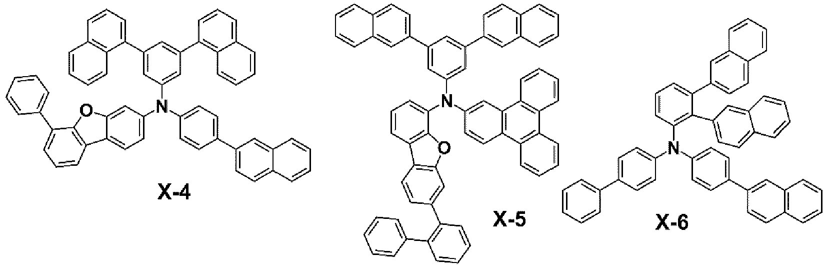

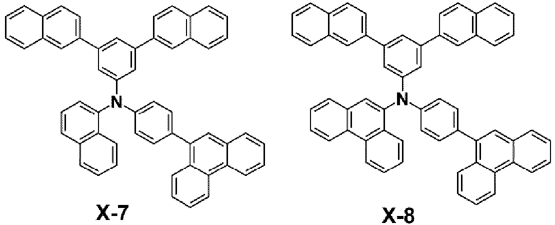

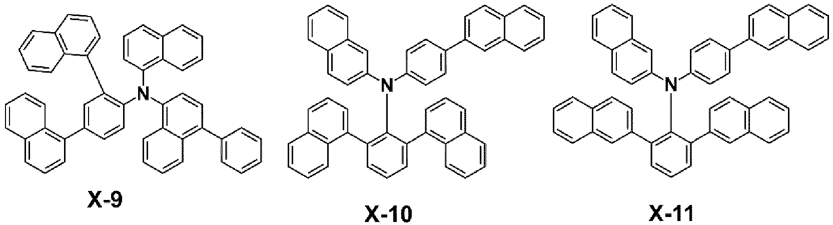

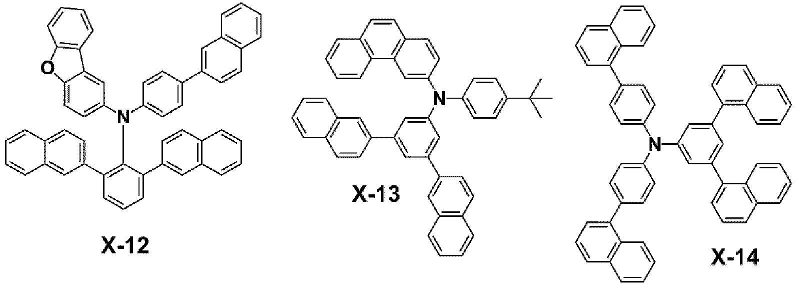

### (2) Evaluation of light emitting element

Table 1 shows evaluation results of each of the light emitting elements of Examples and Comparative Examples. The luminous efficiency and lifespan at a current density of 10 mA/cm² were evaluated using a C9920-11 luminance orientation characteristic measuring device from Hamamatsu Photonics. Lifespan (LT50) indicates the time taken to decrease from an initial luminance of 100% to a luminance of 50%. In Table 1, the luminous efficiency and lifespan are relative values, with the value of Comparative Example 1 set as 100%.

**Table 1**

| Example of element preparation | Hole transport layer | Luminous efficiency (@ 10 mA/cm²) | Lifespan (LT50) |
|---|---|---|---|
| Example 1 | Compound 3 | 105% | 140% |
| Example 2 | Compound 8 | 105% | 150% |
| Example 3 | Compound 18 | 105% | 130% |
| Example 4 | Compound 39 | 103% | 150% |
| Example 5 | Compound 71 | 102% | 160% |
| Example 6 | Compound 94 | 104% | 140% |
| Example 7 | Compound 37 | 102% | 140% |
| Example 8 | Compound 44 | 103% | 140% |
| Example 9 | Compound 104 | 102% | 110% |
| Example 10 | Compound 81 | 101% | 120% |
| Example 11 | Compound 29 | 101% | 150% |
| Example 12 | Compound 36 | 104% | 150% |
| Comparative Example 1 | Comparative Example Compound X-1 | 100% | 100% |
| Comparative Example 2 | Comparative Example Compound X-2 | 99% | 65% |
| Comparative Example 3 | Comparative Example Compound X-3 | 100% | 90% |
| Comparative Example 4 | Comparative Example Compound X-4 | 100% | 90% |
| Comparative Example 5 | Comparative Example Compound X-5 | 99% | 85% |
| Comparative Example 6 | Comparative Example Compound X-6 | 98% | 50% |
| Comparative Example 7 | Comparative Example Compound X-7 | 99% | 50% |
| Comparative Example 8 | Comparative Example Compound X-8 | 100% | 50% |
| Comparative Example 9 | Comparative Example Compound X-9 | 101% | 40% |
| Comparative Example 10 | Comparative Example Compound X-10 | 100% | 40% |
| Comparative Example 11 | Comparative Example Compound X-11 | 99% | 30% |
| Comparative Example 12 | Comparative Example Compound X-12 | 100% | 30% |
| Comparative Example 13 | Comparative Example Compound X-13 | 98% | 90% |
| Comparative Example 14 | Comparative Example Compound X-14 | 100% | 90% |

Referring to Table 1, it is seen that, compared to the light emitting elements of Comparative Examples 1 to 14, the light emitting elements of Examples 1 to 12 each exhibited high luminous efficiency and long lifespan. It is seen that in particular, the light emitting elements of Examples 1 to 8, 11, and 12 each exhibited significantly increased lifespan. The light emitting elements of Examples 1 to 12 respectively include Compounds 3, 8, 18, 39, 71, 94, 37, 44, 104, 81, 29, and 36, and Compounds 3, 8, 18, 39, 71, 94, 37, 44, 104, 81, 29, and 36 are monoamine compounds of an embodiment of the present disclosure. Compounds 3, 8, 18, 39, 71, 94, 37, 44, 104, 81, 29, and 36 each include first to third substituents directly or indirectly bonded to an amine group. The first substituent is a 3,5-dinaphthalenylphenyl group, and the second substituent is a naphthalenyl group. Accordingly, it is seen that the monoamine compounds of an embodiment improve the efficiency and lifespan of a light emitting element. It is seen that the light emitting element including the monoamine compound of an embodiment may exhibit high luminous efficiency and long-life characteristics.

The light emitting element of Comparative Example 1 includes Comparative Example Compound X-1. Comparative Example Compound X-1 differs from the monoamine compound of an embodiment in that Ar^{a} of Formula 1 described above is a dimethylfluorenyl group. The monoamine compound of an embodiment represented by Formula 1 does not include a dimethylfluorenyl group in the definition of Ar^{a}. Comparative Example Compound X-1 including a dimethyl group is thermally unstable and increases deposition temperature. Accordingly, the light emitting element of Comparative Example 1 exhibited low luminous efficiency and short lifespan.

The light emitting element of Comparative Example 2 includes Comparative Example Compound X-2. Comparative Example Compound X-2 differs from the monoamine compound of an embodiment in that L³ of Formula 1 described above is a phenyl group substituted with a naphthalenyl group. The monoamine compound of an embodiment represented by Formula 1 does not include a phenyl group substituted with a naphthalenyl group in the definition of L³. Comparative Example Compound X-2 introduces two dinaphthalenylphenyl groups, which increases deposition temperature and causes thermal decomposition during deposition, thereby reducing the lifespan of the light emitting element. Accordingly, the light emitting element of Comparative Example 2 exhibited low luminous efficiency and short lifespan.

The light emitting element of Comparative Example 3 includes Comparative Example Compound X-3. Comparative Example Compound X-3 does not include a naphthalenyl group at the second substituent position (i.e., the second substituent described above does not include a naphthalenyl group) and instead includes a terphenyl group. Comparative Example Compound X-3 including a terphenyl group increases deposition temperature and causes thermal decomposition during deposition, thereby reducing the lifespan of the light emitting element. Accordingly, the light emitting element of Comparative Example 3 exhibited low luminous efficiency and short lifespan.

The light emitting element of Comparative Example 4 includes Comparative Example Compound X-4. Comparative Example Compound X-4 differs from the monoamine compound of an embodiment in that Ar^{a} of Formula 1 described above is a dibenzofuranyl group substituted with a phenyl group. The monoamine compound of an embodiment represented by Formula 1 does not include a dibenzofuranyl group substituted with a phenyl group in the definition of Ar^{a}, but includes an unsubstituted dibenzofuranyl group. Comparative Example Compound X-4, into which a dibenzofuranyl group substituted with a phenyl group is introduced, has reduced charge transport properties, thereby reducing the efficiency of the light emitting element. Accordingly, the light emitting element of Comparative Example 4 exhibited low luminous efficiency and short lifespan. In addition, a hole transport material including a dibenzothiophenyl group substituted with a phenyl group tends to exhibit reduced charge transport properties compared to a hole transport material including an unsubstituted dibenzothiophenyl group.

The light emitting element of Comparative Example 5 includes Comparative Example Compound X-5. Comparative Example Compound X-5 includes the first substituent (i.e., dinaphthalenylphenyl group) described above, but does not include the second and third substituents. Comparative Example Compound X-5 includes a triphenylenyl group and a dibenzofuranyl group substituted with a phenyl group, which increases deposition temperature and causes thermal decomposition during deposition, thereby reducing the lifespan of the light emitting element. Accordingly, the light emitting element of Comparative Example 5 exhibited low luminous efficiency and short lifespan.

The light emitting element of Comparative Example 6 includes Comparative Example Compound X-6. Comparative Example Compound X-6 includes a dinaphthalenylphenyl group, but differs from the monoamine compound of an embodiment in the bonding positions of two naphthalenyl groups. Comparative Example Compound X-6 includes a 2,3-dinaphthalenylphenyl group, and the monoamine compound of an embodiment includes a 3,5-dinaphthalenylphenyl group. Comparative Example Compound X-6, which includes a 2,3-dinaphthalenylphenyl group, a substituent with a large steric hindrance, causes thermal decomposition during deposition and reduces the lifespan of the light emitting element. Accordingly, the light emitting element of Comparative Example 6 exhibited low luminous efficiency and short lifespan.

The light emitting elements of Comparative Examples 7, 9, and 14 include Comparative Example Compounds X-7, X-9, and X-14, respectively. Comparative Example Compounds X-7, X-9, and X-14 differ from the monoamine compound of an embodiment in the bonding position of the second substituent, the naphthalenyl group. Comparative Example Compounds X-7, X-9, and X-14 include a 1-naphthalenyl group, and the monoamine compound of an embodiment includes a 2-naphthalenyl group. Comparative Example Compounds X-7, X-9, and X-14, into which a 1-naphthalenyl group is introduced, exhibit reduced material stability and reduces the lifespan of the light emitting element. Accordingly, the light emitting elements of Comparative Examples 7, 9, and 14 each exhibited low luminous efficiency and short lifespan.

The light emitting element of Comparative Example 8 includes Comparative Example Compound X-8. Comparative Example Compound X-8 does not include a naphthalenyl group as the second substituent, but includes a phenanthrenyl group. Comparative Example Compound X-8 including a phenanthrenyl group as the second substituent increases deposition temperature, causes thermal decomposition during deposition, and reduces the lifespan of the light emitting element. Accordingly, the light emitting element of Comparative Example 8 exhibited low luminous efficiency and short lifespan.

The light emitting elements of Comparative Examples 10 to 12 each include Comparative Example Compounds X-10 to X-12. Comparative Example Compounds X-10 to X-12 differ from the monoamine compound of an embodiment in that a 2,6-dinaphthalenylphenyl group is included. The monoamine compound of an embodiment includes a 3,5-dinaphthalenylphenyl group. Accordingly, the light emitting element of Comparative Examples 10 to 12 exhibited low luminous efficiency and short lifespan.

The light emitting element of Comparative Example 13 includes Comparative Example Compound X-13. Comparative Example Compound X-13 does not include a 2-naphthalenyl group as the second substituent, and includes a t-butyl group. The t-butyl group is a thermally unstable substituent, and Comparative Example Compound X-13 including the t-butyl group has reduced material stability. Accordingly, the light emitting element of Comparative Example 13 exhibited low luminous efficiency and short lifespan.

In one or more embodiments, an electronic apparatus includes a light emitting element. In the light emitting element of one or more embodiments, a hole transport region includes a monoamine compound of one or more embodiments. The monoamine compound of one or more embodiments includes first to third substituents directly or indirectly bonded to the amine group of the monoamine compound. The first substituent is a 3,5-dinaphthalenylphenyl group, and the second substituent is a 2-naphthalenyl group. Accordingly, the monoamine compound of one or more embodiments may exhibit excellent or suitable hole transport properties and excellent or suitable material stability, and the light emitting element of one or more embodiments including the monoamine compound may exhibit high luminous efficiency and long-life characteristics. The electronic apparatus of one or more embodiments may exhibit excellent or suitable reliability. For example, the structural configuration of the substituents on the monoamine compound may enhance molecular rigidity and reduce deposition temperature, which increases the thermal and chemical stability and improves charge carrier mobility. This contributes to a more balanced charge injection and transport and improved material stability within the light-emitting element, thereby improving overall device performance and operational stability.

A light emitting element of one or more embodiments and an electronic apparatus including the same include a monoamine compound of one or more embodiments, and may thus exhibit high efficiency and long-life. Such performance improvements are advantageous in high-resolution display applications, where uniform brightness and extended operational lifetimes are critical. The monoamine compound's ability to maintain performance under prolonged electrical stress conditions further supports its suitability for integration into next-generation OLED and other electroluminescent display technologies.

A monoamine compound of one or more embodiments may contribute to high efficiency and long life of a light emitting element. This contribution may be attributed to the compound's tailored electronic properties, including its HOMO energy level alignment with adjacent layers, which facilitates efficient hole injection and transport. Additionally, the thermal and morphological stability of the compound helps maintain the integrity of the device structure over time, reducing degradation and preserving emission characteristics.

As utilized herein, the terms "substantially," "about," or similar terms are used as terms of approximation and not as terms of degree, and are intended to account for the inherent deviations in measured or calculated values that would be recognized by those of ordinary skill in the art. "About" as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, or 5% of the stated value.

In the context of the present application and unless otherwise defined, the terms "use," "using," and "used" may be considered synonymous with the terms "utilize," "utilizing," and "utilized," respectively.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein.

The light-emitting element, the display device, the electronic apparatus, the manufacturing apparatuses thereof, or any other relevant apparatuses/devices or components according to embodiments of the present disclosure described herein may be implemented utilizing any suitable hardware, firmware (e.g., an application-specific integrated circuit), software, or a combination of software, firmware, and hardware. For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random-access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments of the present disclosure.

In the present disclosure, each suitable feature of the various embodiments of the disclosure may be combined or combined with each other, partially or entirely, and may be technically interlocked and operated in various suitable ways, and each embodiment may be implemented independently of each other or in conjunction with each other in any suitable manner unless otherwise stated or implied.

In the above, description has been made with reference to one or more embodiments of the disclosure, but those skilled or of ordinary skill in the art may understand that one or more suitable modifications and changes may be made to the disclosure insofar as such modifications and changes do not depart from the technical scope of the disclosure set forth in the appended claims.

Therefore, the technical scope of the disclosure is not to be limited to the contents stated in the detailed description of the disclosure, but should be determined by the appended claims and equivalents thereof.

## Claims

1. A monoamine compound represented by Formula 1: Formula 1 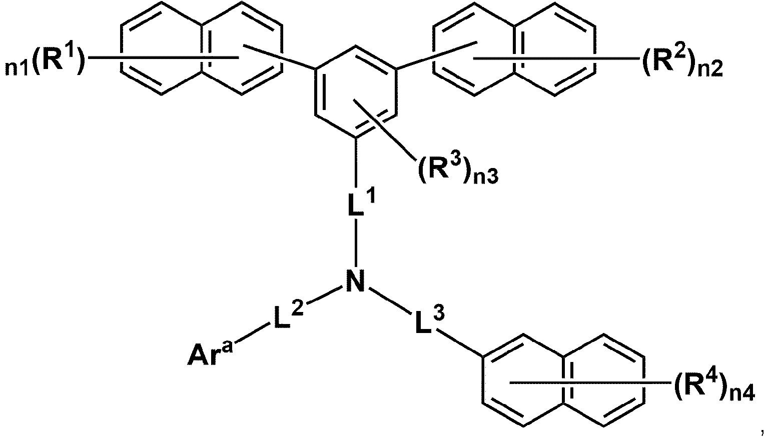 wherein in Formula 1,
L¹ is a direct linkage, a substituted or unsubstituted arylene group having 6 to 30 ring-forming carbon atoms, or a substituted or unsubstituted heteroarylene group having 5 to 30 ring-forming carbon atoms,
L² and L³ are each independently a direct linkage, an unsubstituted phenylene group, a phenylene group substituted with deuterium, or a phenylene group substituted with a phenyl group,
Ar^{a} is an unsubstituted phenyl group, an unsubstituted naphthalenyl group, a naphthalenyl group substituted with a phenyl group, an unsubstituted phenanthrenyl group, a phenanthrenyl group substituted with a phenyl group, an unsubstituted dibenzofuranyl group, an unsubstituted dibenzothiophenyl group, an unsubstituted carbazolyl group, a carbazolyl group substituted with a phenyl group, an unsubstituted benzonaphthofuranyl group, or an unsubstituted benzonaphthothiophenyl group,
R¹ to R³ are each independently hydrogen, deuterium, a halogen, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms,
R⁴ is hydrogen, deuterium, a halogen, a substituted or unsubstituted aryl group having 6 to 30 ring-forming carbon atoms, or a substituted or unsubstituted heteroaryl group having 5 to 30 ring-forming carbon atoms,
n1, n2, and n4 are each independently an integer of 0 to 7,
n3 is an integer of 0 to 3, and
any hydrogen in Formula 1 is optionally substituted with deuterium.

2. The monoamine compound of claim 1, wherein the monoamine compound represented by Formula 1 is represented by any one selected from among Formulas 1-A1 to 1-A3: 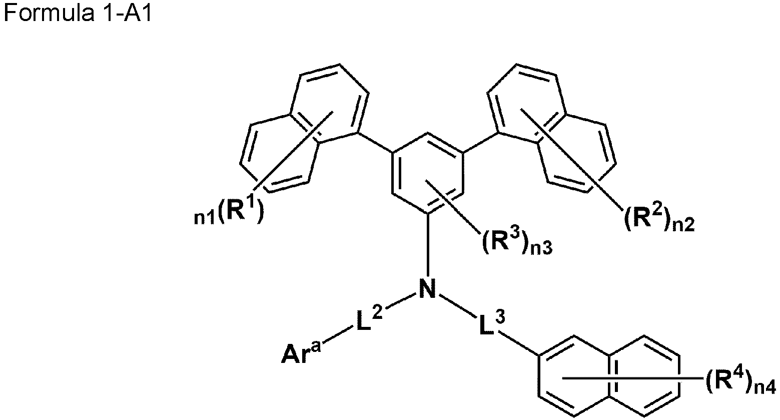 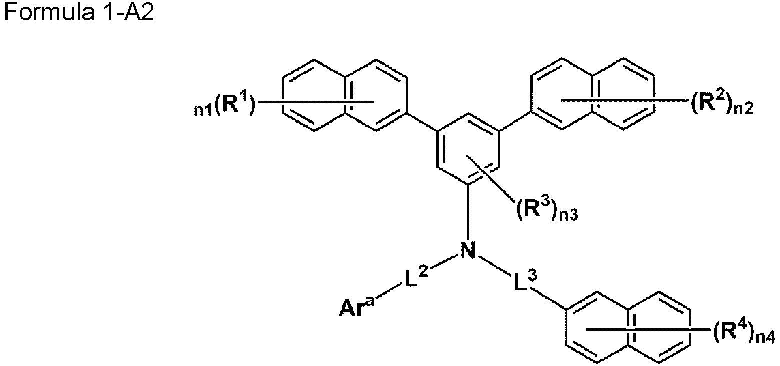 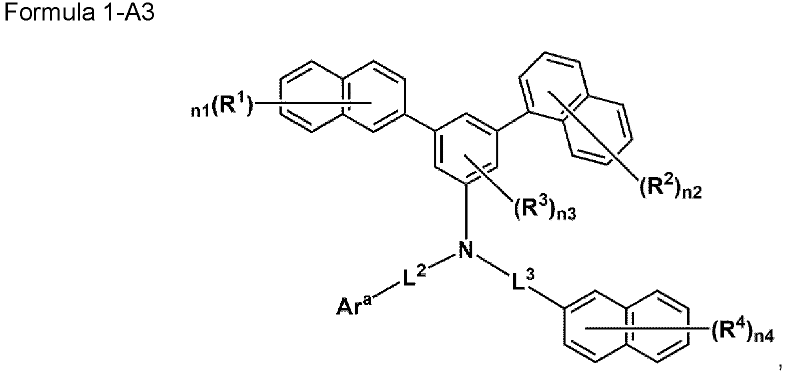 in Formulas 1-A1 to 1-A3,
L², L³, Ar^{a}, R¹ to R⁴, and n1 to n4 being the same as defined in Formula 1.

3. The monoamine compound of claim 1, wherein the monoamine compound represented by Formula 1 is represented by any one selected from among Formulas 1-B1 to 1-B3: 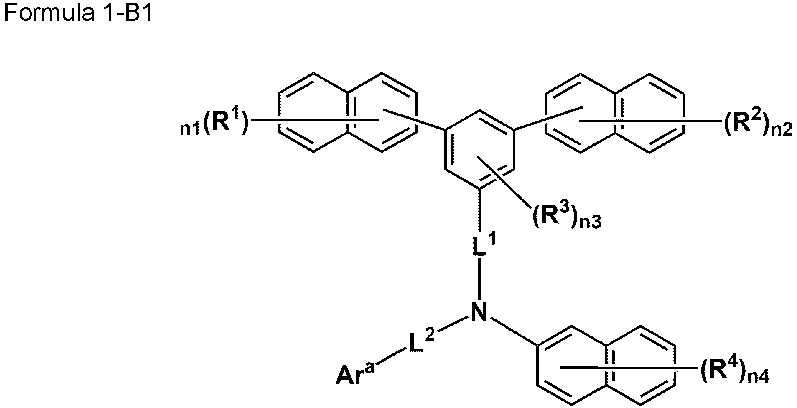 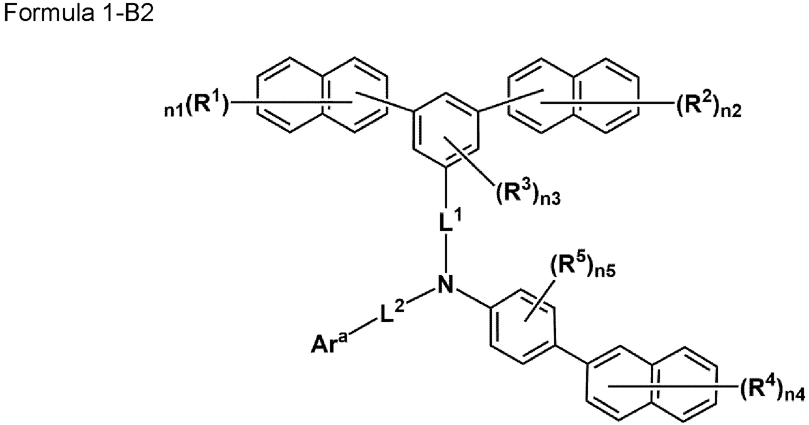 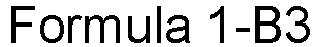 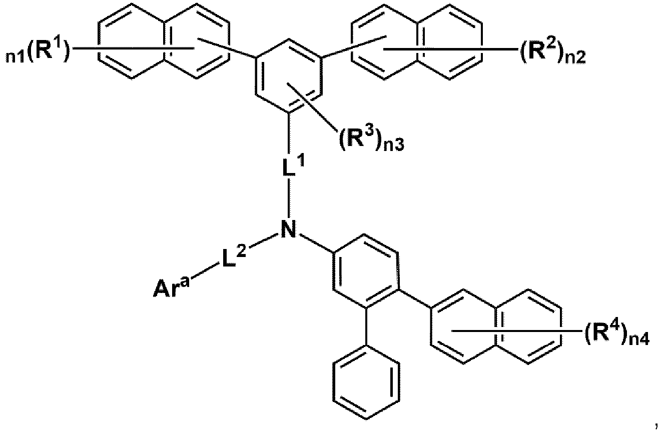 and
wherein in Formula 1-B2,
n5 is an integer of 0 to 4, and
R⁵ is hydrogen or deuterium, and
in Formulas 1-B1 to 1-B3,
L¹, L², Ar^{a}, R¹ to R⁴, and n1 to n4 are the same as defined in Formula 1.

4. The monoamine compound of any one of claims 1 to 3, wherein in Formula 1, L² is a direct linkage or is represented by any one selected from among L2-1 to L2-4:

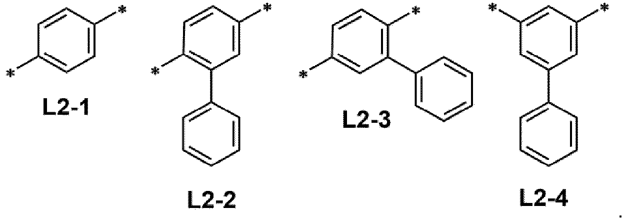

5. The monoamine compound of any one of claims 1 to 4, wherein in Formula 1, Ar^{a} is represented by any one selected from among AR-1 to AR-38:

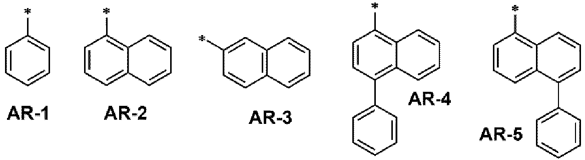

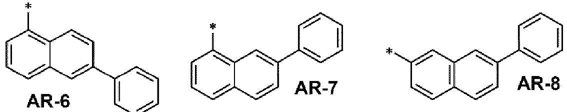

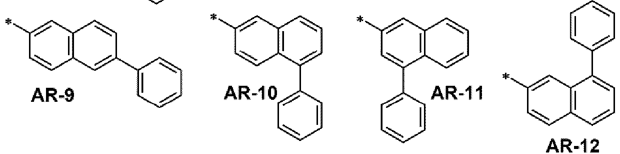

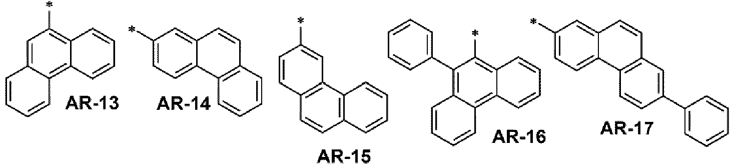

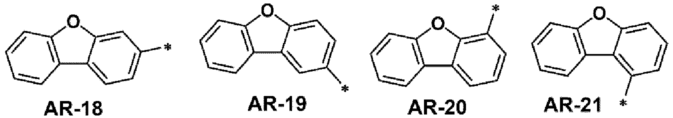

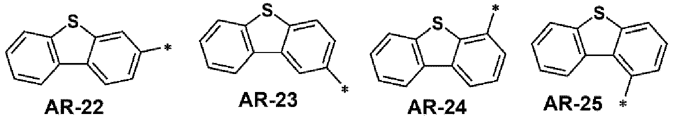

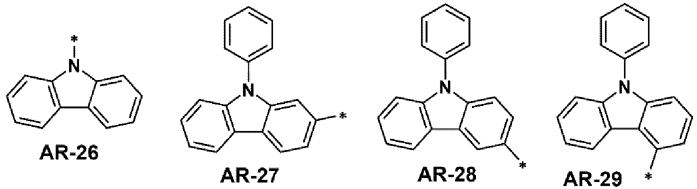

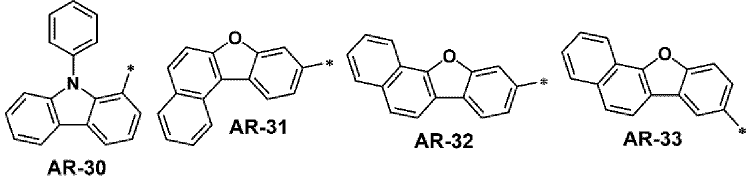

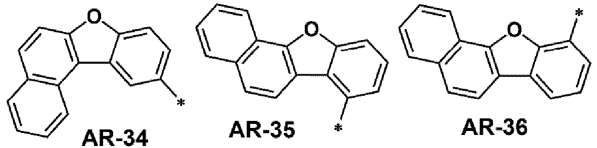

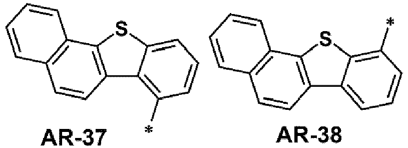

6. The monoamine compound of any one of claims 1 to 5, wherein:
in Formula 1, R¹ to R³ are each independently hydrogen, deuterium, fluorine, or an unsubstituted phenyl group; and
in Formula 1, R⁴ is hydrogen, deuterium, fluorine, an unsubstituted phenyl group, or an unsubstituted dibenzofuranyl group.

7. The monoamine compound of any one of claims 1 to 6, wherein in Formula 1, a naphthalenyl group containing R⁴ is represented by any one selected from among R4-1 to R4-6: 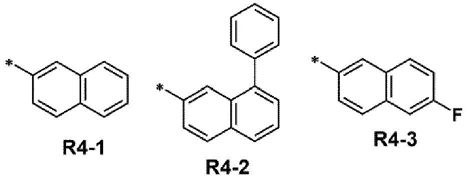 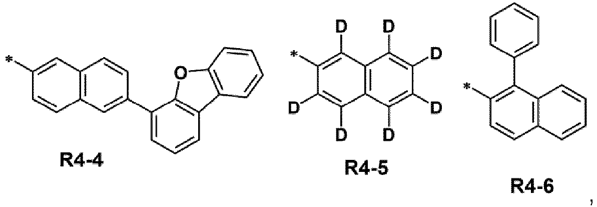 in R4-5, D being deuterium.

8. The monoamine compound of claim 1, wherein the monoamine compound represented by Formula 1 is any one selected from among compounds of Compound Group 1: 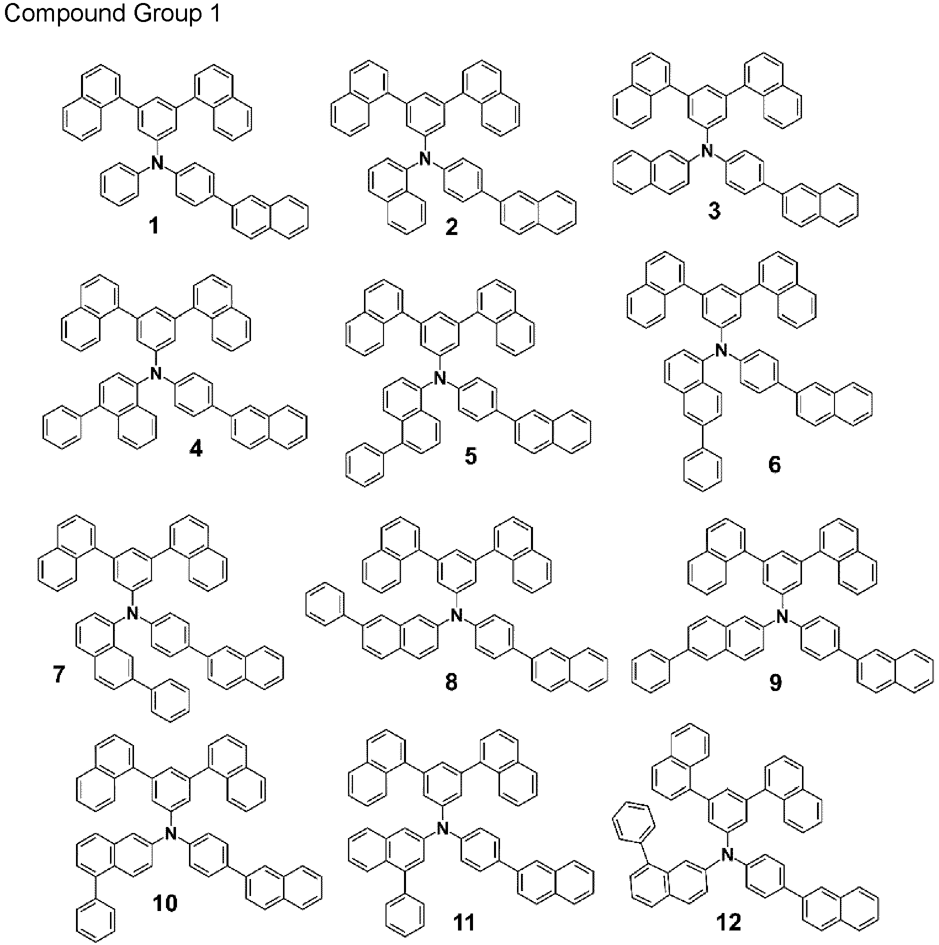 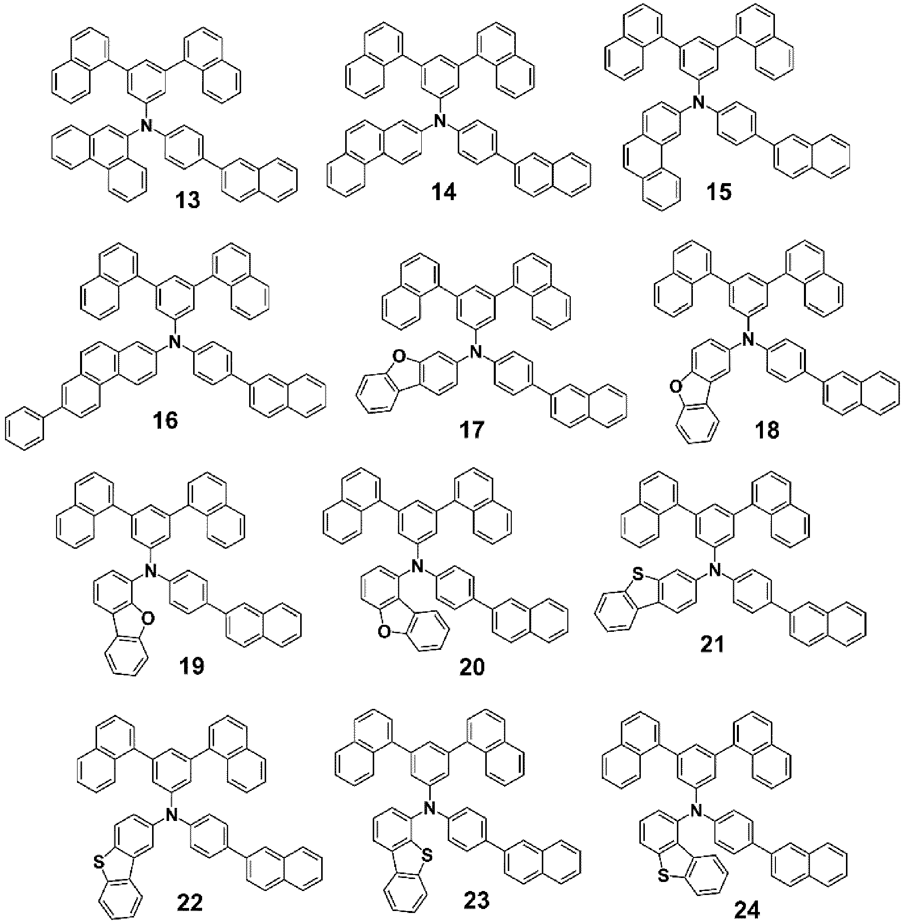 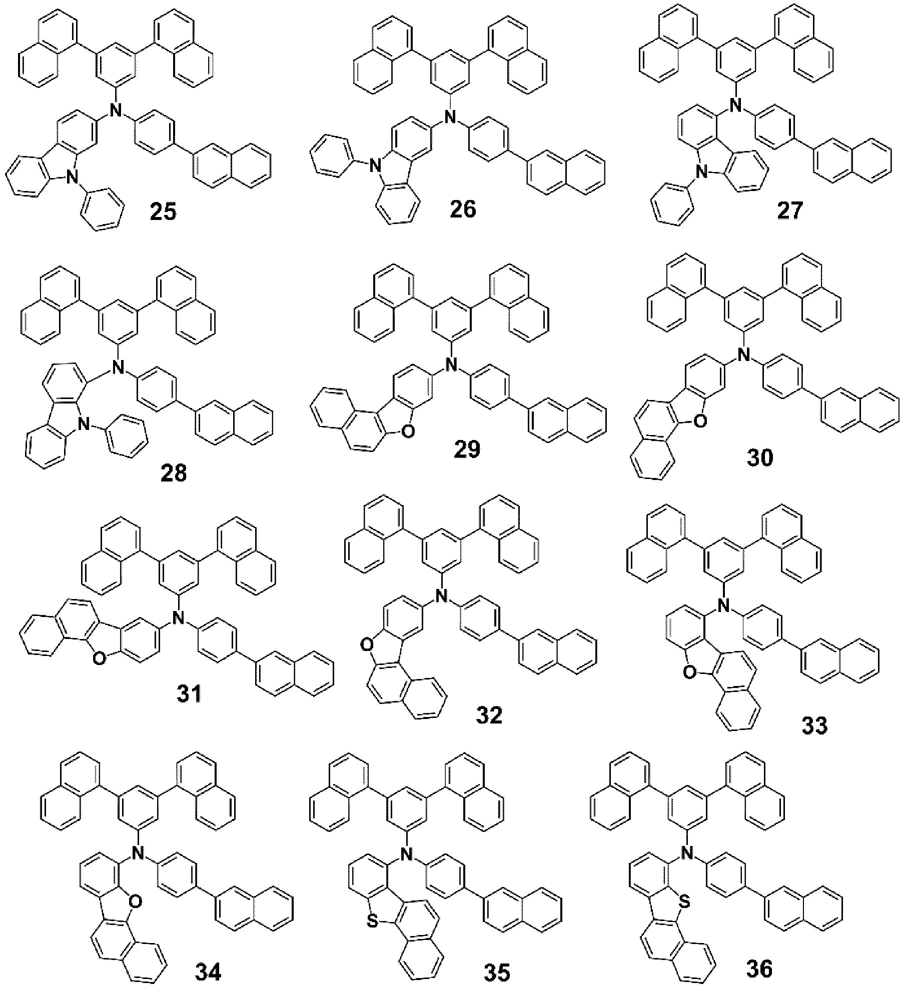 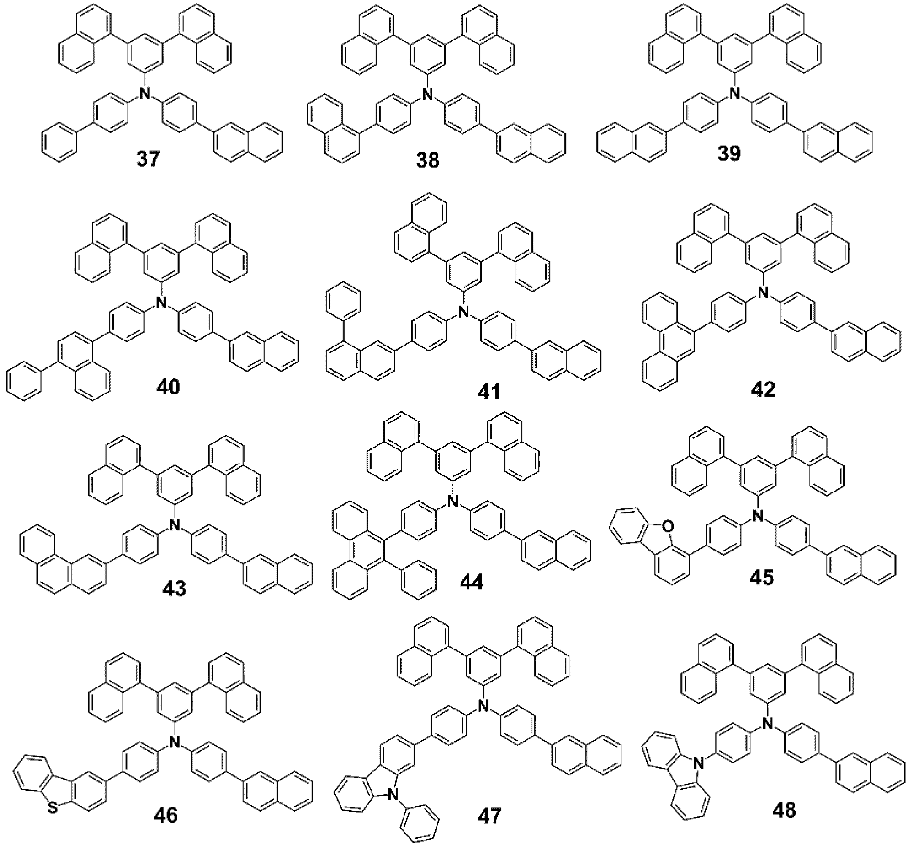 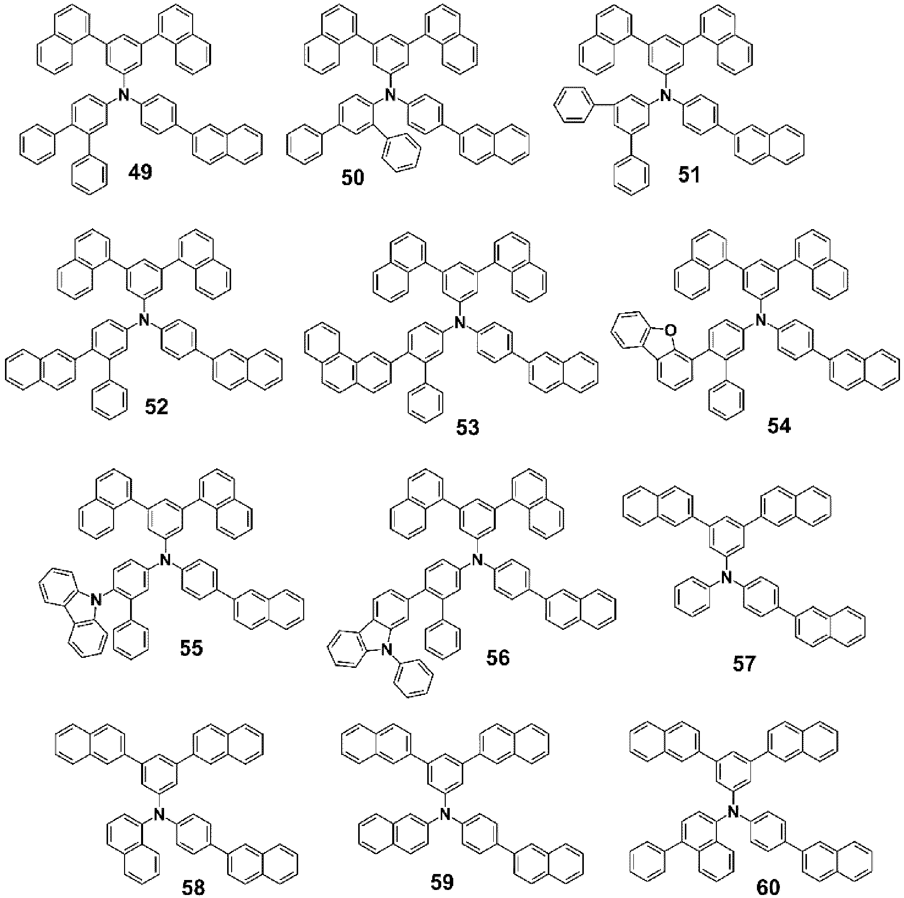 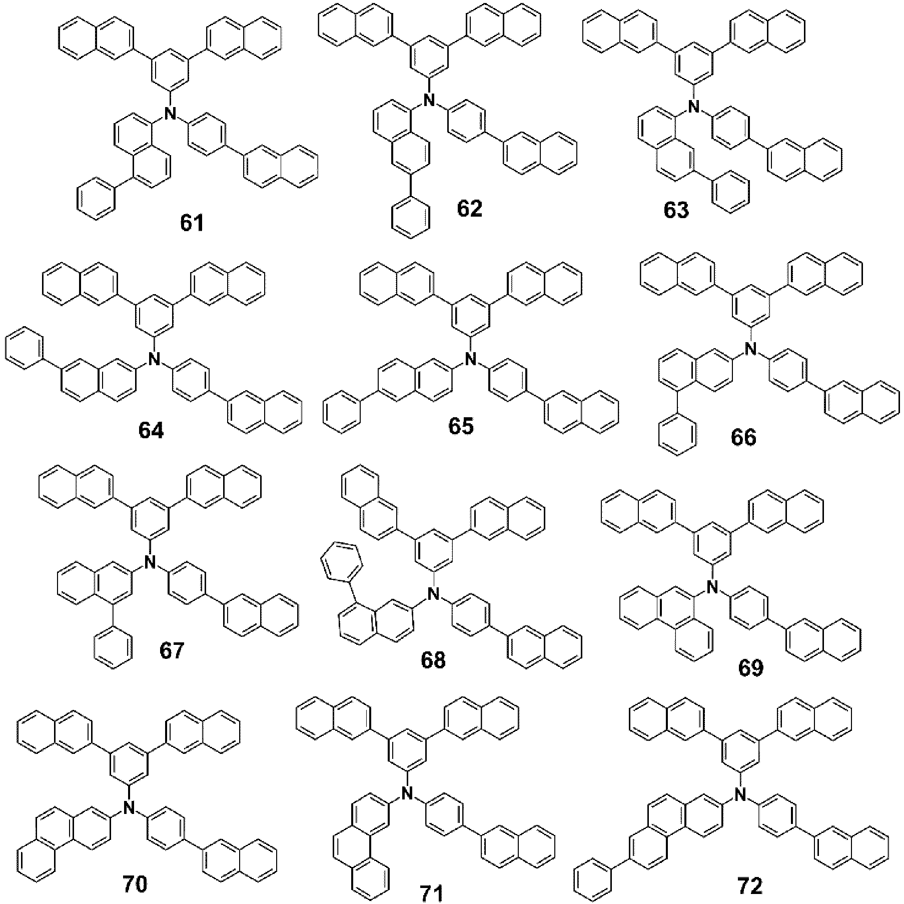 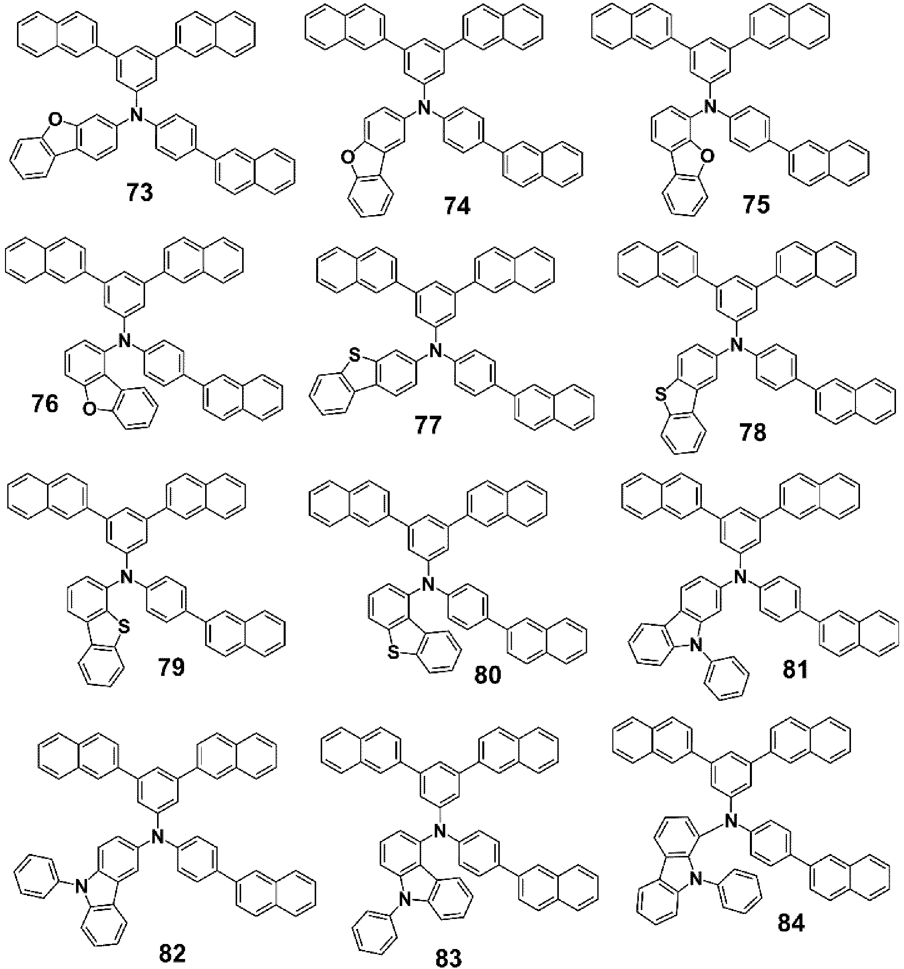 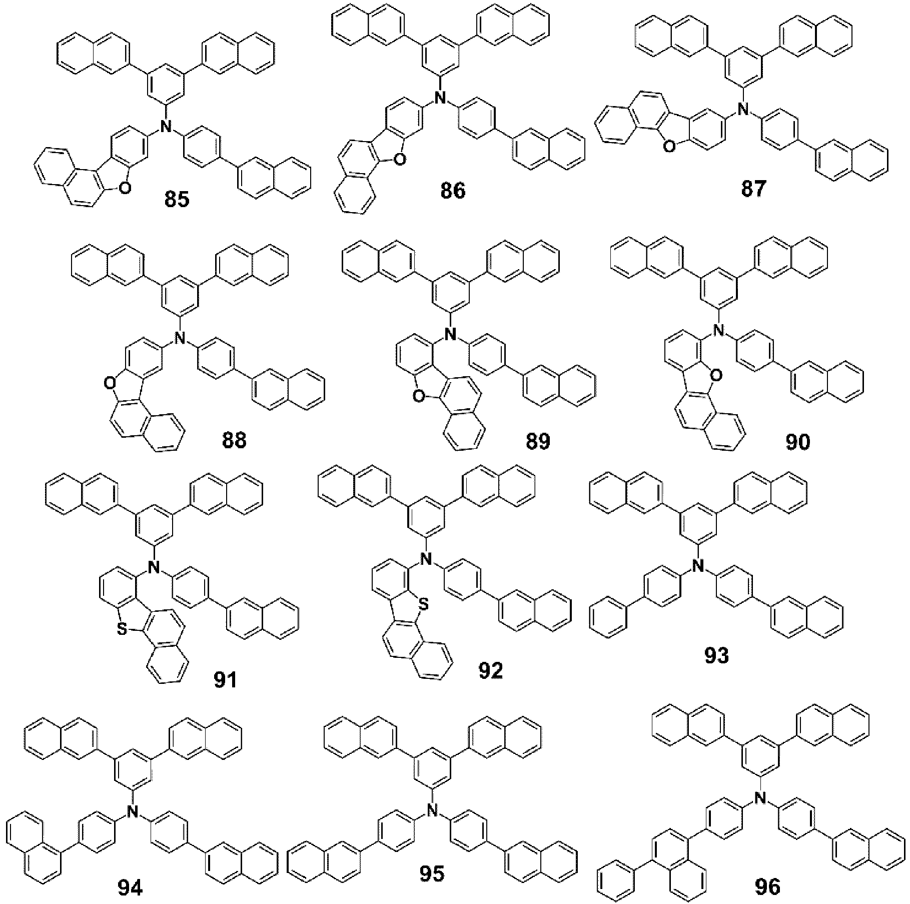 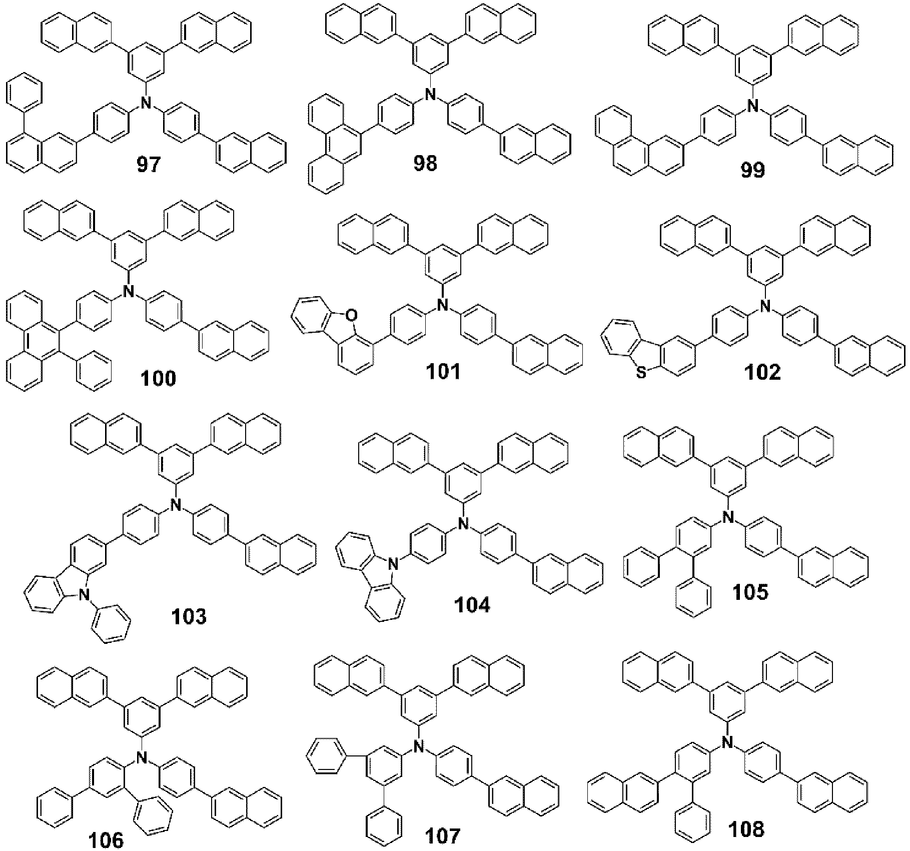 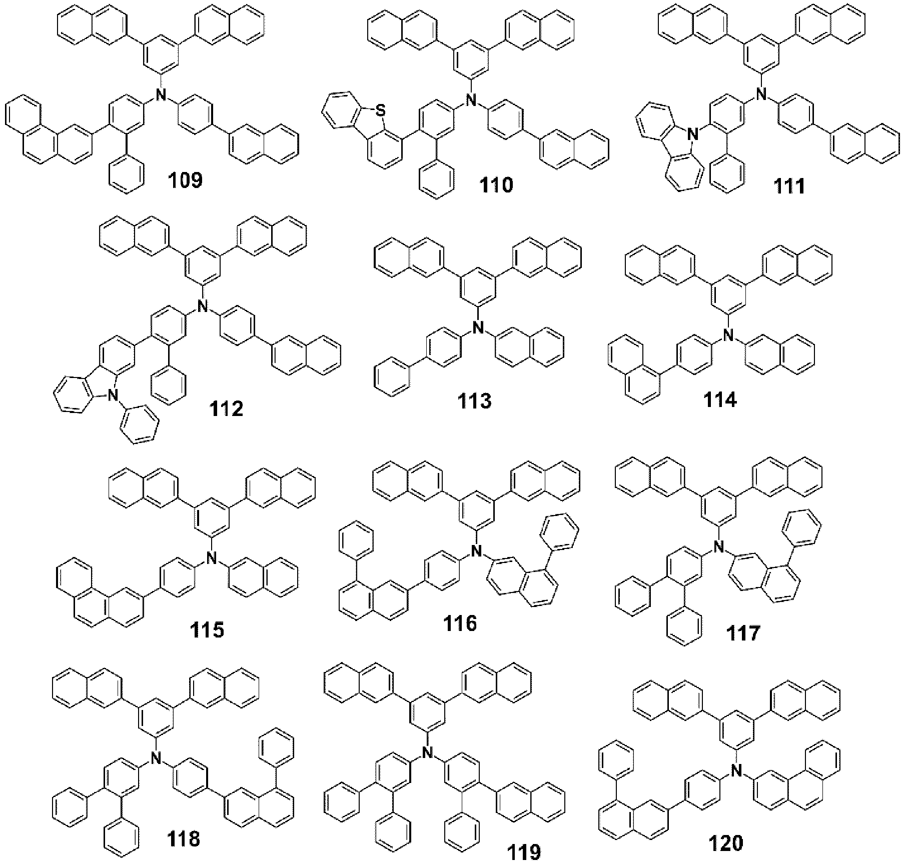 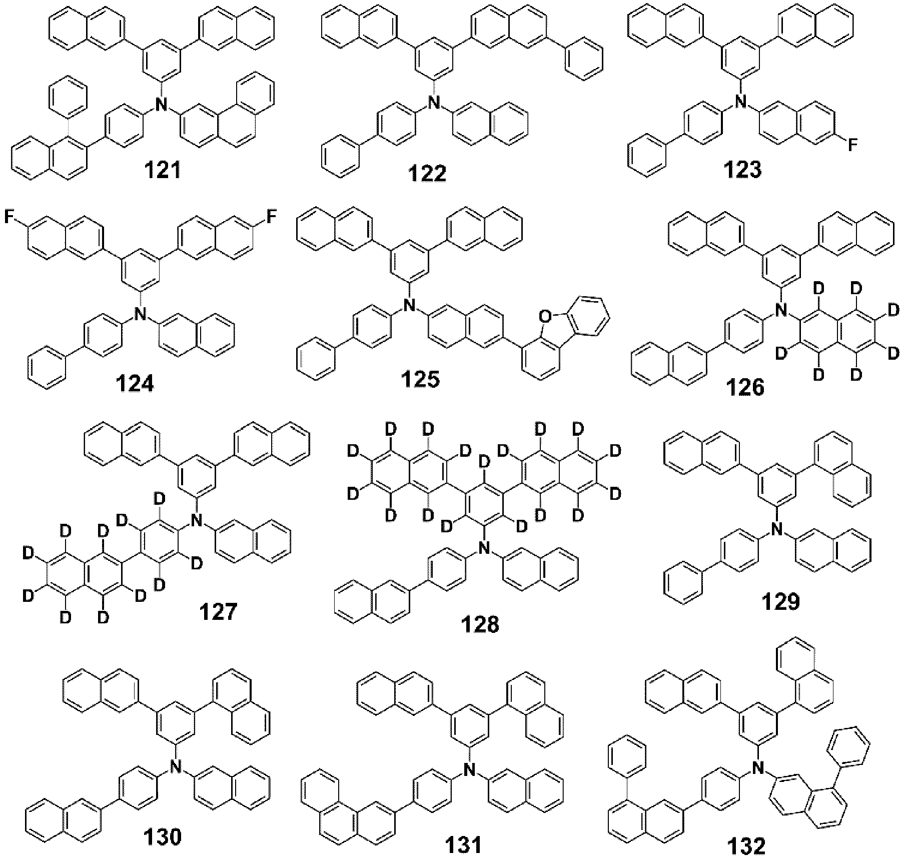 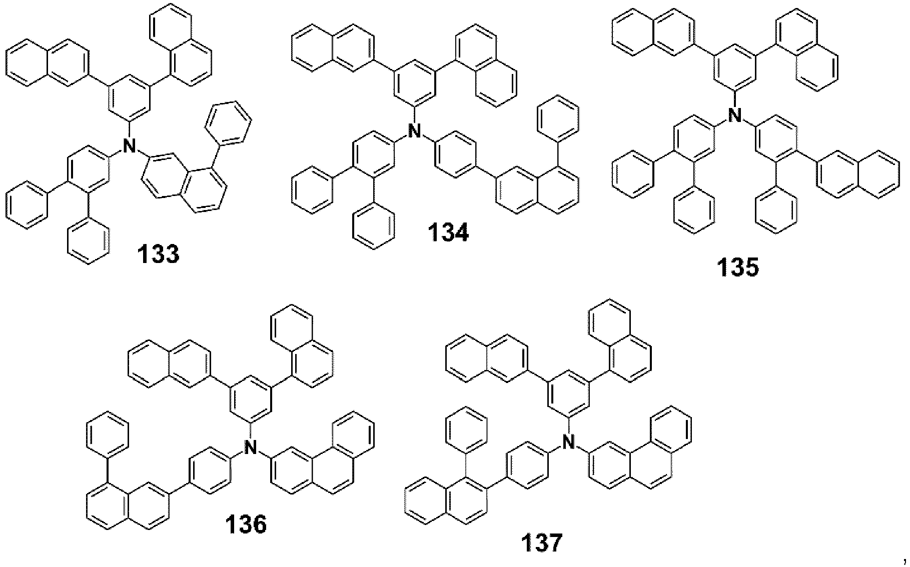 in Compound Group 1, D being deuterium.

9. A light emitting element (ED), comprising:
a first electrode (EL1);
a hole transport region (HTR) on the first electrode (EL1) and comprising a monoamine compound being according to any one of claims 1 to 8;
an emission layer (EML) on the hole transport region (HTR);
an electron transport region (ETR) on the emission layer (EML); and
a second electrode (EL2) on the electron transport region (ETR).

10. The light emitting element (ED) of claim 9, wherein the emission layer (EML) comprises a compound represented by Formula E-1: 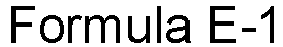 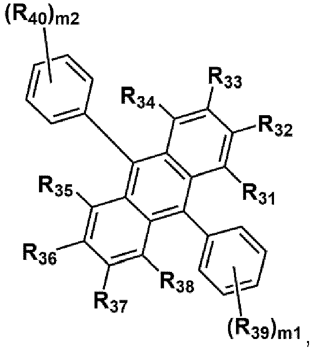 in Formula E-1,
m1 and m2 being each independently an integer of 0 to 5,
R₃₁ to R₄₀ being each independently hydrogen, deuterium, a halogen, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring.

11. An electronic apparatus (EA), comprising:
a base layer (BS);
a circuit layer (DP-CL) on the base layer (BS); and
a display element layer (DP-ED) on the circuit layer (DP-CL) and comprising a light emitting element (ED),
wherein the light emitting element (ED) comprises:
a first electrode (EL1);
a hole transport region (HTR) on the first electrode (EL1) and comprising a monoamine compound being according to any one of claims 1 to 8;
an emission layer (EML) on the hole transport region (HTR);
an electron transport region (ETR) on the emission layer (EML); and
a second electrode (EL2) on the electron transport region (ETR).

12. The electronic apparatus (EA) of claim 11, further comprising at least one of a light control layer (CCL) or a color filter layer (CFL),
wherein the light control layer (CCL) comprises quantum dots, and the color filter layer comprises a pigment and/or a dye.

13. The electronic apparatus (EA) of claim 11 or claim 12, comprising a display device (DD) that comprises the light emitting element (ED),
wherein the display device (DD) comprises any one selected from among a television, a monitor, an outdoor billboard, a personal computer, a laptop computer, a personal digital terminal, a vehicle display device, a game console, a portable electronic device, and a camera.
